# FASCICULE DE BREVET EUROPEEN

(11) **EP 2 235 014 B1**
(45) Date de publication et mention de la délivrance du brevet: **09.10.2013**
(21) Numéro de dépôt: 09718719.9
(22) Date de dépôt: 20.01.2009
(51) Int. Cl.: C07D 471/04, C07D 401/12, C07D 277/42, C07D 233/88, C07D 215/38, C07D 213/72, C07D 207/325, A61K 31/4709, A61K 31/4188, A61K 31/429, A61K 31/404, A61P 29/00, A61P 3/00, A61P 11/00

(54) **DÉRIVÉS BICYCLIQUES DE CARBOXAMIDES AZABICYCLIQUES, LEUR PRÉPARATION ET LEUR APPLICATION EN THÉRAPEUTIQUE.**
BIZYKLISCHE DERIVATE AUS AZABIZYKLISCHEN CARBOXAMIDEN, IHRE HERSTELLUNG UND THERAPEUTISCHE VERWENDUNG
BICYCLIC DERIVATIVES OF AZABICYCLIC CARBOXAMIDES, PREPARATION THEREOF AND THERAPEUTIC USE THEREOF

(30) Priorité: 22.01.2008 FR 0800309
(43) Date de publication de la demande: 06.10.2010
(73) Titulaire: SANOFI, 75008 Paris (FR)
(72) Inventeur: DUBOIS, Laurent, F-75013 Paris (FR); EVANNO, Yannick, F-75013 Paris (FR); GILLE, Catherine, F-75013 Paris (FR); MALANDA, André, F-75013 Paris (FR)
(74) Mandataire: Le Coupanec, Pascale A.M.P.
(86) Numéro de dépôt international: PCT/FR2009/000051
(87) Numéro de publication internationale: WO 2009/112677

(56) Documents cités:
- WO-A-2007/010138

## Description

Les documents WO2006/024776, WO2006/072736, WO2007/010144, WO2007/010138 décrivent des dérivés de carboxamides bicycliques, présentant une activité antagoniste ou agoniste *in vitro* et *in vivo* pour les récepteurs de type TRPV1 (ou VR1).

Il existe toujours un besoin de trouver de nouveaux ligands pour les récepteurs de type TRPV1, présentant des améliorations en termes d'activité fonctionnelle, de profil métabolique et / ou de profil de sécurité.

La présente invention répond à ce besoin en fournissant des dérivés de carboxamides azabicycliques qui présentent une activité antagoniste ou agoniste *in vitro* et *in vivo* pour les récepteurs de type TRPV1 (ou VR1).

Un premier objet de l'invention concerne les composés répondant à la formule générale (I) ci-après.

Un autre objet de l'invention concerne des procédés de préparation des composés de formule générale (I).

Un autre objet de l'invention concerne l'utilisation des composés de formule générale (I) notamment dans des médicaments ou dans des compositions pharmaceutiques.

Les composés de l'invention répondent à la formule générale (I) : 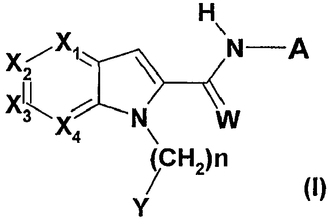 dans laquelle :
X₁, X₂, X₃, et X₄ représentent, indépendamment l'un de l'autre, un atome d'azote ou un groupe C-R₁ ;

Etant entendu que quand l'un des X₁, X₂, X₃, et X₄ représente un atome d'azote, les autres correspondent à un groupe C-R₁ ;
W représente un atome d'oxygène ou de soufre ;
n est égal à 0, 1, 2 ou 3 ;
Y représente un aryle ou un hétéroaryle éventuellement substitué par un ou plusieurs groupes choisis parmi un atome d'halogène, un groupe C₁-C₆-alkyle, C₃-C₇-cycloalkyle, C₃-C₇-cycloalkyl-C₁-C₃-alkylène, C₁-C₆-fluoroalkyle, hydroxy, C₁-C₆-alcoxy, C₃-C₇-cycloalkyloxy, C₃-C₇-cycloalkyl-C₁-C₆-alkylène-O-, C₁-C₆-fluoroalcoxy, cyano, C(O) NR₄R₅, nitro, NR₄R₅, C₁-C₆-thioalkyle, thiol, -S(O)-C₁-C₆-alkyle, -S(O)₂-C₁-C₆-alkyle, SO₂NR₄R₅, NR₆C(O)R₇, NR₂SO₂R₈, C(O)NR₄R₅, OC(O)NR₄R₅, -Si-(C₁-C₆-alkyl)₃, -SF₅, aryle-C₁-C₅-alkylène ou aryle, hétéroaryl-C₁-C₅-alkylène ou hétéroaryle ; les groupes C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₃-C₇-cycloalkyl-C₁-C₆-alkylène-O- étant éventuellement substitués par un groupe hydroxy, C₁-C₆-alcoxy ou NR₄R₅ ; les groupes aryle et hétéroaryle étant éventuellement substitués par un ou plusieurs substituants R₉ identiques ou différents l'un de l'autre ;

A représente le groupe de formule : 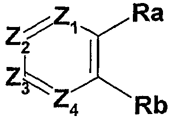 Z₁, Z₂, Z₃ et Z₄ représentent, indépendamment l'un de l'autre, un atome d'azote, un atome de carbone ou un groupe C-R₂,
l'un, au moins, des Z₁, Z₂, Z₃ et Z₄ correspondant à un atome d'azote et
l'un des Z₁, Z₂, Z₃ et Z₄, correspondant à un atome de carbone, étant lié à l'atome d'azote de l'amide ou du thioamide de formule (I) ;
Ra et Rb forment ensemble avec les atomes de carbone qui les portent,
soit un cycloalkyle partiellement insaturé, soit un aryle ;
soit un hétérocycle, soit un hétéroaryle de 5 à 7 chaînons comprenant de un à trois hétéroatomes choisi parmi O, S ou N ;
étant entendu que le cas où Ra et Rb forment ensemble avec les atomes de carbone qui les portent un cycle à cinq chaînons, ce cycle comportant un atome d'azote et des atomes de carbone, ce cycle étant partiellement saturé ou insaturé, est exclu ;
ce cycloalkyle partiellement insaturé, cet aryle, cet hétérocycle ou cet hétéroaryle pouvant être substitués par un ou plusieurs substituants R₃ ;
R₁ est choisi parmi un atome d'hydrogène, un atome d'halogène, un groupe C₁-C₆-alkyle, C₃-C₇-cycloalkyle, C₃-C₇-cycloalkyl-C₁-C₃-alkylène, C₁-C₆-fluoroalkyle, aryloxy-C₁-C₆-alkyle, hétéroaryloxy-C₁-C₆-alkyle, aryl-C₁-C₃-alkylènoxy-C₁-C₆-alkyle, hétéroaryl-C₁-C₃-alkylènoxy-C₁-C₆-alkyle, arylthio-C₁-C₆-alkyle, hétéroarylthio-C₁-C₆-alkyle, aryl-C₁-C₃-alkylène-thio-C₁-C₆-alkyle, hétéroaryl-C₁-C₃-alkylène-thio-C₁-C₆-alkyle, C₁-C₆-alcoxy, C₃-C₇-cycloalkyloxy, C₃-C₇-cycloalkyl-C₁-C₃-alkylènoxy, C₁-C₆-fluoroalcoxy, cyano, C(O)NR₄R₅, nitro, NR₄R₅, C₁-C₆-thioalkyle, C₃-C₇-cycloalkylthio, C₃-C₇-cycloalkyl-C₁-C₃-alkylène-thio, -S(O)-C₁-C₆-alkyle, -S(O)-C₃-C₇-cycloalkyle,-S(O)-C₁-C₃-alkylène-C₃-C₇-cycloalkyle, C₁-C₆-alkyle-S(O)₂-, C₁-C₆-fluoroalkyle-S(O)₂-, C₃-C₇-cycloalkyl-S(O)₂-, C₃-C₇-cycloalkyl-C₁-C₃-alkylène-S(O)₂-, SO₂NR₄R₅, -Si-(C₁-C₆-alkyl)₃, SF₅, NR₆C(O)R₇, NR₆SO₂R₈, C(O)NR₄R₅, OC(O)NR₄R₅, aryle, hétéroaryle, aryl-C₁-C₅-alkylène, hétéroaryl-C₁-C₅-alkylène, aryloxy, arylthio, hétéroaryloxy ou hétéroarylthio ; les groupes hétéroaryle ou aryle étant éventuellement substitués par un ou plusieurs substituants R₉, identiques ou différents l'un de l'autre ;
R₂ représente un atome d'hydrogène, un atome d'halogène, un groupe C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₃-C₇-cycloalkyl-C₁-C₃-alkylénoxy, hydroxy, thiol, C₁-C₆-fluoroalcoxy ;
R₃, représente, lorsqu'il est porté par un atome de carbone, un atome d'hydrogène, un groupe hydroxy, thiol, C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₃-C₇-cycloalkyl-C₁-C₃-alkylénoxy, C₁-C₆-alcoxy-C₁-C₃-alkylène, C₃-C₇-cycloalkyloxy-C₁-C₃-alkylène, C₃-C₇-cycloalkyl-C₁-C₃-alkylénoxy-C₁-C₃-alkylène, C(O)NR₄R₅, C(O)O-C₁-C₆-alkyle, CO₂H, oxo, thio ; les groupes C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₃-C₇-cycloalkyl-C₁-C₃-alkylénoxy, C₁-C₆-alcoxy-C₁-C₃-alkylène, C₃-C₇-cycloalkyloxy-C₁-C₃-alkylène, C₃-C₇-cycloalkyl-C₁-C₃-alkylénoxy-C₁-C₃-alkylène pouvant être substitués par un groupe hydroxy, C₁-C₆-alcoxy, -OC(O)-C₁-C₆-alkyle ou NR₄R₅ ;
ou
R₃, représente, lorsqu'il est porté par un atome d'azote, un atome d'hydrogène, un groupe C₁-C₆-alkyle, C₃-C₇-cycloalkyle, C₃-C₇-cycloalkyl-C₁-C₃-alkylène, C₁-C₆-fluoroalkyle, aryle-C(O)-, C₁-C₆-alkyle-C(O)-, C₃-C₇-cycloalkyl-C(O)-, C₃-C₇-cycloalkyl-C₁-C₃-alkylène-C(O)-, C₁-C₆-fluoroalkyle-C(O)-, aryle-S(O), C₁-C₆-alkyle-S(O)-, C₁-C₆-fluoroalkyle-S(O)-, aryle-S(O)₂-, C₁-C₆-alkyle-S(O)₂-, C₁-C₆-fluoroalkyle-S(O)₂-, C₃-C₇-cycloalkyl-S(O)₂-, C₃-C₇-cycloalkyl-C₁-C₃-alkylène-S(O)₂-, C₁-C₆-alkyle-O-C(O)-, aryle-C₁-C₃-alkyle-O-C(O)-, C₃-C₇-cycloalkyl-O-C(O)-, C₃-C₇-cycloalkyl-C₁-C₃-alkylène-O-C(O)-, C₁-C₆-fluoroalkyle-O-C(O)-, aryle-O-C(O)-, hétéroaryl-O-C(O)-, hétéroaryle ou aryle ; les groupes hétéroaryle et aryle étant éventuellement substitués par un ou plusieurs substituants R₉, identiques ou différents l'un de l'autre ; les groupes C₁-C₆-alkyle, C₃-C₇-cycloalkyle, C₃-C₇-cycloalkyl-C₁-C₃-alkylène, C₁-C₆-fluoroalkyle, pouvant être substitués par un groupe hydroxy, C₁-C₆-alcoxy ou NR₄R₅ ;
R₄ et R₅, représentent, indépendamment l'un de l'autre, un atome d'hydrogène ou un groupe C₁-C₆-alkyle, C₃-C₇-cycloalkyle, C₃-C₇-cycloalkyl-C₁-C₃-alkylène, aryle-C₁-C₅-alkylène ou aryle, ou R₄ et R₅ forment ensemble, avec l'atome d'azote qui les porte, un groupe azétidine, pyrrolidine, pipéridine, azépine, morpholine, thiomorpholine, pipérazine, homopipérazine ; le groupe NR₄R₅ étant éventuellement substitué par un groupe C₁-C₆-alkyle, C₃-C₇-cycloalkyle, C₃-C₇-cycloalkyl-C₁-C₃-alkylène, aryle-C₁-C₆-alkylène, aryle, hétéroaryle, aryle-S(O)₂-, C₁-C₆-alkyle-S(O)₂-, C₁-C₆-fluoroalkyle-S(O)₂, C₃-C₇-cycloalkyl-S(O)₂-, C₃-C₇-cycloalkyl-C₁-C₃-alkylène-S(O)₂-, aryle-C(O)-, C₁-C₆-alkyle-C(O)-, C₃-C₇-cycloalkyl-C(O)-, C₃-C₇-cycloalkyl-C₁-C₃-alkylène-C(O)-, C₁-C₆-fluoroalkyle-C(O)-, hydroxy, C₁-C₆-alkyloxy, C₃-C₇-cycloalkyloxy, C₃-C₇-cycloalkyl-C₁-C₃-alkylénoxy, C₁-C₆-fluoroalkyle, aryloxy-C₁-C₆-alkylène, aryloxy, hétéroaryloxy-C₁-C₆-alkylène, hétéroaryloxy ;
R₆ et R₇ représentent, indépendamment l'un de l'autre, un atome d'hydrogène, un groupe C₁-C₆-alkyle, C₃-C₇-cycloalkyle, C₃-C₇-cycloalkyl-C₁-C₃-alkylène, aryle-C₁-C₆-alkylène ou aryle ; le groupe aryle étant éventuellement substitué par un ou plusieurs substituants choisis parmi un atome d'halogène, un groupe C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₃-C₇-cycloalkyl-C₁-C₃-alkylénoxy, C₁-C₆-fluoroalcoxy, nitro ou cyano ; ou R₆ et R₇ forment ensemble un lactame de 4 à 7 chaînons comprenant l'atome d'azote et le groupe C(O) qui les portent ;
R₈ représente un groupe C₁-C₆-alkyle, C₃-C₇-cycloalkyle, C₃-C₇-cycloalkyl-C₁-C₃-alkylène, aryle-C₁-C₆-alkylène ou aryle ; le groupe aryle étant éventuellement substitué par un ou plusieurs substituants choisis parmi un atome d'halogène, un groupe C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₃-C₇-cycloalkyl-C₁-C₃-alkylénoxy, C₁-C₆-fluoroalcoxy, nitro ou cyano ;
ou R₆ et R₈ forment ensemble un sultame de 4 à 7 chaînons comprenant l'atome d'azote et le groupe S(O)₂ qui les portent ;

R₉ représente un atome d'halogène, un groupe C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₃-C₇-cycloalkyl-C₁-C₃-alkylénoxy, C₁-C₆-fluoroalcoxy ; ces groupes étant éventuellement substitués par un groupe OH, C₁-C₆-alcoxy ou NR₄R₅ ; ou bien R₉ représente un groupe nitro, cyano ou NR₄R₅.

Dans les composés de formule générale (I) :
- le ou les atomes de soufre peuvent être sous forme oxydée (S(O) ou S(O)₂) ;
- le ou les atomes d'azote peuvent éventuellement être sous forme oxydée (N-oxyde).

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels peuvent être préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de la présente invention, on entend par :
- un atome d'halogène: un fluor, un chlore, un brome ou un iode ;
- Cₜ-C_{z} : une chaîne carbonée pouvant avoir de t à z atomes de carbone où t et z peuvent prendre les valeurs de 1 à 7 ; par exemple C₁-C₃ est une chaîne carbonée qui peut avoir de 1 à 3 atomes de carbone ;
- un alkyle : un groupe aliphatique saturé linéaire ou ramifié. A titre d'exemples, on peut citer les groupes méthyle, éthyle, propyle, isopropyle, butyle, isobutyle, tertiobutyle, pentyle, etc ;
- un alkylène : un groupe alkyle divalent saturé, linéaire ou ramifié, par exemple un groupe C₁₋₃-alkylène représente une chaîne carbonée divalente de 1 à 3 atomes de carbone, linéaire ou ramifiée, plus particulièrement un méthylène, éthylène, 1-méthyléthylène, propylène ;
- un cycloalkyle : un groupe alkyle cyclique, saturé ou partiellement insaturé. A titre d'exemples, on peut citer les groupes cyclopropyle, cyclobutyle, cyclopentyle, cyclohexyle, cyclopropènyle, cyclobutènyle, cyclopentènyle, cyclohexènyle etc ;
- un cycloalkyloxy : un radical -O-cycloalkyle où le groupe cycloalkyle est tel que précédemment défini ;
- un fluoroalkyle : un groupe alkyle dont un ou plusieurs atomes d'hydrogène ont été substitués par un atome de fluor ;
- un alcoxy : un radical -O-alkyle où le groupe alkyle est tel que précédemment défini ;
- un fluoroalcoxy : un groupe alcoxy dont un ou plusieurs atomes d'hydrogène ont été substitués par un atome de fluor ;
- un thioalkyle ou alkyle-thio : un radical -S-alkyle où le groupe alkyle est tel que précédemment défini ;
- un aryle : un groupe aromatique mono- ou bicyclique comprenant entre 6 et 10 atomes de carbones. A titre d'exemples de groupe aryle, on peut citer les groupes phényle ou naphthyle ;
- un hétérocycle : un groupe monocyclique saturé ou partiellement insaturé, de 5 à 7 chaînons, comprenant de 1 à 3 hétéroatomes choisis parmi O, S ou N.

A titre d'exemples d'hétérocycle, on peut citer les groupes azétidinyle, pipéridinyle, azépinyle, morpholinyle, thiomorpholinyle, pipérazinyle, homopipérazinyle, dihydrooxazolyle, dihydrothiazolyle, dihydroimidazolyle, dihydropyrrolyle ou tétrahydropyridinyle, [1,3]dioxolyle, [1,3]dioxinyle, dihydro[1,4]dioxinyle, dihydro[1,2]oxazinyle, dihydro[1,3]oxazinyle, dihydro-oxazole, dihydro-isoxazole, dihydro[1,4]oxazinyle, tétrahydro[1,3]oxazépinyle, tétrahydro[1,4]oxazépinyle, tétrahydro[1,3]diazépinyle, tétrahydro[1,4]diazépinyle.
- un hétéroaryle : un groupe mono- ou bicyclique aromatique de 5 à 12 chaînons contenant de 1 à 5 hétéroatomes choisis parmi O, S ou N.
   A titre d'exemples d'hétéroaryle monocyclique, on peut citer les groupes imidazolyle, pyrazolyle, thiazolyle, oxazolyle, isothiazolyle, isoxazolyle, furanyle, thiényle, oxadiazolyle, thiadiazolyle, triazolyle, tétrazolyle, pyridinyle, pyrazinyle, pyrimidinyle, pyridazinyle, triazinyle.
   A titre d'exemples d'hétéroaryle bicyclique, on peut citer les groupes indolyle, isoindolyle, benzofuranyle, benzothiophényle, benzoxazolyle, benzimidazolyle, indazolyle, benzothiényle, isobenzofuranyle, isobenzothiazolyle, pyrrolo[2,3-*c*]pyridinyle, pyrrolo[2,3-*b*]pyridinyle, pyrrolo[3,2-*b*]pyridinyle, pyrrolo[3,2-*c*]pyridinyle, pyrrolo[1,2-*a*]pyridinyle, quinoléinyle, isoquinoléinyle, cinnolinyle, quinazolinyle, quinoxalinyle, pyrrolo[1,2-*a*]imidazolyle, imidazo[1,2-*a*]pyridinyle, imidazo[1,2-*a*]pyridazinyle, imidazo[1,2-*c*]pyrimidinyle, imidazo[1,2-*a*]pyrimidinyle, imidazo[1,2-*a*]pyrazinyle, imidazo[4,5-*b*]pyrazinyle, imidazo[4,5-*b*]pyridinyle, imidazo[4,5-*c*]pyridinyle, pyrazolo[2,3-*a*]pyridinyle, pyrazolo[2,3-*a*]pyrimidinyle, pyrazolo[2,3-*a*]pyrazinyle, thiazolo[5,4-*b*]pyridinyle,
   thiazolo[5,4-*c*]pyridinyle, thiazolo[4,5-*c*]pyridinyle, thiazolo[4,5-*b*]pyridinyle, oxazolo[5,4-*b*]pyridinyle, oxazolo[5,4-*c*]pyridinyle, oxazolo[4,5-*c*]pyridinyle, oxazolo[4,5-*b*]pyridinyle, isothiazolo[5,4-*b*]pyridinyle, isothiazolo[5,4-*c*]pyridinyle, isothiazolo[4,5-*c*]pyridinyle, isothiazolo[4,5-*b*]pyridinyle, isoxazolo[5,4-*b*]pyridinyle, isoxazolo[5,4-*c*]pyridinyle, isoxazolo[4,5-*c*]pyridinyle, isoxazolo[4,5-*b*]pyridinyle.
- « oxo » signifie « =O »;
- « thio » signifie « = S ».

Parmi les composés de formule générale (I) objets de l'invention, un premier sous-groupe de composés est constitué par les composés pour lesquels
X₁, X₂, X₃, et X₄ représentent, indépendamment l'un de l'autre un groupe C-R₁ ; R₁ étant tel que défini dans la formule générale (I).

Parmi les composés de formule générale (I) objets de l'invention, un second sous-groupe de composés est constitué par les composés pour lesquels
X₁, X₂, X₃, et X₄ représentent, indépendamment l'un de l'autre, un atome d'azote ou un groupe C-R₁ ;
Etant entendu que l'un des X₁, X₂, X₃, et X₄ représente un atome d'azote, les autres correspondent à un groupe C-R₁ ;
R₁ étant tel que défini dans la formule générale (I).

Parmi les composés de formule générale (I) objets de l'invention, un troisième sous-groupe de composés est constitué par les composés pour lesquels
X₁, X₂, X₃ représentent un groupe C-R₁ ; X₄ représente un atome d'azote ;
R₁ étant tel que défini dans la formule générale (I).

Parmi les composés de formule générale (I) objets de l'invention, un quatrième sous-groupe de composés est constitué par les composés pour lesquels
R₁ est choisi parmi un atome d'hydrogène, un atome d'halogène, un groupe C₁-C₆-fluoroalkyle, -Si-(C₁-C₆-alkyl)₃,

Parmi les composés de formule générale (I) objets de l'invention, un cinquième sous-groupe de composés est constitué par les composés pour lesquels
R₁ est choisi parmi un atome d'hydrogène, un atome de fluor, un groupe CF₃, -Si-(CH₃)₃.

Parmi les composés de formule générale (I) objets de l'invention, un sixième sous-groupe de composés est constitué par les composés pour lesquels
X₁, X₂, X₃, et X₄ représentent, indépendamment l'un de l'autre un groupe C-R₁ ; R₁ est choisi parmi un atome d'hydrogène, un atome d'halogène, un groupe C₁-C₆-fluoroalkyle, -Si-(C₁-C₆-alkyl)₃.

Parmi les composés de formule générale (I) objets de l'invention, un septième sous-groupe de composés est constitué par les composés pour lesquels
X₁, X₂, X₃ représentent un groupe C-R₁ ; X₄ représente un atome d'azote ;
R₁ est choisi parmi un atome d'hydrogène, un groupe C₁-C₆-fluoroalkyle.

Parmi les composés de formule générale (I) objets de l'invention, un huitième sous-groupe de composés est constitué par les composés pour lesquels n est égal à 1.

Parmi les composés de formule générale (I) objets de l'invention, un neuvième sous-groupe de composés est constitué par les composés pour lesquels
Y représente un aryle ou un hétéroaryle éventuellement substitué par un ou plusieurs groupes choisis parmi un atome d'halogène, un groupe C₁-C₆-alkyle, C₁-C₆-fluoroalkyle.

Parmi les composés de formule générale (I) objets de l'invention, un dixième sous-groupe de composés est constitué par les composés pour lesquels
Y représente un phényle, un thiazolyle, un pyridinyle, ce groupe étant éventuellement substitué par un ou plusieurs groupes choisis parmi un atome d'halogène, un groupe C₁-C₆-alkyle, C₁-C₆-fluoroalkyle.

Parmi les composés de formule générale (I) objets de l'invention, un onzième sous-groupe de composés est constitué par les composés pour lesquels
Y représente un phényle, un thiazolyle, un pyridinyle, ce groupe étant éventuellement substitué par un ou plusieurs groupes choisis parmi un atome de fluor, un groupe méthyle ou CF₃.

Parmi les composés de formule générale (I) objets de l'invention, un douzième sous-groupe de composés est constitué par les composés pour lesquels
Y représente un phényle éventuellement substitué par un ou plusieurs groupes choisis parmi un atome de fluor, un groupe méthyle ou CF₃.

Parmi les composés de formule générale (I) objets de l'invention, un treizième sous-groupe de composés est constitué par les composés pour lesquels W représente un atome d'oxygène.

Parmi les composés de formule générale (I) objets de l'invention, un quatorzième sous-groupe de composés est constitué par les composés pour lesquels
Z₁, Z₂, Z₃ et Z₄ représentent, indépendamment l'un de l'autre, un atome d'azote, un atome de carbone ou un groupe C-R₂,
l'un des Z₁, Z₂, Z₃ et Z₄ correspondant à un atome d'azote et
l'un des Z₁, Z₂, Z₃ et Z₄, correspondant à un atome de carbone, étant lié à l'atome d'azote de l'amide ou du thioamide de formule (I) ;
et les deux autres de Z₁, Z₂, Z₃ et Z₄ correspondant à un groupe C-R₂ ;
R₂ étant tel que défini dans la formule générale (I).

Parmi les composés de formule générale (I) objets de l'invention, un quinzième sous-groupe de composés est constitué par les composés pour lesquels
Z₁, Z₂, Z₃ et Z₄ représentent, indépendamment l'un de l'autre, un atome d'azote, un atome de carbone ou un groupe C-R₂,
l'un des Z₁, Z₂, Z₃ et Z₄ correspondant à un atome d'azote et
l'un des Z₁, Z₂, Z₃ et Z₄, correspondant à un atome de carbone, étant lié à l'atome d'azote de l'amide ou du thioamide de formule (I) ;
et les deux autres de Z₁, Z₂, Z₃ et Z₄ correspondant à un groupe CH.

Parmi les composés de formule générale (I) objets de l'invention, un seizième sous-groupe de composés est constitué par les composés pour lesquels
Ra et Rb forment ensemble avec les atomes de carbone qui les portent,
soit un cycloalkyle partiellement insaturé, soit un aryle ;
soit un hétérocycle, soit un hétéroaryle de 5 ou 6 chaînons comprenant de un ou deux hétéroatomes choisi parmi O, S ou N ;
étant entendu que le cas où Ra et Rb forment ensemble avec les atomes de carbone qui les portent un cycle à cinq chaînons, ce cycle comportant un atome d'azote et des atomes de carbone, ce cycle étant partiellement saturé ou insaturé, est exclu ;
ce cycloalkyle partiellement insaturé, cet aryle, cet hétérocycle ou cet hétéroaryle pouvant être substitués par un ou plusieurs substituants R₃ ;
R₃ étant tel que défini dans la formule générale (I).

Parmi les composés de formule générale (I) objets de l'invention, un dix-septième sous-groupe de composés est constitué par les composés pour lesquels
Ra et Rb forment ensemble avec les atomes de carbone qui les portent,
soit un cycloalkyle partiellement insaturé, soit un aryle ;
soit un hétérocycle, soit un hétéroaryle de 5 ou 6 chaînons comprenant de un ou deux hétéroatomes choisi parmi O, S ou N ;
étant entendu que le cas où Ra et Rb forment ensemble avec les atomes de carbone qui les portent un cycle à cinq chaînons, ce cycle comportant un atome d'azote et des atomes de carbone, ce cycle étant partiellement saturé ou insaturé, est exclu ;
ce cycloalkyle partiellement insaturé, cet aryle, cet hétérocycle ou cet hétéroaryle pouvant être substitués par un ou plusieurs substituants R₃ ;
R₃, représente, lorsqu'il est porté par un atome de carbone, un atome d'hydrogène, un groupe hydroxy, C₁-C₆-alkyle, oxo ; le groupe C₁-C₆-alkyle pouvant être substitué par un groupe hydroxy ou -OC(O)-C₁-C₆-alkyle ;
ou
R₃, représente, lorsqu'il est porté par un atome d'azote, un atome d'hydrogène ou un groupe C₁-C₆-alkyle.

Parmi les composés de formule générale (I) objets de l'invention, un dix-huitième sous-groupe de composés est constitué par les composés pour lesquels
Ra et Rb forment ensemble avec les atomes de carbone qui les portent,
soit un cycloalkyle partiellement insaturé, soit un aryle ;
soit un hétérocycle, soit un hétéroaryle de 5 ou 6 chaînons comprenant de un ou deux hétéroatomes choisi parmi O, S ou N ;
étant entendu que le cas où Ra et Rb forment ensemble avec les atomes de carbone qui les portent un cycle à cinq chaînons, ce cycle comportant un atome d'azote et des atomes de carbone, ce cycle étant partiellement saturé ou insaturé, est exclu ;
ce cycloalkyle partiellement insaturé, cet aryle, cet hétérocycle ou cet hétéroaryle pouvant être substitués par un ou plusieurs substituants R₃ ;
R₃, représente, lorsqu'il est porté par un atome de carbone, un atome d'hydrogène, un groupe hydroxy, méthyle, oxo ; le groups méthyle pouvant être substitué par un groupe hydroxy ou -OC(O)-ter-butyle ;
ou
R₃, représente, lorsqu'il est porté par un atome d'azote, un atome d'hydrogène ou un groupe méthyle.

Parmi les composés de formule générale (I) objets de l'invention, un dix-neuvième sous-groupe de composés est constitué par les composés pour lesquels A représente le groupe de formule : 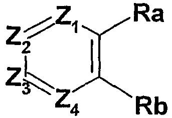 où A est choisi parmi les groupes 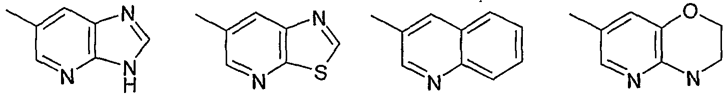 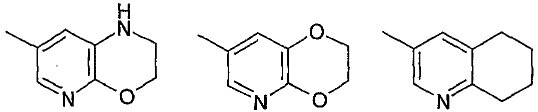 ces groupes étant éventuellement substitués par R₂ et R₃ comme défini dans la formule générale (I) ci-dessus.

Parmi les composés de formule générale (I) objets de l'invention, un vingtième sous-groupe de composés est constitué par les composés pour lesquels A représente le groupe de formule : 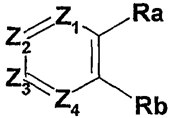 où A est choisi parmi les groupes 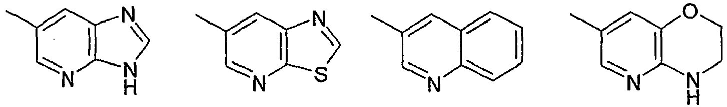 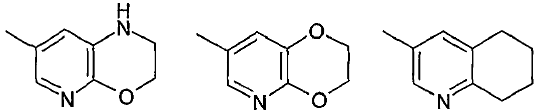 ces groupes étant éventuellement substitués par R₂ et R₃ comme défini dans la formule générale (I) ci-dessus ;
R₂ représente un atome d'hydrogène ;
R₃, représente, lorsqu'il est porté par un atome de carbone, un atome d'hydrogène, un groupe hydroxy, C₁-C₆-alkyle, oxo ; le groupe C₁-C₆-alkyle pouvant être substitué par un groupe hydroxy ou -OC(O)-C₁-C₆-alkyle ;
ou
R₃, représente, lorsqu'il est porté par un atome d'azote, un atome d'hydrogène ou un groupe C₁-C₆-alkyle.

Parmi les composés de formule générale (I) objets de l'invention, un vingt-et-unième sous-groupe de composés est constitué par les composés pour lesquels A représente le groupe de formule : 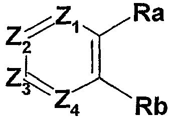 où A est choisi parmi les groupes 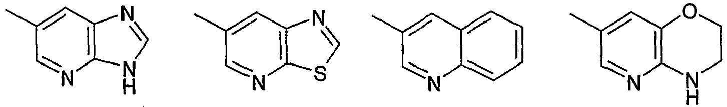 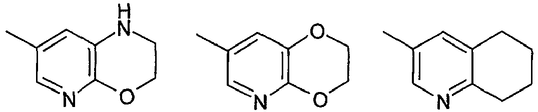 ces groupes étant éventuellement substitués par R₂ et R₃ comme défini dans la formule générale (I) ci-dessus ;
R₃, représente, lorsqu'il est porté par un atome de carbone, un atome d'hydrogène, un groupe hydroxy, méthyle, oxo ; le groups méthyle pouvant être substitué par un groupe hydroxy ou -OC(O)-ter-butyle ;
ou
R₃, représente, lorsqu'il est porté par un atome d'azote, un atome d'hydrogène ou un groupe méthyle.

Parmi les composés de formule générale (I) objets de l'invention, un vingt-deuxième sous-groupe de composés est constitué par les composés pour lesquels les définitions de X₁, X₂, X₃, et X₄, n, Y, W, Z₁, Z₂, Z₃, Z₄; Ra et Rb données ci-dessus sont combinées.

Parmi les composés de formule générale (I) objets de l'invention, un vingt-troisième sous-groupe de composés est constitué par les composés pour lesquels
X₁, X₂, X₃, et X₄ représentent, indépendamment l'un de l'autre un groupe C-R; ou bien X₁, X₂, X₃ représentent un groupe C-R₁ ; X₄ représente un atome d'azote ;
R₁ est choisi parmi un atome d'hydrogène, un atome d'halogène, un groupe C₁-C₆-fluoroalkyle, -Si-(C₁-C₆-alkyl)₃ ;
n est égal à 1 ;
Y représente un aryle ou un hétéroaryle éventuellement substitué par un ou plusieurs groupes choisis parmi un atome d'halogène, un groupe C₁-C₆-alkyle, C₁-C₆-fluoroalkyle ;
W représente un atome d'oxygène ;
A représente le groupe de formule : 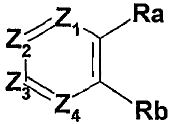 où A est choisi parmi les groupes 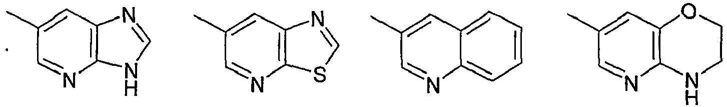 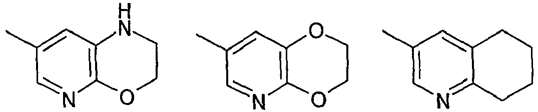 ces groupes étant éventuellement substitués par R₂ et R₃ comme défini dans la formule générale (I) ci-dessus ;
R₂ représente un atome d'hydrogène ;
R₃, représente, lorsqu'il est porté par un atome de carbone, un atome d'hydrogène, un groupe hydroxy, C₁-C₆-alkyle, oxo ; le groupe C₁-C₆-alkyle pouvant être substitué par un groupe hydroxy ou -OC(O)-C₁-C₆-alkyle ;
ou
R₃, représente, lorsqu'il est porté par un atome d'azote, un atome d'hydrogène ou un groupe C₁-C₆-alkyle.

Parmi les composés de formule générale (I) objets de l'invention, un vingt-quatrième sous-groupe de composés est défini tel que
les composés pour lesquels
X₁, X₂, X₃, et X₄ représentent, indépendamment l'un de l'autre, un groupe C-R₁ ;
R₁ est choisi parmi un atome d'hydrogène, un atome d'halogène, un groupe C₁-C₆-alkyle, C₁-C₆-fluoroalkyle, C₁-C₆-alcoxy, C₁-C₆-fluoroalcoxy, NR₄R₅, C₁-C₆-thioalkyle, phényle ou isoxazolyle ; le groupe phényle étant éventuellement substitué par un ou plusieurs substituants R₉, identiques ou différents l'un de l'autre;
R₄ et R₅, représentent, indépendamment l'un de l'autre, un atome d'hydrogène ou un groupe C₁-C₆-alkyle,
W représente un atome d'oxygène ;
n est égal à 0 ;
Y représente un phényle éventuellement substitué par un ou plusieurs substituants R₉, identiques ou différents l'un de l'autre ; ou Y représente un isoxazole ;
R₉ représente un atome d'halogène, un groupe C₁-C₆-alkyle, C₁-C₆-alcoxy, cyano ;
A représente le groupe D : 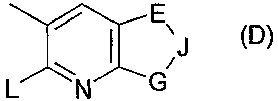 dans lequel
L représente un atome d'hydrogène, un atome d'halogène ou un groupe C₁-C₄-alcoxy ; le cycle à cinq chaînons est partiellement saturé ou insaturé ; J représente N ou C=O ; E et G représentent, indépendamment l'un de l'autre, un atome d'oxygène ou de soufre, ou un groupe C=O, CH₂ ou N-R' ; R' représente un atome d'hydrogène ou un groupe C₁-C₄-alkyle ou aryle-C(O)-, le groupe aryle étant éventuellement substitué par un ou plusieurs groupe C₁-C₆-alkyle ;
sont exclus.

Parmi les composés de formule générale (I) objets de l'invention, une sous-famille est représentée par les composés de formule générale (I') pour lesquels :
R₃, représente, lorsqu'il est porté par un atome de carbone, un atome d'hydrogène, un groupe hydroxy, thiol, C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₃-C₇-cycloalkyl-C₁-C₃-alkylénoxy, C₁-C₆-alcoxy-C₁-C₃-alkylène, C₃-C₇-cycloalkyloxy-C₁-C₃-alkylène, C₃-C₇-cycloalkyl-C₁-C₃-alkylénoxy-C₁-C₃-alkylène, C(O)NR₄R₅, C(O)O-C₁-C₆-alkyle, CO₂H, oxo, thio ; les groupes C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₃-C₇-cycloalkyl-C₁-C₃-alkylénoxy, C₁-C₆-alcoxy-C₁-C₃-alkylène, C₃-C₇-cycloalkyloxy-C₁-C₃-alkylène, C₃-C₇-cycloalkyl-C₁-C₃-alkylénoxy-C₁-C₃-alkylène pouvant être substitués par un groupe hydroxy, C₁-C₆-alcoxy, ou NR₄R₅ ;

Parmi les composés de formule générale (I') objets de l'invention, un premier sous-groupe de composés est constitué par les composés pour lesquels X₁, X₂, X₃, et X₄ représentent, indépendamment l'un de l'autre un groupe C-R₁ ; et R₁ est choisi parmi un atome d'hydrogène, un atome d'halogène, plus particulièrement un atome de fluor.

Parmi les composés de formule générale (I') objets de l'invention, un second sous-groupe de composés est constitué par les composés pour lesquels n est égal à 1 et Y représente un aryle, plus particulièrement un phényle, éventuellement substitué par un ou plusieurs atomes d'halogène, plus particulièrement de fluor.

Parmi les composés de formule générale (I') objets de l'invention, un troisième sous-groupe de composés est constitué par les composés pour lesquels W représente un atome d'oxygène.

Parmi les composés de formule générale (I') objets de l'invention, un quatrième sous-groupe de composés est constitué par les composés pour lesquels A représente le groupe de formule : 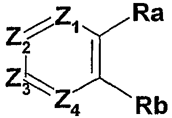 où A est choisi parmi les groupes 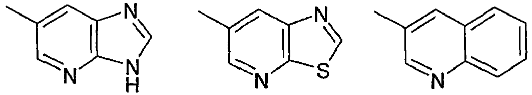 ces groupes étant éventuellement substitués par R₂ et R₃ comme défini dans la formule générale (I) ci-dessus.

Parmi les composés de formule générale (I') objets de l'invention, un cinquième sous-groupe de composés est constitué par les composés pour lesquels lesquels X₁, X₂, X₃, et X₄ représentent, indépendamment l'un de l'autre un groupe C-R₁ et R₁ est choisi parmi un atome d'hydrogène, un atome d'halogène, plus particulièrement un atome de fluor ;
lesquels n est égal à 1 ;
Y représente un aryle, plus particulièrement un phényle, éventuellement substitué par un ou plusieurs atomes d'halogène, plus particulièrement de fluor ;
W représente un atome d'oxygène ;
A représente le groupe de formule : 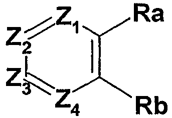 où A est choisi parmi les groupes 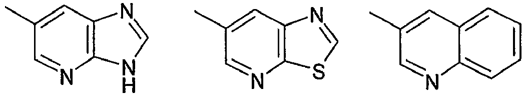 ces groupes étant éventuellement substitués par R₂ et R₃ comme défini dans la formule générale (I) ci-dessus.

Parmi les composés de formule générale (I) objets de l'invention, on peut notamment citer les composés suivants :
1 *N*-(2,3-Diméthyl-3*H*-imidazo[4,5-*b*]pyridin-6-yl)-5-fluoro-1-[(3-fluorophényl)méthyl]-1*H*-indole-2-carboxamide ;
2 *N*-(2-Méthyl-thiazolo[5,4-*b*]pyridin-6-yl)-5-fluoro-1-[(3-fluorophényl)méthyl]-1*H-*indole-2-carboxamide ;
3 *N*-(Thiazolo[5,4-*b*]pyridin-6-yl)-5-fluoro-1-[(3-fluorophényl)méthyl]-1*H*-indole-2-carboxamide ;
4 *N*-(2-Hydroxyméthyl-thiazolo[5,4-*b*]pyridin-6-yl)-5-fluoro-1-[(3-fluorophényl)méthyl]-1*H*-indole-2-carboxamide ;
5 *N*-(Quinoléin-3-yl)-5-fluoro-1-[(3-fluorophényl)méthyl]-1*H*-indole-2-carboxamide.
6 *N*-(2-Hydroxyméthyl-3-méthyl-3*H*-imidazo[4,5-*b*]pyridin-6-yl)-5-fluoro-1-[(3-fluorophényl)méthyl]-1*H*-indole-2-carboxamide
7 *N*-(3-Méthyl-3*H*-imidazo[4,5-*b*]pyridin-6-yl)-5-fluoro-1-[(3-fluorophényl)méthyl]-1*H*-indole-2-carboxamide
8 *N*-(2,3-Diméthyl-3*H*-imidazo[4,5-*b*]pyridin-6-yl)-5-trifluorométhyl-1-[(3-fluorophényl)méthyl]-1*H*-pyrrolo[2,3-*b*]pyridine-2-carboxamide
9 *N*-(3,4-Dihydro-2*H*-pyrido[3,2-*b*][1,4]oxazin-7-yl)-5-fluoro-1-[(3-fluorophényl)méthyl]-1*H*-indole-2-carboxamide
10 *N*-(4-Méthyl-3,4-dihydro-2*H*-pyrido[3,2-*b*][1,4]oxazin-7-yl)-5-fluoro-1-[(3-fluorophényl)méthyl]-1*H*-indole-2-carboxamide
11 *N*-(2-Méthyl-3*H*-imidazo[4,5-*b*]pyridin-6-yl)-5-fluoro-1-[(3-fluorophényl)méthyl]-1*H*-indole-2-carboxamide
12 *N*-(1-Méthyl-2-oxo-2,3-dihydro-1H-pyrido[2,3-*b*][1,4]oxazin-7-yl)-5-fluoro-1-[(3-fluorophényl)méthyl]-1*H*-indole-2-carboxamide
13 2,2-Diméthylpropanoate de [6-[[[5-fluoro-1-[(3-fluorophényl)méthyl]-1*H*-indol-2-yl]carbonyl]amino]thiazolo[5,4-*b*]pyridin-2-yl]méthyle
14 *N*-(2,3-dihydro-[1,4]dioxino[2,3-*b*]pyridin-7-yl))-5-fluoro-1-[(3-fluorophényl)méthyl]-1*H*-indole-2-carboxamide
15 *N*-(6-Hydroxy-5,6,7,8-tétrahydroquinoléin-3-yl)-5-fluoro-1-[(3-fluorophényl)méthyl]-1*H*-indole-2-carboxamide
16 *N*-(2-Hydroxyméthyl-thiazolo[5,4-*b*]pyridin-6-yl)-5-trifluorométhyl-1-[(3-méthylphényl) méthyl]-1*H*-indole-2-carboxamide
17 *N*-(2-Hydroxyméthyl-thiazolo[5,4-*b*]pyridin-6-yl)-6-trifluorométhyl-1-[(3-méthylphényl)méthyl]-1*H-*indole-2-carboxamide
18 *N*-(2-Hydroxyméthyl-thiazolo[5,4-*b*]pyridin-6-yl)-5-triméthylsilyl-1-[(3-méthylphényl)méthyl]-1*H*-indole-2-carboxamide
19 *N*-(2-Hydroxyméthyl-thiazolo[5,4-b]pyridin-6-yl)-6-fluoro-1-[(3-méthylphényl)méthyl]-1*H*-indole-2-carboxamide
20 *N*-(2-Hydroxyméthyl-thiazolo[5,4-*b*]pyridin-6-yl)-5-trifluorométhyl-1-[(thiazol-2-yl)méthyl]-1*H*-indole-2-carboxamide
21 *N*-(2-Hydroxyméthyl-thiazolo[5,4-*b*]pyridin-6-yl)-6-trifluorométhyl-1-[(thiazol-2-yl)méthyl]-1*H*-indole-2-carboxamide
22 *N*-(2-Hydroxyméthyl-thiazolo[5,4-*b*]pyridin-6-yl)-5-triméthylsilyl-1-[(thiazol-2-yl)méthyl]-1*H*-indole-2-carboxamide
23 *N*-(2-Hydroxyméthyl-thiazolo[5,4-*b*]pyridin-6-yl)-6-fluoro-1-[(thiazol-2-yl)méthyl]-1*H*-indole-2-carboxamide
24 *N*-(2-Hydroxyméthyl-thiazolo[5,4-*b*]pyridin-6-yl)-5-trifluorométhyl-1-[(thiazol-2-yl)méthyl]-1*H*-pyrrolo[2,3-*b*]pyridine-2-carboxamide
25 *N*-(2-Hydroxyméthyl-thiazolo[5,4-*b*]pyridin-6-yl)-6-triméthylsilyl-1-[(thiazol-2-yl)méthyl]-1*H*-indole-2-carboxamide
26 *N*-(2-Hydroxyméthyl-thiazolo[5,4-*b*]pyridin-6-yl)-5-trifluorométhyl-1-[(pyridin-4-yl)méthyl]-1*H*-indole-2-carboxamide
27 *N*-(2-Hydroxyméthyl-thiazolo[5,4-*b*]pyridin-6-yl)-6-trifluorométhyl-1-[(pyridin-4-yl)méthyl]-1*H*-indole-2-carboxamide
28 *N*-(2-Hydroxyméthyl-thiazolo[5,4-*b*]pyridin-6-yl)-5-triméthylsilyl-1-[(pyridin-4-yl)méthyl]-1*H*-indole-2-carboxamide
29 *N*-(2-Hydroxyméthyl-thiazolo[5,4-*b*]pyridin-6-yl)-6-fluoro-1-[(pyridin-4-yl)méthyl]-1*H*-indole-2-carboxamide
30 *N*-(2-Hydroxyméthyl-thiazolo[5,4-*b*]pyridin-6-yl)-5-trifluorométhyl-1-[(pyridin-4-yl)méthyl]-1*H*-pyrrolo[2,3-*b*]pyridine-2-carboxamide
31 *N*-(2-Hydroxyméthyl-thiazolo[5,4-*b*]pyridin-6-yl)-6-triméthylsilyl-1-[(pyridin-4-yl)méthyl]-1*H*-indole-2-carboxamide
32 *N*-(2-Hydroxyméthyl-thiazolo[5,4-*b*]pyridin-6-yl)-5-triméthylsilyl-1-[[(3-trifluorométhyl)phényl]méthyl]-1*H*-indole-2-carboxamide
33 *N*-(2-Hydroxyméthyl-thiazolo[5,4-*b*]pyridin-6-yl)-6-fluoro-1-[[(3-trifluorométhyl)phényl]méthyl]-1*H*-indole-2-carboxamide
34 *N*-(2-Hydroxyméthyl-thiazolo[5,4-*b*]pyridin-6-yl)-5-trifluorométhyl-1-[[(3-trifluorométhyl)phényl]méthyl]-1*H*-pyrrolo[2,3-*b*]pyridine-2-carboxamide
35 *N*-(2-Hydroxyméthyl-thiazolo[5,4-*b*]pyridin-6-yl)-6-triméthylsilyl-1-[[(3-trifluorométhyl)phényl]méthyl]-1*H*-indole-2-carboxamide
36 *N*-(2-Hydroxyméthyl-thiazolo[5,4-*b*]pyridin-6-yl)-5-trifluorométhyl-1-[(3-méthylphényl)méthyl]-1*H*-pyrrolo[2,3-*b*]pyridine-2-carboxamide
37 *N*-(2-Hydroxyméthyl-thiazolo[5,4-*b*]pyridin-6-yl)-6-triméthylsilyl-1-[(3-méthylphényl)méthyl]-1*H*-indole-2-carboxamide
38 *N*-(2-Hydroxyméthyl-thiazolo[5,4-*b*]pyridin-6-yl)-5-trifluorométhyl-1-[[(3-trifluorométhyl)phényl]méthyl]-1*H*-indole-2-carboxamide
39 *N*-(2-Hydroxyméthyl-thiazolo[5,4-*b*]pyridin-6-yl)-6-trifluorométhyl-1-[[(3-trifluorométhyl)phényl]méthyl]-1*H*-indole-2-carboxamide

On entend par groupe partant, dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un méthanesulfonate, benzènesulfonate, p-toluènesulfonate, triflate, acétate, etc. Des exemples de groupes partants ainsi que des références pour leur préparation sont donnés dans « Advances in Organic Chemistry », J. March, 5th Edition, Wiley Interscience, 2001.

On entend par groupe protecteur, dans ce qui suit, un groupe pouvant être momentanément incorporé à une structure chimique dans le but d'inactiver temporairement une partie de la molécule lors d'une réaction et qui peut être facilement être enlevé à une étape ultérieure de la synthèse. Des exemples de groupes protecteurs ainsi que des références concernant leurs propriétés sont données dans T.W. Greene, P.G.M. Wutz, 3rd Edition, Wiley Interscience 1999.

Conformément à l'invention, on peut préparer les composés de formule générale (I) selon le procédé illustré par le schéma général 1 qui suit

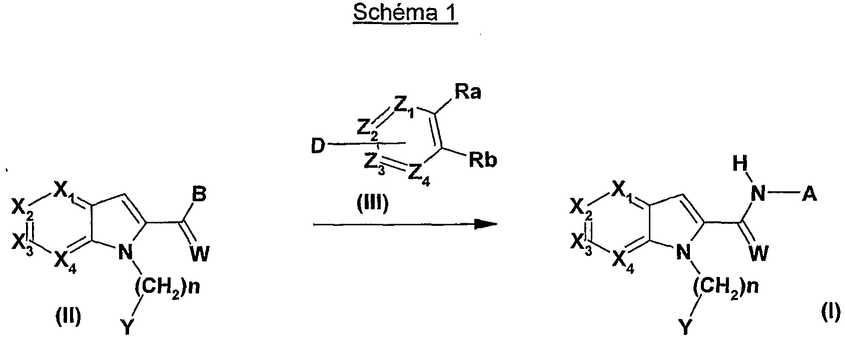

Les composés (I) peuvent être obtenus par réaction d'un composé de formule générale (II) dans laquelle B correspond à un groupe hydroxyle et X₁, X₂, X₃, X₄, n, Y et W sont tels que définis dans la formule générale (I) ci-dessus avec une amine de formule générale (III), dans laquelle Z₁, Z₂, Z₃, Z₄, Ra et Rb sont tels que définis dans la formule générale (I) ci-dessus et D correspond à un groupe amino, en présence d'un agent de couplage tel qu'un dialkylcarbodiimide, l'hexafluorophosphate de [(benzotriazol-1-yl)oxy][tris(pyrrolidino)]phosphonium, le diéthylcyanophosphonate ou tout autre agent de couplage connu de l'homme du métier, éventuellement en présence d'une base comme la triéthylamine, dans un solvant tel que par exemple le diméthylformamide.

D'autre part, les composés (I) peuvent être obtenus par réaction d'un composé de formule générale (II), dans laquelle B représente un groupe C₁-C₆-alcoxy, C₃-C₇-cycloalkyloxy, C₃-C₇-cycloalkyl-C₁-C₃-alkylènoxy- ou aryle-C₁-C₃-alkylènoxy et X₁, X₂, X₃, X₄, n, Y et W sont tels que définis dans la formule générale (I) ci-dessus, avec un amidure du composé de formule générale (III), dans laquelle Z₁, Z₂, Z₃, Z₄, Ra et Rb sont tels que définis dans la formule générale (I) ci-dessus et D correspond à un groupe amino, au reflux d'un solvant tel que le toluène. L'amidure d'aluminium du composé de formule générale (III) est préparé par action préalable du triméthylaluminium sur les amines de formule générale (III).

Les composés (I) peuvent être également obtenus par réaction d'un composé de formule générale (II) dans laquelle B correspond à un atome de chlore et X₁, X₂, X₃, X₄, n, Y et W sont tels que définis dans la formule générale (I) ci-dessus avec une amine de formule générale (III), dans laquelle Z₁, Z₂, Z₃, Z₄, Ra et Rb sont tels que définis dans la formule générale (I) ci-dessus et D correspond à un groupe amino, par réaction en solution dans un solvant tel que le dichlorométhane ou le toluène. Les composés de formule générale (II) dans laquelle B correspond à un atome de chlore, sont préparés à partir de composés de formule générale (II) dans laquelle B correspond à un groupe hydroxyle par réaction avec un réactif tel que le chlorure de thionyle ou le chlorure d'oxalyle, éventuellement en présence d'une base comme la triéthylamine, en solution dans un solvant tel que le dichlorométhane.

En partant de composés de formule générale (II), dans laquelle B représente un groupe NH₂, W représente un atome d'oxygène et X₁, X₂, X₃, X₄, n et Y sont tels que définis dans la formule générale (1) ci-dessus, le composé de formule générale (I) peut être obtenu par réaction avec le composé de formule générale (III), dans laquelle Z₁, Z₂, Z₃, Z₄, Ra et Rb sont tels que définis dans la formule générale (I) ci-dessus et D correspond à un groupe partant tel que défini ci-dessus, tel qu'un atome de brome ou un groupe triflate, par exemple selon une méthode analogue à celle décrite dans J.Am.Chem.Soc. 2001, 123 (31), 7727, ou selon des méthodes décrites dans la littérature ou connues de l'homme du métier, en présence d'un sel de cuivre en quantité catalytique, en présence d'une quantité catalytique d'un ligand du cuivre, tel qu'une diamine, le tout en présence d'une base comme le carbonate de potassium, dans un solvant tel que le dioxane.

Dans le schéma 1, les composés de formule générale (II) et les autres réactifs, quand leur mode de préparation n'est pas décrit, sont disponibles dans le commerce, décrits dans la littérature ou préparés par analogie à des procédés décrits dans la littérature (D. Knittel Synthesis 1985, 2, 186 ; T.M. Williams J.Med.Chem. 1993, 36 (9), 1291 ; JP2001151771A2, WO2006/024776, WO2006/072736, WO2007/010144, WO2007/010138 par exemple).

Les composés de formule générale (III), quand leur mode de préparation n'est pas décrit, sont disponibles dans le commerce, décrits dans la littérature ou préparés par analogie à des procédés décrits dans la littérature (Ukrainskii Khimicheskii Zhurnal 1981, 47, 867; Yakugaku Zasshi 1950, 70, 187 ; W02007/100758).

Les composés de formule générale (II) ou (I), pour lesquels l'un des X₁, X₂, X₃ ou X₄, correspond à un atome de carbone substitué par un groupe alkyle, peuvent être obtenus par une réaction de couplage, catalysée par un métal tel que le palladium ou le fer, réalisée sur les composés de formules générales (II) ou (I) correspondants, substitués par un atome d'halogène, tel qu'un chlore, en présence par exemple d'un halogénure d'alkylmagnésium ou d'un halogénure d'alkylzinc, selon les méthodes décrites dans la littérature (A. Furstner et al J Am Chem Soc 2002, 124(46), 13856 ; G. Quéguiner et al J Org Chem 1998, 63(9), 2892) par exemple, ou connues de l'homme du métier.

Les composés de formule générale (II) ou (I), pour lesquels l'un des X₁, X₂, X₃ ou X₄, correspond à un atome de carbone substitué par un groupe cyano, aryle ou hétéroaryle, peuvent être obtenus par une réaction de couplage, catalysée par un métal tel que le palladium, réalisée sur les composés de formule générale (II) ou (I) correspondants, substitués, par exemple, par un atome de brome, en présence de cyanure de triméthylsilyle, d'acide arylboronique ou d'acide hétéroarylboronique, ou par toute autre méthode décrite dans la littérature ou connue de l'homme du métier. Les composés de formule générale (I) ou (II) pour lesquels l'un des X₁, X₂, X₃ ou X₄, correspond à un atome de carbone substitué par un groupe NR₄R₅, NR₆COR₇ ou NR₆SO₂R₈, peuvent être obtenus à partir des composés de formule générale (I) ou (II) correspondants, substitués, par exemple, par un atome de brome, par réaction de couplage respectivement avec une amine, un amide ou une sulfonamide en présence d'une base, d'une phosphine et d'un catalyseur à base de palladium, selon des méthodes décrites dans la littérature ou connues de l'homme du métier.

Les composés de formule générale (I) ou (II) substitués par un groupe C(O)NR₄R₅, peuvent être obtenus à partir des composés de formule générale (I) ou (II) correspondants substitués par un groupe cyano, selon des méthodes décrites dans la littérature ou connues de l'homme du métier.

Les composés de formule générale (I) ou (II) substitués par un groupe -S(O)-alkyle ou-S(O)₂-alkyle, peuvent être obtenus par oxydation des composés de formule générale (II) ou (I) correspondants, substitués par un groupe thioalkyle, selon des méthodes décrites dans la littérature ou connues de l'homme du métier.

Les composés de formule générale (II) ou (I), substitués par un groupe NR₄R₅, NR₆COR₇ ou NR₆SO₂R₈, peuvent être obtenus à partir des composés de formule générale (II) ou (I) correspondants, substitués par un groupe nitro, par exemple par réduction, puis acylation ou sulfonylation, selon des méthodes décrites dans la littérature ou connues de l'homme du métier.

Les composés de formule générale (II) ou (I), substitués par un groupe SO₂NR₄R₅ peuvent être obtenus par une méthode analogue à celle décrite dans Pharmazie 1990, 45, 346, ou selon des méthodes décrites dans la littérature ou connues de l'homme du métier.

Les composés de formule générale (I) ou (II) dans laquelle W représente un atome de soufre peuvent, par exemple, être obtenus par réaction des composés de formule générale (I) ou (II) correspondants dans lesquels W représente un atome d'oxygène, avec un réactif tel que le réactif de Lawesson.

Les composés de formule générale (I) pour lesquelles R₃ correspond à un groupe protecteur porté par un azote, tel qu'un groupe acétyle, éthoxycarbonyle, terbutyloxycarbonyle ou un groupe benzyloxycarbonyle, peuvent être déprotégés, selon des méthodes chimiques connues de l'homme de l'art, pour conduire à des composés de formule générale (I) où R₃ est un atome d'hydrogène.

Les composés de formule générale (I) pour lesquelles R₃ correspond à un groupe hydroxyalkyle peuvent être obtenus à partir des composés de formule générale (I) pour lesquelles R₃ correspond, par exemple, à un groupe acétoxyalkyle ou pivaloyloxyalkyle selon des méthodes chimiques connues de l'homme de l'art, telle que la réaction avec une base, par exemple une solution aqueuse de soude, ou la réaction avec un alcoolate, par exemple un méthanolate, d'un sel tel que le lithium ou de sodium, dans un solvant alcoolique tel que le méthanol ou l'éthanol, ou la réaction avec un agent réducteur tel que le borohydrure de sodium, dans un solvant tel que le tétrahydrofurane.

Alternativement, les composés de formule générale (I) pour lesquelles R₃ correspond à un groupe hydroxyalkyle peuvent être obtenus à partir des composés de formule générale (III), dans laquelle D correspond à un groupe amino et R₃ correspond, par exemple, à un groupe pivaloyloxyalkyle, par réaction avec un organométallique, tel que le triméthylaluminium, suivie d'un couplage avec un composé de formule générale (II), dans laquelle B correspond à un groupe C₁-C₆-alcoxy, C₃-C₇-cycloalkyloxy, C₃-C₇-cycloalkyl-C₁-C₃-alkylénoxy- ou aryle-C₁-C₃-alkylénoxy et X₁, X₂, X₃, X₄, n, Y et W sont tels que définis dans la formule générale (I) ci-dessus, suivi d'un traitement en milieu aqueux acide.

Les composés de formule générale (II) du schéma 1, dans laquelle l'un des X₁, X₂, X₃ ou X₄ correspond à un groupe C-R₁ où R₁ représente un groupe -Si-(C₁-C₆-alkyl)₃ et B représente un groupe C₁-C₆-alcoxyle, peuvent être obtenus, par exemple, selon la méthode illustrée dans le schéma 2.

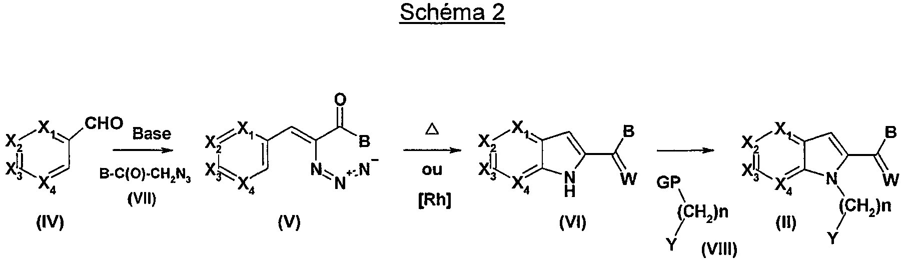

Selon cette méthode, les composés de formule générale (II), définis tel que n est égal à 1, 2 ou 3, sont obtenus par réaction des composés (VI) correspondants avec un réactif (VIII) dans lequel GP représente un groupe partant tel qu'un atome de chlore, de brome ou d'iode et n est égal à 1, 2 ou 3. La réaction de formation des composés de formule générale (II) peut être réalisée en présence d'une base telle que l'hydrure de sodium ou le carbonate de potassium, dans un solvant polaire tel que le diméthylformamide, le diméthylsulfoxyde ou l'acétone (n = 1 : Kolasa T., Bioorg.Med.Chem. 1997, 5 (3) 507, n = 2 : Abramovitch R., Synth. Commun., 1995, 25 (1), 1).

Lorsque le composé de formule générale (VIII) est défini tel que n est égal à 1, 2 ou 3 et GP représente un groupe hydroxyle, les composés de formule générale (II), peuvent être obtenus par réaction du composé de formule générale (VI) avec un composé de formule générale (VIII) en présence d'une phosphine telle que, par exemple, la triphénylphosphine et d'un réactif tel que, par exemple, l'azodicarboxylate de diéthyle en solution dans un solvant tel que le dichlorométhane ou le tétrahydrofuranne (O. Mitsonobu, Synthesis, 1981, 1-28).

De façon analogue, les composés de formule générale (II), peuvent être obtenus par réaction du composé de formule générale (VI) avec un composé de formule générale (VIII) en présence d'une phosphine supportée sur une résine et d'un réactif tel que, par exemple, l'azodicarboxylate de diisopropyle en solution dans un solvant tel que le dichlorométhane ou le tétrahydrofuranne.

Lorsque le composé de formule générale (VIII) est défini tel que n est égal à 0 et GP représente un groupe partant tel qu'un atome de chlore, de brome ou d'iode, la réaction de formation des composés de formule générale (II) peut être réalisée par application et adaptation des méthodes décrites par S. L. Buchwald et al. (J. Am. Chem. Soc., 2001, 123, 7727 et 2002, 124, 11684), de préférence sous atmosphère inerte en milieu basique, par exemple en présence de triphosphate de potassium, en présence d'un sel de cuivre tel que l'iodure de cuivre, éventuellement en présence d'un additif tel que la N,N'-diméthylcyclohexane-1,2-diamine, le tout dans un solvant organique tel que le toluène.

Les composés de formule générale (VI) sont préparés à partir d'aldéhydes aromatiques ou hétéroaromatiques substitués par un groupe silanyle de formule générale (IV) dans laquelle X₁, X₂, X₃ et X₄ sont tels que définis dans la formule générale (I), l'un d'entre eux correspondant à un groupe silanyle, par réaction avec un azidoacétate d'alkyle de formule générale (VII) dans laquelle B représente un groupe C₁-C₆-alcoxyle, tel que l'azidoacétate d'éthyle par exemple, en présence d'une base telle que l'éthoxide de sodium, dans un solvant comme l'éthanol ou le méthanol, pour conduire aux azido-2-cinnamates d'alkyles de formule générale (V). Ces derniers sont ensuite transformés en ester indoliques ou azaindoliques au reflux d'un solvant, par exemple dans le xylène ou le toluène, par adaptation de protocoles décrits dans la littérature (Hemetsberger et al Monatsh. Chem., 1969, 100, 1599 et 1970, 101, 161 ; P. Roy et al Synthesis., 2005, 16, 2751-2757 ; R. Guilard et al J. Heterocyclic. Chem., 1981, 18, 1365-1377 ; W.Rees et al J. Chem. Soc, perkin trans 1 1984, 2189-2196 ; P. Molina et al J. org. Chem., 2003, 68(2), 489-499; C. Moody et al J. Chem. Soc., Perkin Trans. 1 1984, 2189-2196 ; J. Sawyer et al J. Med. Chem , 2005, 48, 893-896 ; D. Tanner Synlett 2006, 18, 3140-3144).

Alternativement, la formation des composés de formule générale (VI) peut être obtenue par décomposition de l'azido-2-cinnamate d'alkyle de formule générale (V), en présence d'un complexe dimère de rhodium, dans un solvant tel que le toluène à une température comprise entre 25°C et 60°C, selon une adaptation de protocoles décrits dans la littérature (Tom G. Drivers et al J. Am. Chem. Soc., 2007, 129, 7500-7501 ; J. Sawyer et al J. Med. Chem , 2005, 48, 893-896).

Les aldéhydes aromatiques ou hétéroaromatiques substitués par un groupe silanyle de formule générale (IV), quand ils ne sont pas disponibles dans le commerce, peuvent être obtenus à partir des aldéhydes aromatiques ou hétéroaromatiques correspondants, de préférence masqués sous la forme d'un acétal par exemple, substitués par un atome d'halogène tel qu'un brome ou un iode, dans la position où le groupe silanyle doit être introduit :
- par exemple par réaction avec un disilane tel que l'hexaméthyldisilane, en présence d'une quantité catalytique d'un complexe métallique, de préférence un complexe de palladium, comme par exemple le tétrakis(triphénylphosphine)palladium, sans solvant ou dans un solvant, de préférence polaire comme par exemple l'hexaméthylphosphoramide, en présence d'une base comme le carbonate de potassium, à une température comprise entre 20°C et la température d'ébullition du solvant (adaptation des protocoles décrits dans la littérature : J. Babin et al J. organométal. Chem., 1993, 446 (1-2), 135-138 ; E. Shirakawa et al Chem. Commun., 2000, 1895-1896 ; L. Goossen et al Synlett, 2000, 1801-1803 ; H. Matsumoto et al J. organométal. Chem., 1975, 85, C1 ; FR2677358).
- par exemple par réaction avec un disilane tel que l'hexaméthyldisilane, en présence d'une base forte, comme par exemple le méthoxide de potassium ou de sodium, dans un solvant polaire comme par exemple l'hexaméthylphosphoric triamide (HMPT), à une température proche de 20°C (adaptation des protocoles décrits dans la littérature : A.I Meyers et al J. org. Chem., 1977, 42 (15), 2654-2655 ; K. Ishimaru et al Heterocycles., 2001, 55 (8), 1591-1597)**.**

Les aldéhydes aromatiques ou hétéroaromatiques substitués par un groupe silanyle de formule générale (IV), quand ils ne sont pas disponibles dans le commerce, peuvent également être obtenus à partir des aromatiques ou hétéroaromatiques dihalogénés correspondants, tel qu'un dérivé dibromé, dans la position où le groupe silanyle doit être introduit, par échange avec un organométallique, comme par exemple le n-butyllithium. Les aromatiques ou hétéroaromatiques métalliques ainsi formés peuvent ensuite réagir avec des organohalogénosilanes ou être transformés en dérivés formylés par adaptation des méthodes décrits dans la littérature. La réaction est menée de préférence à des températures basses comprises entre - 110°C et la température ambiante, dans un solvant comme l'éther ou le THF (adaptation des protocoles décrits dans la littérature : Bao-Hui Ye et al Tetrahedron., 2003, 59, 3593-3601 ; P. Pierrat et al Synlett 2004, 13, 2319-2322 ; K.T.Warner et al Heterocycles 2002, 58, 383 ; D. Deffieux et al J. organométal. Chem., 1994, 13 (6), 2415-2422 ; W02005080328 ; S.G.Davies et al J. Chem. Soc, perkin trans 1 1991, 501 ; G. Queguiner et al J. Org. Chem. , 1981, 46, 4494-4497 ; G. Breton et al. Tetrahedron 2000, 56 (10), 1349-1360 ; S. De Montis et al. Tetrahedron 2004, 60 (17), 3915-3920 ; L. Buchwald et al J. Am. Chem. Soc., 1998, 120, 4960-4976).

L'invention, selon un autre de ses aspects, a également pour objet les composés de formule générale (IIa), (IIb), (IIc), (IId), (IIe), (IIf), (IIg) et (IIh), dans lesquelles Me représente un groupe méthyle et Et représente un groupe éthyle. Ces composés sont utiles comme intermédiaires de synthèse des composés de formule générale (I).

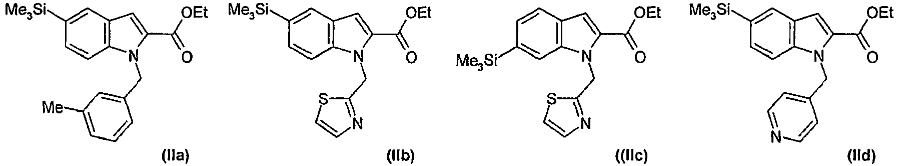

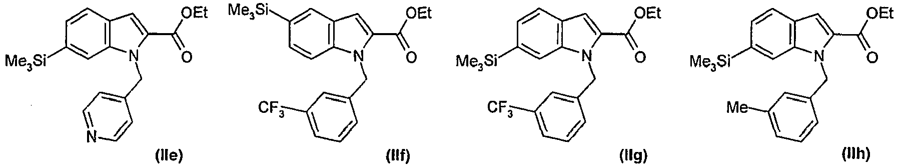

Les esters (IIa), (IIb), (IIc), (IId), (IIe), (IIf), (IIg) et (IIh) sont préparés selon les procédés décrits dans les exemples n° 18, 22, 25, 28, 31, 32, 35 et 37.

Les exemples suivants décrivent la préparation de certains composés conformes à l'invention. Ces exemples ne sont pas limitatifs et ne font qu'illustrer la présente invention. Les numéros des composés exemplifiés renvoient à ceux du tableau 1. Les microanalyses élémentaires, les analyses LC-MS (chromatographie liquide couplée à la spectrométrie de masse) et les spectres I.R. ou R.M.N. confirment les structures des composés obtenus.

### Exemple 1 (Composé N°1)

### N-(2,3-Diméthyl-3H-imidazo[4,5-b]pyridin-6-yl)-5-fluoro-1-(3-fluorobenzyl)-1H-indole-2-carboxamide

### 1.1 Acide 5-fluoro-1-(3-fluorobenzyl)-1H-indole-2-carboxylique

On ajoute une solution aqueuse de soude, préparée à partir de 1,15 g (28,92 mmoles) de pastille de soude dans 50 mL d'eau, à une solution de 7,6 g (24,10 mmoles) de 5-fluoro-1-(3-fluorobenzyl)-1*H*-indole-2-carboxylate d'éthyle (WO2006/024776) dans 241 mL d'éthanol. Le mélange est chauffé durant deux heures puis concentré sous pression réduite. Le solide résultant est repris dans 200 mL d'eau. La solution est lavée avec deux fois 100 mL d'éther éthylique, acidifiée par ajouts successifs de petites quantités d'acide chlorhydrique concentré puis extraite avec 200 mL d'acétate d'éthyle. La phase organique est finalement lavée deux fois avec 100 mL d'eau, une fois avec 50 ml d'une solution saturée en chlorure de sodium, séchée sur sulfate de magnésium et concentrée sous pression réduite. On obtient, après séchage à 50°C sous pression réduite, 6,4 g du produit attendu sous la forme d'un solide qui sera utilisé tel quel dans la suite de la synthèse.

### 1.2 N-(2,3-Diméthyl-3H-imidazo[4,5-b]pyridin-6-yl)-5-fluoro-1-(3-fluorobenzyl)-1H-indole-2-carboxamide (Composé n°1)

A une solution, agitée à 20°C, de 0,25 g (0,87 mmole) d'acide 5-fluoro-1-(3-fluorobenzyl)-1*H*-indole-2-carboxylique préparé à l'étape 1.1, de 183 mg (0,96 mmole) de chlorhydrate de *N*-(3-diméthylaminopropyl)-*N*'-éthylcarbodiimide (EDAC) et de 129 mg (0,96 mmole) de 1-hydroxy-benzotriazole (HOBT) dans 8 mL de DMF sont ajoutés 130 microlitres (0,96 mole) de triéthylamine puis 224 mg (1,13 mmole) de chlorhydrate de 6-amino-2,3-diméthyl-3*H*-imidazo[4,5-*b*]pyridine (Ukrainskii Khimicheskii Zhurnal 1981, 47, 867). Le mélange réactionnel est agité 8 heures à 20°C puis est concentré sous pression réduite. Le produit résultant est repris par 100 mL d'eau et on recueille, par filtration, un précipité que l'on purifie par chromatographie sur colonne de silice en éluant avec un mélange de dichlorométhane et de méthanol. Les fractions contenant le produit sont réunies et évaporées au quart du volume. On ajoute 50 mL de n-heptane à la solution et on recueille par filtration un solide que l'on sèche sous pression réduite. On isole ainsi 150 mg du produit attendu.
PF = 274 - 275°C
R.M.N. ¹H (DMSO-D₆), δ ppm : 10,59 (s, 1 H) ; 8,59 (s, 1 H) ; 8,3 (s, 1H) ; 7,6 (m, 2H) ; 7,46 (s, 1 H) ; 7,32 (m, 1 H) ; 7,19 (m, 1 H) ; 7,07 (m, 1 H) ; 6,92 (m, 2H) ; 5,91 (s, 2H) ; 3,78 (s, 3H); 2,6 (s, 3H).

### Exemple 2 (Composé N°2)

### N-(2-Méthyl-thiazolo[5,4-b]pyridin-6-yl)-5-fluoro-1-(3-fluorobenzyl)-1H-indole-2-carboxamide.

On procède selon une méthode similaire à celle dans l'exemple 1.2 à partir de 0,5 g (1,74 mmole) d'acide 5-fluoro-1-(3-fluorobenzyl)-1*H*-indole-2-carboxylique préparé à l'étape 1.1 et de 0,345 g (2,09 mmoles) de 6-amino-2-méthyl-thiazolo[5,4-*b*]pyridine (Yakugaku Zasshi 1950, 70, 187). En fin de réaction, le produit est concentré sous pression réduite puis repris par 100 mL d'eau. On recueille, par filtration, un précipité que l'on purifie par chromatographie sur colonne d'alumine en éluant avec un mélange de dichlorométhane et de méthanol. Le produit résultant est recristallisé dans le méthanol et on recueille par filtration un solide que l'on sèche sous pression réduite. On isole ainsi 340 mg du produit attendu.
PF = 264 - 265 °C
R.M.N. ¹H (DMSO-D₆), δ ppm : 10,85 (s, 1H) ; 8,9 (s, 1H) ; 8,67 (s, 1 H) ; 7,61 (m, 2H) ; 7,51 (s, 1 H) ; 7,31 (m, 1 H) ; 7,2 (m, 1 H) ; 7,05 (m, 1 H) ; 6,9 (m, 2H) ; 5,9 (s, 2H) ; 2,87 (s, 3H).

### Exemple 3 (Composé N°3)

### N-(Thiazolo[5,4-b]pyridin-6-yl)-5-fluoro-1-(3-fluorobenzyl)-1H-indole-2-carboxamide

On procède selon une méthode similaire à celle de l'exemple 1.2 à partir de 0,32 g (1,11 mmole) d'acide 5-fluoro-1-(3-fluorobenzyl)-1*H*-indole-2-carboxylique préparé à l'étape 1.1 et de 0,2021 g (1,34 mmole) de 6-amino-thiazolo[5,4-*b*]pyridine (WO2007/100758). En fin de réaction, le produit est concentré sous pression réduite puis repris par 100 mL d'eau. On recueille par filtration un précipité que l'on purifie par chromatographie sur colonne d'alumine en éluant avec un mélange de dichlorométhane et de méthanol. Le produit résultant est recristallisé dans un mélange de dichlorométhane et de n-heptane. On isole ainsi 30 mg du produit attendu.
PF = 250 - 251 °C
R.M.N. ¹H (DMSO-D₆), δ ppm : 10,9 (s, 1H) ; 9,57 (s, 1H) ; 9 (s, 1H) ; 8,98 (s, 1H) ; 7,61 (m, 2H) ; 7,51 (s, 1 H) ; 7,31 (m, 1 H) ; 7,2 (m, 1 H) ; 7,08 (m, 1H) ; 6,92 (m, 2H) ; 5,91 (s, 2H).

### Exemple 4 (Composé N°13)

### 2,2-Diméthylpropanoate de [6-[[[5-fluoro-1-[(3-fluorophényl)méthyl]-1H-indol-2-yl]carbonyl]amino]thiazolo[5,4-b]pyridin-2-yl]méthyle

### 4.1 2,2-Diméthyl-propanoate de (6-nitro-thiazolo[5,4-b]pyridin-2-yl)méthyle

On chauffe 2 heures à 105 °C un mélange de 6 g (29,48 mmoles) de 2-chloro-3,5-dinitropyridine et de 7,74 g (44,22 mmoles) de pivalate de 2-amino-2-thioxoéthyle dans 50 mL de sulfolane. Après ce temps, on ajoute au mélange 150 mL d'acétate d'éthyle et la phase organique est lavée 3 fois avec 200 mL d'eau puis une fois avec 100 mL d'une solution saturée en chlorure de sodium. La phase organique est ensuite séparée, séchée sur sulfate de sodium puis concentrée sous pression réduite. Le produit obtenu est purifié par chromatographie sur colonne de silice en éluant avec un mélange d'heptane et d'acétate d'éthyle. On obtient 1,45 g d'un solide jaune.
R.M.N. ¹H (CDCl₃), δ ppm : 9,55 (d, 1H) ; 9,09 (d, 1H) ; 5,59 (s, 2H) ; 1,38 (s, 9H).

### 4.2 2,2-Diméthyl-propanoate de (6-amino-thiazolo[5,4-b]pyridin-2-yl)méthyle

On agite 30 minutes à 50°C un mélange de 0,75 g (2,54 mmoles) de 2,2-Diméthyl-propanoate de (6-amino-thiazolo[5,4-*b*]pyridin-2-yl)méthyle, préparé à l'étape 4.1, et de 1,68 g (8,89 mmoles) de chlorure d'étain dans 50 mL d'acétate d'éthyle. Après ce temps, le mélange réactionnel est versé sur 100 mL d'eau glacée. Le pH de la solution aqueuse est neutralisé par ajouts successifs d'une solution de soude. On extrait ensuite la phase aqueuse avec 3 fois 50 mL d'acétate d'éthyle. Les phases organiques sont réunies, séchées puis concentrées sous pression réduite. On obtient ainsi 0,7 g d'un solide qui sera utilisé tel quel dans la suite de la synthèse.
R.M.N. ¹H (CDCl₃), δ ppm : 8,05 (d, 1H) ; 7,42 (d, 1 H) ; 5,37 (s, 2H) ; 1,21 (s, 9H).

### 4.3 2,2-Diméthylpropanoate de [6-[[[5-fluoro-1-[(3-fluorophényl)méthyl]-1H-indol-2-yl]carbonyl]amino]thiazolo[5,4-b]pyridin-2-yl]méthyle (Composé N° 13)

On verse, goutte à goutte à 0°C, 240 microlitres (2,78 mmoles) de chlorure d'oxalyle sur une solution de 0,4 g (1,39 mmole) d'acide 5-fluoro-1-[(3-fluorophényl)méthyl]-1*H-*indole-2-carboxylique, préparé à l'étape 1.1, dans 10 mL de dichlorométhane et 0,5 mL de diméthylformamide. Le mélange est agité 15 minutes à 0° puis 1 heure à 20°C. Après ce temps, on additionne 120 microlitres (1,39 mmole) de chlorure d'oxalyle supplémentaire et l'agitation est maintenue 30 minutes à 20°C. Le mélange réactionnel est ensuite concentré sous pression réduite et repris dans 5 mL de tétrahydrofurane. On addtionne, sous atmosphère inerte, 290 microlitres de triéthylamine puis une solution de 0,44 g (1,67 mmole) de 2,2-Diméthyl-propanoate de (6-amino-thiazolo[5,4-*b*]pyridin-2-yl)méthyle, préparé à l'étape 4.2, dans 10 mL de tétrahydrofurane. Le mélange réactionnel est agité 15 heures à 20°C, une heure à 40°C puis concentré sous pression réduite et repris par 100 mL d'eau. La phase aqueuse est extraite trois fois avec 50 mL d'acétate d'éthyle. Les phases organiques sont réunies, successivement lavées avec 50 mL d'une solution saturée en hydrogénocarbonate de sodium, 50 mL d'une solution saturée en chlorure de sodium puis séchées sur sulfate de sodium et concentrées sous pression réduite. Le produit ainsi obtenu est purifié sur colonne d'alumine en éluant avec du dichlorométhane. On isole ainsi 0,15 g du produit attendu.
PF = 224 - 225°C
R.M.N. ¹H (DMSO-D₆), δ ppm : 8,89 (s, 1H) ; 8,71 (s, 1 H) ; 7,52 (m, 2H) ; 7,45 (s, 1H) ; 7,29 (m, 1 H) ; 7,15 (m, 1 H) ; 7,00 (m, 1 H) ; 6,9 (m, 1 H) ; 6,81 (m, 1H) ; 5,86 (s, 2H) ; 5,49 (s, 2H) ; 1,12 (s, 9H).

### Exemple 5 (Composé N°5)

### N-(Quinoléin-3-yl)-5-fluoro-1-(3-fluorobenzyl)-1H-indole-2-carboxamide

On ajoute, goutte à goutte, 1,19 mL (2,38 mmoles) d'une solution de triméthylaluminium 2M dans le toluène sur une solution, agitée à 0°C sous atmosphère inerte, de 0,274 g (1,9 mmole) de 3-aminoquinoléine dans 40 mL de toluène sec. Le mélange est ensuite agité à 50°C. Après 15 minutes on ajoute, goutte à goutte, une solution de 0,5 g (1,59 mmole) de 5-fluoro-1-(3-fluorobenzyl)-1*H*-indole-2-carboxylate d'éthyle (WO2006/024776) dans 10 mL de toluène. On agite le mélange réactionel 18 heures à reflux puis on verse la solution refroidie sur 20 mL d'eau. On ajoute au mélange 15 mL d'une solution d'acide chlorhydrique 1 N et 100 mL d'acétate d'éthyle. La phase organique est séparée puis successivement lavée avec 20 mL d'une d'une solution d'acide chlorhydrique 1N, 20 mL d'eau et 20 mL d'une solution saturée en chlorure de sodium puis séchée sur sulfate de sodium et concentrée sous pression réduite. Le produit ainsi obtenu est trituré dans 10 mL de toluène chaud. On recueille un précipité par filtration. Après séchage, on isole ainsi 295 mg du produit attendu.
PF = 244 - 246 °C
R.M.N. ¹H (CDCl₃), δ ppm : 10,91 (s, 1H) ; 9,15 (d, 1H) ; 8,88 (d, 1H) ; 8 (m, 2H) ; 7,62 (m, 5H) ; 7,32 (m, 1 H) ; 7,21 (m, 1 H) ; 7,08 (m, 1 H) ; 6,92 (m, 2H) ; 5,95 (s, 2H).

### Exemple 6 (Composé N°4)

### N-(2-Hydroxyméthyl-thiazolo[5,4-b]pyridin-6-yl)-5-fluoro-1-[(3-fluorophényl)méthyl]-1H-indole-2-carboxamide

On additionne 50 mg (2,03 mmoles) de sodium à 8 mL de méthanol sous agitation à 20°C. Après 15 minutes, on additionne une solution de 0,15 g (0,28 mmole) de 2,2-diméthylpropanoate de [6-[[[5-fluoro-1-[(3-fluorophényl)méthyl]-1*H*-indol-2-yl]carbonyl]amino]thiazolo[5,4-*b*]pyridin-2-yl]méthyle n°13, préparé à l'étape 4.3, dans 16 mL de méthanol. L'agitation à 20°C est maintenue 15 minutes, puis le mélange réactionnel est concentré sous pression réduite puis repris par 200 mL d'une solution molaire de chlorure d'ammonium. La phase aqueuse est extraite trois fois avec 50 mL d'acétate d'éthyle. Les phases organiques sont réunies, lavées avec 50 mL d'une solution saturée en chlorure de sodium puis séchées sur sulfate de sodium et concentrées sous pression réduite. Le produit ainsi obtenu est purifié sur colonne de silice en éluant avec un mélange de dichlorométhane et de méthanol. On isole ainsi 70 mg du produit attendu.
PF = 225 - 226°C
R.M.N. ¹H (CDCl₃), δ ppm : 10,84 (s, 1 H) ; 8,92 (d, 1H) ; 8,72 (d, 1H) ; 7,61 (m, 2H) ; 7,5 (s, 1H) ; 7,35 (m, 1H) ; 7,2 (m, 1 H) ; 7,08 (m, 1H) ; 6,92 (m, 2H) ; 6,37 (m, 1H) ; 5,91 (s, 2H) ; 4,9 (s, 2H).

### Exemple 7 (Composé N°6)

### N-(2-Hydroxyméthyl-3-méthyl-3H-imidazo[4,5-b]pyridin-6-yl)-5-fluoro-1-[(3-fluorophényl)méthyl]-1H-indole-2-carboxamide

### 7.1 2-Hydroxyméthyl-6-nitro-3-méthyl-3H-imidazo[4,5-b]pyridine et 3-méthyl-6-nitro -3H-imidazo[4,5-b]pyridine.

On chauffe à 150°C un mélange de 3,1 g (18,44 mmoles) de 3-amino-5-nitro-2-méthylaminopyridine et de 3,5 g (46,09 mmoles) d'acide glycolique. Après 75 minutes le mélange est refroidi puis dissout dans 50 mL d'acide chlorhydrique 1 N. On ajuste le pH de la phase aqueuse à 9, par ajouts successifs de soude concentrée, puis on concentre le mélange sous pression réduite. Le solide résultant est purifié par chromatographie sur colonne de silice en éluant avec un mélange de dichlorométhane et de méthanol. On isole ainsi 1,5 g du produit attendu. Cette purification permet également d'isoler 0,2 g de 3-méthyl-6-nitro-3*H*-imidazo[4,5-*b*]pyridine. 2-Hydroxyméthyl-6-nitro-3-méthyl-3*H*-imidazo[4,5-*b*]pyridine :
LCMS : [MH]⁺ = 209
R.M.N. ¹H (DMSO-D₆), δ ppm : 9,3 (d, 1H) ; 8,89 (d, 1 H) ; 5,88 (m, 1H) ; 4,83 (m, 2H) ; 3,95 (t, 3H).
3-méthyl-6-nitro -3*H*-imidazo[4,5-b]pyridine:
LCMS : [MH]⁺ = 179
R.M.N. ¹H (DMSO-D₆), δ ppm : 9,31 (d, 1H) ; 8,95 (d, 1H) ; 8,79 (s, 1H) ; 3,95 (t, 3H).

### 7.2 6-Amino-2-hydroxyméthyl-3-méthyl-3H-imidazo[4,5-b]pyridine

On agite 3 heures sous 2,5 atmosphère d'hydrogène, une suspension de 0,5 g (2,4 moles) de 2-hydroxyméthyl-6-nitro-3-méthyl-3*H*-imidazo[4,5-*b*]pyridine, préparé à l'étape précédente, et 0,3 g de palladium sur charbon à 10%. Après ce temps, la suspension est filtrée sur un tampon de célite et le filtrat est concentré sous pression réduite. On isole ainsi 0,4 g du produit attendu qui sera utilisé tel quel dans la suite de la synthèse.
LCMS : [MH]⁺ = 179

### 7.3 N-(2-Hydroxyméthyl-3-méthyl-3H-imidazo[4,5-b]pyridin-6-yl)-5-fluoro-1-[(3-fluorophényl)méthyl]-1H-indole-2-carboxamide (Composé N°6)

Le composé N° 6 est synthétisé selon un procédé similaire à celui décrit dans l'exemple 1.2, à partir de 6-amino-2-hydroxyméthyl-3-méthyl-3*H*-imidazo[4,5-*b*]pyridine décrit à l'étape précédente et d'acide 5-fluoro-1-[(3-fluorophényl)méthyl]-1*H*-indole-2-carboxylique décrit à l'étape 1.1.
PF : 231 - 232 °C
R.M.N. ¹H (DMSO-D₆), δ ppm : 10,67 (s, 1H) ; 8,68 (s, 1H) ; 8,4 (s, 1H) ; 7,6 (m, 2H) ; 7,48 (s, 1H) ; 7,33 (m, 1 H) ; 7,18 (txd, 1 H) ; 7,055 (txd, 1H) ; 6,94 (m, 2H) ; 5,92 (s, 2H) ; 5,69 (t, 1H) ; 4,8 (d, 2H) ; 3,88 (s, 3H)

### Exemple 8 (Composé N°7)

### N-(3-Méthyl-3H-imidazo[4,5-b]pyridin-6-yl)-5-fluoro-1-[(3-fluorophényl)méthyl]-1H-indole-2-carboxamide

### 8.1 6-Amino-3-méthyl-3H-imidazo[4,5-b]pyridine

Ce composé est préparé selon un procédé similaire à celui décrit à l'étape 7.2 à partir de la 3-méthyl-6-nitro-3*H*-imidazo[4,5-*b*]pyridine préparée à l'étape 7.1.
LCMS : [MH]⁺ = 149

### 8.3 N-(3-Méthyl-3H-imidazo[4,5-b]pyridin-6-yl)-5-fluoro-1-[(3-fluorophényl)méthyl]-1H-indole-2-carboxamide (composé N°7)

Le composé N° 7 est synthétisé selon un procédé similaire à celui décrit dans l'exemple 1.2, à partir de 6-amino-3-méthyl-3*H*-imidazo[4,5-*b*]pyridine, décrit à l'étape précédente, et d'acide 5-fluoro-1-[(3-fluorophényl)méthyl]-1*H*-indole-2-carboxylique, décrit à l'étape 1.1.
PF : 235 - 236 °C
R.M.N. ¹H (DMSO-D₆), δ ppm : 10,68 (s, 1H) ; 8,69 (s, 1H) ; 8,42 (m, 2H) ; 7,6 (m, 2H) ; 7,49 (s, 1 H) ; 7,32 (m, 1 H) ; 7,19 (m, 1 H) ; 7,06 (m, 1 H) ; 6,92 (m, 2H) ; 5,91 (s, 2H) ; 3,87 (s, 3H).

### Exemple 9 (Composé N°8)

### N-(2,3-Diméthyl-3H-imidazo[4,5-b]pyridin-6-yl)-5-trifluorométhyl-1-[(3-fluorophényl)méthyl]-1H-pyrrolo[2,3-b]pyridine-2-carboxamide

Ce composé a été préparé selon un procédé similaire à celui décrit à l'exemple 1.2 à partir de l'acide 5-trifluorométhyl-1-[(3-fluorophényl)méthyl]-1 H-pyrrolo[2,3-b]pyridine-2-carboxylique (WO2008093024).
PF : 274 - 275 °C
R.M.N. ¹H (DMSO-D₆), δ ppm : 10,75 (s, 1H) ; 8,83 (s, 1H) ; 8,76 (s, 1H) ; 8,55 (s, 1H) ; 8,28 (s, 1 H) ; 7,63 (s, 1 H) ; 7,32 (m, 1 H) ; 7,07 (m, 1H) ; 6,98 (m, 2H) ; 6,01 (s, 2H) ; 3,76 (s, 3H) ; 2,6 (s, 3H).

### Exemple 10 (Composé N°9)

### N-(3,4-Dihydro-2H-pyrido[3,2-b][1,4]oxazin-7-yl)-5-fluoro-1-[(3-fluorophényl)méthyl]-1H-indole-2-carboxamide

### 10.1 5-fluoro-1-[(3-fluorophényl)méthyl]-1H-indole-2-carboxamide

A une suspension, agitée à 20°C, de 2 g (6,96 mmoles) d'acide 5-fluoro-1-[(3-fluorophényl)méthyl]-1*H*-indole-2-carboxylique préparé à l'étape 1.1, dans 80 mL de toluène sec, sont ajoutés 5,08 mL (69,62 mmoles) de chlorure de thionyle. Le mélange réactionnel est agité 2 heures à reflux puis est concentré sous pression réduite. Le produit résultant est repris par 10 mL de dichlorométhane et on verse cette solution, goutte à goutte, sur une solution de 9,12 mL (69,62 mmoles) d'ammoniaque à 30% dans l'eau. Le mélange réactionnel est agité 14 heures à 20°C. Après ce temps, on recueille, par filtration, un solide que l'on triture dans 50 mL d'éther diisopropylique. On recueille après filtration et séchage sous pression réduite 0,58 g de produit attendu. R.M.N. ¹H (DMSO-D₆), δ ppm : 8,11 (pic élargi, 1H) ; 7,5 (m, 3H) ; 7,32 (m, 1H) ; 7,25 (s, 1 H) ; 7,09 (m, 2H) ; 6,89 (m, 2H) ; 5,91 (s, 2H).

### 10.2 N-(3,4-Dihydro-2H-pyrido[3,2-b][1,4]oxazin-7-yl)-5-fluoro-1-[(3-fluorophényl)méthyl]-1H-indole-2-carboxamide (composé n° 9)

Dans un tube à pression muni d'un agitateur magnétique, on introduit 0,4 g (1,4 mmole) de l'amide préparée à l'étape précédente, 0,31 g (1,47 mmole) de 7-bromo-3,4-dihydro-2*H*-pyrido[3,2-*b*][1,4]oxazine, 0,08 g (0,42 mmole) d'iodure de cuivre, 0,39 g (2,79 mmoles) de carbonate de potassium et 10 mL de dioxane sec. La suspension est dégazée puis on ajoute 53 mg (0,46 mmole) de trans-1,2-cyclohexanediamine, on ferme le tube et on chauffe à 120°C sous agitation durant 16 heures. Après ce temps, on ajoute au milieu 50 mL d'acétate d'éthyle et 50 mL d'eau. La phase aqueuse est séparée puis extraite avec 2x30 mL d'acétate d'éthyle. Les phases organiques sont réunies, lavées avec 50 mL d'eau, séchées sur sulfate de sodium puis concentrées sous pression réduite. Le produit résultant est purifié par chromatographie sur colonne de silice, en éluant avec un mélange d'heptane et d'acétate d'éthyle, puis par cristallisation dans un mélange d'heptane et de dichlorométhane. On isole ainsi 0,4 g de produit attendu.
PF : 265-266°C
R.M.N. ¹H (DMSO D₆), δ (ppm) : 10,22 (s, 1H) ; 7,98 (s, 1H) ; 7,58 (m, 2H) ; 7,34 (m, 3H) ; 7,18 (m, 1 H) ; 7,06 (m, 1H) ; 6,91 (m, 2H) ; 6,58 (s, 1H) ; 5,9 (s, 2H) ; 4,14 (m, 2H) ; 3,4 (m, 2H).

### Exemple 11 (Composé N°10)

### N-(4-Méthyl-3,4-dihydro-2H-pyrido[3,2-b][1,4]oxazin-7-yl)-5-fluoro-1-[(3-fluorophényl)méthyl]-1H-indole-2-carboxamide

Ce composé a été préparé selon un procédé similaire à celui décrit à l'exemple 10.2 à partir de 7-amino-4-méthyl-3,4-dihydro-2*H*-pyrido[3,2-*b*][1,4]oxazine.
PF : 214-215 °C
R.M.N. ¹H (DMSO D₆), δ (ppm) : 10,29 (s, 1 H) ; 8,03 (s, 1H) ; 7,58 (m, 2H) ; 7,34 (m, 3H) ; 7,18 (m, 1H) ; 7,05 (m, 1H) ; 6,91 (m, 2H) ; 5,9 (s, 2H) ; 4,26 (m, 2H) ; 3,41 (m, 2H) ; 3,02 (s, 3H).

### Exemple 12 (Composé N°11)

### N-(2-Méthyl-3H-imidazo[4,5-b]pyridin-6-yl)-5-fluoro-1-[(3-fluorophényl)méthyl]-1H-indole-2-carboxamide

### 12.1 2-Méthyl-6-nitro-3H-imidazo[4,5-b]pyridine

On ajoute, à une solution agitée à 15°C de 1,5 g (9,73 mmoles) de 2,3-diamino-5-nitro-pyridine dans 15 mL d'acide acétique, 2,3 mL (24,33 moles) d'anhydride acétique. Après 15 minutes d'agitation à température ambiante, le mélange est chauffé à 110 °C durant 2 heures puis à 140°C durant 7 heures. Le mélange résultant est concentré sous pression réduite et repris dans 100 mL d'eau. On recueille un précipité par filtration. Après purification par chromatographie sur colonne de silice, on obtient 0,4 g du produit attendu.
LCMS : [MH]⁺ = 179
R.M.N. ¹H (DMSO D₆), δ (ppm) : 9,2 (d, 1H) ; 8,7 (d, 1H) ; 2,62 (s, 3H).

### 12.2 6-Amino-2-méthyl-3H-imidazo[4,5-b]pyridine

Ce composé est préparé selon un procédé similaire à celui décrit à l'étape 7.2 à partir de la 2-Méthyl-6-nitro-3*H*-imidazo[4,5-*b*]pyridine préparée à l'étape 12.1.
LCMS : [MH]⁺ = 149
R.M.N. ¹H (DMSO D₆), δ (ppm) : 7,7 (d, 1H) ; 6,99 (d, 1 H) ; 2,4 (s, 3H).

### 12.3 N-(2-Méthyl-3H-imidazo[4,5-b]pyridin-6-yl)-5-fluoro-1-[(3-fluorophényl)méthyl]-1H-indole-2-carboxamide (Composé N°11).

Ce composé a été préparé selon un procédé similaire à celui décrit à l'exemple 1.2 à partir de l'acide 5-fluoro-1-[(3-fluorophényl)méthyl]-1*H*-indole-2-carboxylique, préparé à l'exemple 1.1 et de la 6-amino-2-méthyl-3*H*-imidazo[4,5-b]pyridine, préparée à l'étape précédente.
PF : 299 - 300 °C
R.M.N. ¹H (DMSO D₆), δ (ppm) : 12,37 (s, 1H) ; 10,6 (s, 1H) ; 8,53 (s, 1H) ; 8,29 (m, 1 H) ; 7,61 (dxd, 1H) ; 7,58 (dxd, 1H) ; 7,46 (s, 1 H) ; 7,33 (m, 1H) ; 7,18 (txd, 1H) ; 7,05 (txd, 1H) ; 6,94 (m, 2H) ; 5,9 (s, 2H) ; 2,48 (s, 3H).

### Exemple 13 (Composé N°12)

### N-(1-Méthyl-2-oxo-2,3-dihydro-1H-pyrido[2,3-b][1,4]oxazin-7-yl)-5-fluoro-1-[(3-fluorophényl)méthyl]-1H-indole-2-carboxamide

### 13.1 7-Bromo-1-méthyl-2-oxo-2,3-dihydro-1H-pyrido[2,3-b][1,4]oxazine

On ajoute 0,16 mL (2,62 mmoles) d'iodure de méthyle sur une suspension de 0,5 g (2,18 moles) de 7-bromo-2-oxo-2,3-dihydro-1*H*-pyrido[2,3-*b*][1,4]oxazine, 0,24 g (1,09 mmole) de chlorure de benzyltriéthylammonium et de 0,75 g (5,46 mmoles) de carbonate de potassium dans 25 mL d'acétonitrile. Le mélange est agité 6 heures à 60°C. Après ce temps, on élimine un précipité par filtration. Le filtrat est concentré sous pression réduite et le solide résultant est repris dans 50 mL de dichlorométhane puis lavé successivement avec 20 ml d'eau, 10 mL d'une solution d'acide chlorhydrique 0,1N, 10 mL d'une solution saturée en hydrogénocarbonate de sodium puis 10 mL d'eau. Après séchage sur sulfate de sodium, la solution est finalement concentrée sous pression réduite pour obtenir le produit attendu qui sera utilisé tel quel dans l'étape suivante.
R.M.N. ¹H (DMSO D₆), δ (ppm) : 7,98 (d, 1H) ; 7,79 (d, 1H) ; 4,9 (s, 2H) ; 3,29 (s, 3H).

### 13.2 N-(1-Méthyl-2-oxo-2,3-dihydro-1H-pyrido[2,3-b][1,4]oxazin-7-yl)-5-fluoro-1-[(3-fluorophényl)méthyl]-1H-indole-2-carboxamide (Composé N°12)

Ce composé a été préparé selon un procédé similaire à celui décrit à l'exemple 10.2 à partir de 7-bromo-1-méthyl-2-oxo-2,3-dihydro-1*H*-pyrido[2,3-*b*][1,4]oxazine préparé à l'étape précédente.
PF : 248 - 250 °C
R.M.N. ¹H (DMSO D₆), δ (ppm) : 10,61 (s, 1H) ; 8,23 (d, 1H) ; 7,91 (d, 1H) ; 7,59 (txd, 2H) ; 7,46 (s, 1H) ; 7,33 (m, 1H) ; 7,19 (txd, 1H) ; 7,06 (txd, 1H) ; 6,9 (m, 2H) ; 5,91 (s, 2H) ; 4,87 (s, 2H) ; 3,25 (s, 3H).

### Exemple 14 (Composé N°14)

### N-(2,3-dihydro-[1,4]dioxino[2,3-b]pyridin-7-yl))-5-fluoro-1-[(3-fluorophényl)méthyl]-1H-indole-2-carboxamide

Ce composé a été préparé selon un procédé similaire à celui décrit à l'exemple 10.2 à partir de 7-bromo-2,3-dihydro-[1,4]dioxino[2,3-*b*]pyridine (WO2003087098).
PF : 224 - 225 °C
R.M.N. ¹H (DMSO D₆), δ (ppm) : 10,49 (s, 1 H) ; 8,1 (s, 1 H) ; 7,71 (s, 1H) ; 7,59 (m, 2H) ; 7,41 (s, 1H) ; 7,31 (m, 1H) ; 7,18 (m, 1H) ; 7,04 (m, 1H) ; 6,91 (m, 2H) ; 5,89 (s, 2H) ; 4,4 (m, 2H) ; 3,88 (m, 2H).

### Exemple 15 (Composé N°15)

### N-(6-Hydroxy-5,6,7,8-tétrahydroquinoléin-3-yl)-5-fluoro-1-[(3-fluorophényl)méthyl]-1H-indole-2-carboxamide

Ce composé a été préparé selon un procédé similaire à celui décrit à l'exemple 1.2 à partir de la 3-amino-6-hydroxy-5,6,7,8-tétrahydroquinoléine (WO2007100758).
PF : 204 - 206 °C
R.M.N. ¹H (DMSO D₆), δ (ppm) : 10,51 (s, 1H) ; 8,61 (s, 1H) ; 7,89 (s, 1H) ; 7,59 (m, 2H) ; 7,43 (s, 1H) ; 7,32 (m, 1H) ; 7,19 (m, 1H) ; 7,04 (m, 1H) ; 6,90 (m, 2H) ; 5,90 (s, 2H) ; 4,82 (d, 1H) ; 4,01 (m, 1H) ; 2,92 (m, 2H) ; 2,78 (m, 1H) ; 2,62 (m, 1H) ; 1,93 (m, 1H) ; 1,79 (m, 1 H).

### Exemple 16 (Composé N°96)

### N-(2-Hydroxyméthyl-thiazolo[5,4-b]pyridin-6-yl)-5-trifluorométhyl-1-[(3-méthylphényl)méthyl]-1H-indole-2-carboxamide

**16.1** 5-Trifluorométhyl-1-[(3-méthylphényl)méthyl]-1*H*-indole-2-carboxylate d'éthyle Ce composé a été préparé en faisant réagir 333 mg (1,295 mmole) de 5-trifluorométhyl-1*H*-indole-2-carboxylate d'éthyle avec 0,31 mL (2,59 mmoles) de 3-méthylphénylméthanol en présence de 0,92 g (3,826 mmoles) de (cyanométhylène)tributylphosphorane (CMBP). Le mélange réactionnel est agité à 110°C pendant 15 heures puis concentré à sec. Le brut réactionnel est ensuite purifié par chromatographie flash sur colonne de gel de silice dans un mélange d'heptane et d'acétate d'éthyle pour donner 376 mg du produit attendu sous la forme d'une huile. R.M.N. ¹H (DMSO D₆), δ (ppm) : 8,18 (s, 1H) ; 7,81-7,78 (m, 1H) ; 7,61-7,57 (m, 1H), 7,53 (s, 1H) ; 7,14-7,12 (m, 2H) ; 7,01-6,91 (m, 1H) ; 6,75-6,73 (m, 1H) ; 5,88 (s, 2H) ; 4,31 (q, 2H) ; 2,21 (s, 3 H) ; 1,30 (t, 3H).
LC-MS: 362([M+H]⁺)

### 16.2 N-(2-Hydroxyméthyl-thiazolo[5,4-b]pyridin-6-yl)-5-trifluorométhyl-1-[(3-méthylphényl)méthyl]-1H-indole-2-carboxamide (Composé N°16)

A une solution de 150 mg (0,415 mmole) de 5-trifluorométhyl-1-[(3-méthylphényl)méthyl]-1*H*-indole-2-carboxylate d'éthyle préparé selon le protocole décrit à l'étape précédente et de 132 mg (0,498 mmole) de 2,2-diméthyl-propanoate de (6-amino-thiazolo[5,4-*b*]pyridin-2-yl)méthyle, obtenu à l'étape 4.2, dans 1,5 mL de toluène sec, maintenue sous atmosphère inerte, est ajoutée goutte à goutte, à 0°C, 0,31 mL (0,623 mmoles) d'une solution de triméthylaluminium (2M / toluène). Le mélange réactionnel est agité à 110°C pendant 15 heures puis concentré à sec. Le brut réactionnel est ensuite dilué avec une solution normale d'acide chlorhydrique. Le produit est extrait avec de l'acétate d'éthyle puis purifié par chromatographie sur colonne de silice en éluant avec un mélange d'heptane et d'acétate d'éthyle pour donner 82 mg du produit attendu.
PF : 315 - 316 °C
R.M.N. ¹H (DMSO D₆), δ (ppm) : 10,97 (s, 1H) ; 8,92 (d, 1H) ; 8,70 (d, 1H) ; 8,22 (s, 1H); 7,81 (d, 1 H) ; 7,62 (s, 1H) ; 7,60 (d, 1 H) ; 7,14 (t, 1 H) ; 7,03-6,97 (m, 2H) ; 6,87-6,84 (m, 1 H), 6,38 (t, 1 H) ; 5,91 (s, 2H) ; 4,87 (d, 2H) ; 2,19 (s, 3H).

### Exemple 17 (Composé N°17)

### N-(2-Hydroxyméthyl-thiazolo[5,4-b]pyridin-6-yl)-6-trifluorométhyl-1-[(3-méthylphényl)méthyl]-1H-indole-2-carboxamide

Le produit est préparé selon un procédé similaire à celui décrit à l'exemple 16.
PF : 221-222°C
R.M.N. ¹H (DMSO D₆), δ (ppm) : 11,0 (s, 1 H) ; 8,95 (s, 1 H) ; 8,7 (s, 1 H) ; 8,1 (s, 1H) ; 8,0 (d, 1 H) ; 7,6 (s, 1H) ; 7,5 (d, 1 H) ; 7,15 (t, 1H) ; 7,05 (m, 1 H) ; 6,95 (s, 1 H) ; 6,85 (m, 1H) ; 6,35 (t, 1H) ; 5,95 (s, 2H) ; 4,9 (d, 2H) ; 2,2 (s, 3H).

### Exemple 18 (Composé N°18)

### N-(2-Hydroxyméthyl-thiazolo[5,4-b]pyridin-6-yl)-5-triméthylsilyl-1-[(3-méthylphényl)méthyl]-1H-indole-2-carboxamide

### 18.1 1-Bromo-3-triméthylsilylbenzène

A une solution de 10 g (42,39 mmoles) de 1,3-dibromobenzène dans 80 mL d'Et₂O anhydre, refroidie à -78°C et maintenue sous atmosphère d'azote, sont ajoutés goutte à goutte sous agitation, durant 30 min, 26,49 mL (42,39 mmoles) d'une solution de BuLi (1,5M / hexane). Après 30 min d'agitation supplémentaire à -78°C, 5,96 mL (46,63 mmoles) de TMSCI sont introduits goutte à goutte dans le mélange réactionnel. L'agitation est maintenue à cette température pendant 90 min et le mélange réactionnel est ensuite hydrolysé par ajout de 15 mL d'eau. Le produit est extrait avec de l'acétate d'éthyle (3 x 50 mL). Les phases organiques rassemblées sont lavées avec une solution aqueuse saturée en NaCl (2 x 25 mL), séchées sur Na₂SO₄, filtrées et évaporées sous pression réduite. Le brut réactionnel est purifié par chromatographie sur colonne de gel de silice en éluant avec de l'heptane, pour isoler 9,3 g du 1-bromo-3-triméthylsilylbenzène attendu, sous la forme d'une huile incolore.
R.M.N. ¹H (DMSO D₆), δ (ppm) : 5,75 (s, 1H), 7,46 (m, 1 H), 7,4 (m, 1H), 7,22 (t, 1 H), 0,2 (s, 9H).

### 18.2 3-Timéthylsilylbenzaldehyde

A une solution de 5 g (21,89 mmoles) du 1-bromo-3-triméthylsilylbenzène préparé selon le protocole décrit à l'étape précédente, dans 40 ml d'Et₂O anhydre, refroidie à 0°C et maintenue sous atmosphère d'azote, sont ajoutés goutte à goutte, sous agitation et durant 30 min, 16,36 mL (26,18 mmoles) de BuLi (1,6M/hexane). L'agitation est poursuivie à 0°C pendant 30 min supplémentaire, puis maintenue à température ambiante pendant 90 min. 2,69 mL (34,91 mmoles) de DMF, dilués dans 17 mL d' Et₂O anhydre sont ensuite introduits dans le mélange réactionnel. Après 3h d'agitation à température ambiante, le mélange réactionnel est hydrolysé à 0°C par ajouts successifs de 10 mL d'une solution d'HCI concentré et de 100 mL d'eau. Le produit est extrait avec 3 x 50 mL de CH₂Cl₂. Les phases organiques rassemblées sont lavées avec 100 mL d'eau, séchées sur Na₂SO₄, filtrées et évaporées sous pression réduite. Le brut réactionnel est purifié par chromatographie flash sur colonne de gel de silice, en éluant avec un gradient de 10 à 20% de CH₂Cl₂ dans l'heptane pour donner 1,82 g de 3-triméthylsilylbenzaldéhyde attendu sous la forme d'une huile jaune.
R.M.N. ¹H (DMSO D₆), δ (ppm) : 10,01 (s, 1H) ; 8,0 (s, 1H) ; 7,85 (d, 1H) ; 7,8 (d, 1H) ; 7,5 (dd, 1 H) 0,3 (s, 9H)

### 18.3 2-Azido-3-(3-triméthylsilylphényl)propénoate d'éthyle

A une solution de 2 g (87,5 mmoles) de sodium dans 30 mL d'EtOH anhydre, maintenue sous atmosphère d'azote et refroidie à -10°C, est ajouté, goutte à goutte, un mélange de 31,4 mL (87,5 mmoles) d'azidoacétate d'éthyle (à 34% dans CH₂Cl₂) et 3,9 g (21,87 mmoles) du 3-triméthylsilylbenzaldehyde préparé selon la procédure décrite à l'étape précédente, dilué dans 3 mL d'EtOH. Le mélange réactionnel est agité à 0°C pendant 4 h. Il est ensuite hydrolysé par ajout sous agitation vigoureuse de 100 mL d'une solution aqueuse de NH₄Cl (30%). La phase aqueuse est extraite avec 3 x 50 mL d'AcOEt. Les phases organiques rassemblées sont lavées à l'eau, séchées sur Na₂SO₄ et concentrées sous pression réduite. Le brut réactionnel est purifié par chromatographie sur colonne de gel de silice, en éluant avec un mélange isocratique d'heptane et de CH₂Cl₂ (80/20). On isole ainsi 1,7 g du 2-azido-3-(3-triméthylsilylphényl)propénoate d'éthyle attendu, sous forme d'une huile jaune.
R.M.N. ¹H (DMSO D₆), δ (ppm) : 7,9 (d, 1H) ; 7,8 (s, 1H) ; 7,4 (d, 1H) ; 7,3 (dd, 1H) ; 6,9 (s, 1H) ; 4,2 (q, 2H) ; 1,2 (t, 3H) ; 0,15 (s, 9H)
MS: [MH]⁺ = 289

### 18.4 5-Triméthylsilyl-1H-indole-2-carboxylate d'éthyle

A une solution de 1,7 g (5,90 mmoles) du 2-azido-3-(3-triméthylsilylphényl)propénoate d'éthyle préparé selon la procédure décrite à l'étape précédente, dans 25 mL de toluène sec, maintenue sous atmosphère inerte, est ajoutée 0,62 g (0,59 mmoles) du complexe dimère d'heptafluorobutyrate de dirhodium (II). Le mélange réactionnel est agité pendant 7 h à 40°C. Une deuxième portion de 0,62 g (0,59 mmoles) du complexe dimère d'heptafluorobutyrate de dirhodium (II) est ajoutée au mélange réactionnel en maintenant l'agitation et le chauffage à 40°C pendant 1 h supplémentaire. Après retour à température ambiante, le mélange réactionnel est filtré sur gel de silice en éluant avec du toluène. Le filtrat est ensuite concentré sous pression réduite. Le solide verdâtre obtenu est trituré plusieurs fois dans un minimum d'heptane, jusqu'à obtenir une poudre blanche. Celle-ci est séchée sous pression réduite pour donner 0,87 g du 5-triméthylsilyl-1*H*-indole-2-carboxylate d'éthyle attendu, sous la forme d'une poudre blanche.
PF = 114-115°C
R.M.N. ¹H (DMSO D₆), δ (ppm) : 7,7 (s, 1 H) ; 7,35 (d, 1 H) ; 7,25 (d, 1 H) ; 7,0 (s, 1H) ; 4,2 (q, 2H) ; 1,2 (t, 3H) ; 0,15 (s, 9H)
LC-MS: [MH]⁻ = 260

### 18.5 5-triméthylsilyl-1-[(3-méthylphényl)méthyl]-1H-indole-2-carboxylate d'éthyle (Composé N° IIa)

Le produit est préparé selon un procédé similaire à celui décrit à l'exemple 16.1 R.M.N. ¹H (DMSO D₆), δ (ppm) : 7,87 (s, 1H) ; 7,55 (d, 1 H) ; 7,43 (d, 1 H) ; 7,36 (s, 1 H) ; 7,13 (t, 1 H) ; 7,01 (d, 1 H) ; 6,91 (s, 1H) ; 6,73 (d, 1 H) ; 5,80 (s, 2H) ; 4,29 (q, 2H) ; 2,21 (s, 3H) ; 1,29 (t, 3H) ; 0,26 (s, 9H).

### 18.6 N-(2-Hydroxyméthyl-thiazolo[5,4-b]pyridin-6-yl)-5-triméthylsilyl-1-[(3-méthylphényl)méthyl]-1H-indole-2-carboxamide (Composé N°18)

Le produit est préparé selon un procédé similaire à celui décrit à l'exemple 16.2 à partir du 5-triméthylsilyl-1-[(3-méthylphényl)méthyl]-1*H*-indole-2-carboxylate d'éthyle (composé N° IIa).
PF : 154-155°C
R.M.N. ¹H (DMSO D₆), δ (ppm) : 10,8 (s, 1 H) ; 8,95 (s, 1 H) ; 8,7 (s, 1 H) ; 7,9 (s, 1H) ; 7,6 (d, 1H) ; 7,45 (s, 1 H) ; 7,4 (d, 1 H) ; 7,15 (t, 1 H) ; 7,0 (m, 2H) ; 6,85 (d, 1 H) ; 6,35 (t, 1 H) ; 5,9 (s, 2H) ; 4,9 (d, 2H) ; 2,2 (s, 3H) ; 0,3 (s, 9H).

### Exemple 19 (Composé N°19)

### N-(2-Hydroxyméthyl-thiazolo[5,4-b]pyridin-6-yl)-6-fluoro-1-[(3-méthylphényl)méthyl]-1H-indole-2-carboxamide

Le produit est préparé selon un procédé similaire à celui décrit à l'exemple 16.
PF : 257-258°C
R.M.N. ¹H (DMSO D₆), δ (ppm) : 10,8 (s, 1H) ; 8,95 (s, 1H) ; 8,7 (s, 1H) ; 7,8 (m, 1H) ; 7,55 (s, 1 H) ; 7,45 (d, 1 H) ; 7,15 (t, 1 H) ; 7,05 (m, 2H) ; 6,95 (s, 1 H) ; 6,85 (m, 1H) ; 6,35 (t, 1 H) ; 5,9 (s, 2H) ; 4,9 (d, 2H) ; 2,2 (s, 3H).

### Exemple 20 (Composé N°20)

### N-(2-Hydroxyméthyl-thiazolo[5,4-b]pyridin-6-yl)-5-trifluorométhyl-1-[(thiazol-2-yl)méthyl]-1H-indole-2-carboxamide

### 20.1 2-(Chlorométhyl)thiazole

A une solution de 0,5 g (4,34 mmoles) de thiazol-2-yl-méthanol dans 5 mL de tétrachlorure de carbone et 6 mL de benzène est ajoutée 1,71 g (6,51mmoles) de triphénylphosphine. Le mélange réactionnel est agité à 95°C pendant 2 heures. Après retour à température ambiante, le mélange réactionnel est dilué avec du dichlorométhane puis filtré sur célite. Le filtrat est concentré sous pression réduite et le résidu obtenu est purifié par chromatographie flash sur colonne de gel de silice en éluant avec un mélange d'hexane et d'acétate d'éthyle pour donner 0,4 g de 2-(chlorométhyl)thiazole attendu sous la forme d'une huile jaune claire.
R.M.N. ¹H (DMSO D₆), δ (ppm): 7,83-7,80 (m, 2H) ; 5,11 (s, 2H).

### 20.2 5-Trifluorométhyl-1-[(thiazol-2-yl)]méthyl-1H-indole-2-carboxylate d'éthyle

A une suspension de 87 mg (1,983 mmole) d'hydrure de sodium (55%) dans 10 mL de DMF sec, maintenue sous atmosphère inerte, est ajoutée, goutte à goutte à température ambiante, 340 mg (1,322 mmoles) de 5-trifluorométhyl-1H-indole-2-carboxylate d'éthyle dans 5 mL de DMF sec. Le mélange réactionnel est agité à 50°C pendant 1 heure. On ajoute ensuite, goutte à goutte, à 0°C, une solution de 265 mg (1,983 mmole) de 2-(chlorométhyl)thiazole, préparé selon le protocole décrit à l'étape précédente, dans 10 mL de THF. Le mélange réactionnel est alors agité pendant 20 h à température ambiante puis dilué avec 100 mL d'acétate d'éthyle. On extrait la phase aqueuse avec 30 mL d'acétate d'éthyle. Les phases organiques rassemblées sont lavées successivement avec une solution aqueuse saturée en hydrogénocarbonate de sodium, à l'eau, avec une solution aqueuse saturée en chlorure de sodium puis séchées sur du sulfate de sodium, filtrées et concentrées sous pression réduite. On purifie l'huile résultante par chromatographie sur colonne de silice en éluant avec un mélange d'heptane et d'acétate d'éthyle. On isole 265 mg du produit attendu sous la forme d'une huile incolore.
R.M.N. ¹H (DMSO D₆), δ (ppm) : 8,18 (s, 1 H) ; 7,95 (d, 1 H) ; 7,72 (d, 1 H) ; 7,65 (d, 1 H) ; 7,62 (d, 1 H) ; 7,54 (s, 1 H) ; 6,20 (s, 2H) ; 4,34 (q, 2H) ; 1,31 (t, 3H).
LC-MS: 355 ([M+H]⁺

### 20.3 N-(2-Hydroxyméthyl-thiazolo[5,4-b]pyridin-6-yl)-5-trifluorométhyl-1-[(thiazol-2-yl)méthyl]-1H-indole-2-carboxamide (Composé N°20)

Ce composé a été préparé selon un procédé similaire à celui décrit à l'étape 16.2 en faisant réagir 125 mg (0,353 mmole) de 5-trifluorométhyl-1-[(thiazol-2-yl)]méthyl]-1*H-*indole-2-carboxylate d'éthyle, préparé selon le protocole décrit à l'étape précédente, avec 94 mg (0,353 mmole) de 2,2-Diméthyl-propanoate de (6-amino-thiazolo[5,4-*b*]pyridin-2-yl)méthyle, obtenu à l'étape 4.2, en présence de 0,26 mL (0,529 mmole) d'une solution de triméthylaluminium (2M / toluène). Le brut réactionnel est ensuite purifié par chromatographie flash sur colonne de gel de silice dans un mélange de dichlorométhane et de méthanol puis lavé avec un mélange (1/1) d'éther et de dichlorométhane pour donner 64 mg du produit attendu.
PF : 282 - 283°C
R.M.N. ¹H (DMSO D₆), δ (ppm) : 10,99 (s, 1H) ; 8,94 (d, 1H) ; 8,72 (d, 1H) ; 8,23 (s, 1 H), 7,95 (d, 1 H) ; 7,72-7.61 (m, 4H) ; 6,38 (t, 1 H) ; 6,24 (s, 2H) ; 4,88 (d, 2H).
LC-MS: 490 ([M+H]⁺

### Exemple 21 (Composé N°21)

### N-(2-Hydroxyméthyl-thiazolo[5,4-b]pyridin-6-yl)-6-trifluorométhyl-1-[(thiazol-2-yl)méthyl]-1H-indole-2-carboxamide

Le produit est préparé selon un procédé similaire à celui décrit à l'exemple 20.
PF : 243-244°C
R.M.N. ¹H (DMSO D₆), δ (ppm) : 11,0 (s, 1H) ; 8,95 (d, 1H) ; 8,7 (d, 1H) ; 8,2 (s, 1H) ; 8,0 (d, 1 H) ; 7,75 (d, 1 H) ; 7,6 (m, 2H) ; 7,5 (d, 1 H) ; 6,35 (t, 1 H) ; 6,25 (s, 2H) ; 4,9 (d, 2H).

### Exemple 22 (Composé N°22)

### N-(2-Hydroxyméthyl-thiazolo[5,4-b]pyridin-6-yl)-5-triméthylsilyl-1-[(thiazol-2-yl)méthyl]-1H-indole-2-carboxamide

### 22.1 5-Triméthylsilyl-1-[(thiazol-2-yl)méthyl]-1H-indole-2-carboxylate d'éthyle (composé N° IIb)

Ce composé est préparé selon un procédé similaire à celui décrit à l'étape 20.2 à partir du 5-triméthylsilyl-1*H*-indole-2-carboxylate d'éthyle décrit à l'étape 18.4.
R.M.N. ¹H (DMSO D₆), δ (ppm) : 7,80 (s, 1H) ; 7,64 (d, 1H) ; 7,61 (d, 1H) ; 7,51 (d, 1H) ; 7,40 (d, 1 H) ; 7,30 (s, 1H) ; 6,04 (s, 2H) 4,24 (q, 2H) ; 1,23 (t, 3H) ; 0,19 (s, 9H).

### 22.6 N-(2-Hydroxyméthyl-thiazolo[5,4-b]pyridin-6-yl)-5-triméthylsilyl-1-[(thiazol-2-yl)méthyl]-1H-indole-2-carboxamide (composé N°22)

Ce composé est préparé selon un procédé similaire à celui décrit à l'étape 16.2 à partir 5-triméthylsilyl-1-[(thiazol-2-yl)méthyl]-1*H*-indole-2-carboxylate d'éthyle décrit à l'étape précédente.
PF : 270-271°C
R.M.N. ¹H (DMSO D₆), δ (ppm) : 10,8 (s, 1 H) ; 8,95 (d, 1 H) ; 8,7 (d, 1 H) ; 7,9 (s, 1H) ; 7,7 (m, 2H) ; 7,55 (d, 1 H) ; 7,5 (s, 1 H) ; 7,45 (d, 1 H) ; 6,35 (t, 1 H) ; 6,2 (s, 2H) ; 4,9 (d, 2H) ; 0,3 (s, 9H).

### Exemple 23 (Composé N°23)

*N*-(2-Hydroxyméthyl-thiazolo[5,4-*b*]pyridin-6-yl)-6-fluoro-1-[(thiazol-2-yl)méthyl]-1*H-*indole-2-carboxamide
Le produit est préparé selon un procédé similaire à celui décrit à l'exemple 20.
PF : 151-153°C
R.M.N. ¹H (DMSO D₆), δ (ppm) : 10,8 (s, 1H) ; 8,95 (d, 1H) ; 8,7 (d, 1H) ; 7,8 (m, 1H) ; 7,7 (d, 1 H) ; 7,6 (m, 3H) ; 7,1 (m, 1H) ; 6,35 (t, 1H) ; 6,2 (s, 2H) ; 4,9 (d, 2H).

### Exemple 24 (Composé N°24)

### N-(2-Hydroxyméthyl-thiazolo[5,4-b]pyridin-6-yl)-5-trifluorométhyl-1-[(thiazol-2-yl)méthyl]-1H-pyrrolo[2,3-b]pyridine-2-carboxamide

Le produit est préparé selon un procédé similaire à celui décrit à l'exemple 20.
PF : 207-208°C
R.M.N. ¹H (DMSO D₆), δ (ppm) : 11,1 (s, 1 H) ; 8,95 (d, 1 H) ; 8,85 (s, 1 H) ; 8,8 (s, 1 H) ; 8,7 (s, 1 H) ; 7,7 (s, 1 H) ; 7,65 (d, 1 H) ; 7,58 (d, 1 H) ; 6,35 (t, 1 H) ; 6,3 (s, 2H) ; 4,9 (d, 2H).

### Exemple 25 (Composé N°25)

### N-(2-Hydroxyméthyl-thiazolo[5,4-b]pyridin-6-yl)-6-triméthylsilyl-1-[(thiazol-2-yl)méthyl]-1H-indole-2-carboxamide

### 25.1 2-Azido-3-(4-triméthylsilylphényl)propénoate d'éthyle

Dans un ballon de 100 mL, muni d'un agitateur magnétique et maintenu sous atmosphère d'azote sont introduits 1,26 g (54,96 mmoles) de sodium et 30 mL d'éthanol anhydre. Le mélange réactionnel est agité à température ambiante jusqu'à obtention d'une solution homogène. A cette solution refroidie à -10°C, on ajoute, goutte à goutte, une solution contenant 16,83 mL (54,96 mmoles) d'azidoacétate d'éthyle (34% dans le CH₂Cl₂) et 5 g (27,48 mmoles) de 4-triméthylsilylbenzaldehyde dans 5 mL d'éthanol. On agite ensuite le mélange réactionnel à 0°C pendant 4 heures. On hydrolyse le milieu réactionnel par ajout sous agitation vigoureuse de 100 mL d'une solution de chlorure d'ammonium (30% aqueuse). On extrait le produit avec trois fois 50 mL d'acétate d'éthyle. On lave les phases organiques rassemblées avec deux fois 20 mL d'eau, on sèche sur sulfate de sodium et on concentre sous pression réduite. On purifie l'huile résultante par chromatographie sur colonne de gel de silice en éluant avec un mélange d'heptane et de dichlorométhane. On isole 4,96 g du produit attendu sous forme d'une huile jaune.
R.M.N. ¹H (DMSO D₆), δ (ppm) : 7,6 (d, 2H) ; 7,35 (d, 2H) ; 6,7 (s, 1H) ; 4,1 (q, 2H) ; 1,1 (t, 3H) ; 0 (s, 9H).

### 25.2 6-Triméthylsilyl-1H-indole-2-carboxylate d'éthyle

A une solution de 1,0 g (3,14 mmoles) de 2-azido-3-(4-triméthylsilylphényl)propénoate d'éthyle obtenu à l'étape précédente, dans 20 mL de toluène sec, maintenue sous atmosphère inerte, est ajouté 0,17 g (0,16 mmole) du complexe dimère d'heptafluorobutyrate de dirhodium (II). Le mélange réactionnel est alors agité pendant 12 h à 70°C. Après retour à température ambiante, on filtre le mélange réactionnel sur gel de silice en éluant avec de l'acétate d'éthyle. Le filtrat est ensuite concentré sous pression réduite. On purifie le résidu par chromatographie sur colonne de gel de silice en éluant avec un mélange d'heptane et de dichlorométhane. On isole 0,61 g du produit attendu sous la forme d'une poudre beige.
PF = 127-129°C
R.M.N. ¹H (DMSO D₆), δ (ppm) : 11,7 (s, 1H) ; 7,41 (dd, 1H) ; 7,39 (d, 1H) ; 6,97 (dd, 1H) ; 6,88 (d, 1H) ; 4,1 (q, 2H) ; 1,1 (t, 3H) ; 0,0 (s, 9H).

### 25.3 6-triméthylsilyl-1-[(thiazol-2-yl)méthyl]-1H-indole-2-carboxylate d'éthyle (Composé N° IIc)

Ce composé a été préparé selon un procédé similaire à celui décrit à l'étape 20.2 en faisant réagir 570 mg (2,18 mmoles) de 6-triméthylsilyl-1*H*-indole-2-carboxylate d'éthyle avec 440 mg (3,27 mmoles) de 2-(chloromethyl)thiazole en présence de 0,14 g (3,27 mmoles) d'hydrure de sodium (55%). Le brut réactionnel est ensuite purifié par chromatographie flash sur colonne de gel de silice dans un mélange d'heptane et d'acétate d'éthyle pour donner 520 mg du produit attendu.
R.M.N. ¹H (DMSO D₆), δ (ppm) : 7,76 (s, 1H) ; 7,65-7,61 (m, 2H) ; 7,52 (d, 1H) ; 7,28 (s, 1 H) ; 7,22 (d, 1H) ; 6,09 (s, 2H) ; 4,23 (q, 2H) ; 1,22 (t, 3H) ; 0,2 (s, 9H).
LC-MS: 359 ([M+H]⁺

### 25.4 N-(2-Hydroxyméthyl-thiazolo[5,4-b]pyridin-6-yl)-6-triméthylsilyl-1-[(thiazol-2-yl)méthyl]-1H-indole-2-carboxamide (Composé N°25)

Ce composé a été préparé selon un procédé similaire à celui décrit à l'étape 16.2 en faisant réagir 200 mg (0,523 mmole) de 6-triméthylsilyl-1-[(thiazol-2-yl)]méthyl-1*H-*indole-2-carboxylate d'éthyle, préparé à l'étape précédente, avec 178 mg (0,670 mmole) de 2,2-Diméthyl-propanoate de (6-amino-thiazolo[5,4-*b*]pyridin-2-yl)méthyle, obtenu à l'étape 4.2, en présence de 0,39 mL (0,784 mmoles) d'une solution de triméthylaluminium (2M / toluène). Le produit est isolé par purification par chromatographie sur colonne de silice en éluant avec un mélange de dichlorométhane et d'éthanol. On obtient 120 mg de produit attendu.
PF : 219 - 220°C
R.M.N. ¹H (DMSO D₆), δ (ppm) : 10,76 (s, 1 H) ; 8,86 (d, 1 H) ; 8,64 (d, 1H) ; 7,76 (s, 1H) ; 7,67 (d, 1H) ; 7,63 (d, 1 H) ; 7,50 (d, 1 H) ; 7,42 (s, 1 H) ; 7,22 (d, 1 H) ; 6,29 (t, 1H) ; 6,14 (s, 2H) ; 4,79 (d, 2H) ; 0,19 (s, 9H).
LC-MS: 494 ([M+H]⁺

### Exemple 26 (Composé N°26)

### N-(2-Hydroxyméthyl-thiazolo[5,4-b]pyridin-6-yl)-5-trifluorométhyl-1-[(pyridin-4-yl)méthyl]-1H-indole-2-carboxamide

Le produit est préparé selon un procédé similaire à celui décrit à l'exemple 16.
PF : 237-238°C
R.M.N. ¹H (DMSO D₆), δ (ppm) : 8,9 (s, 1H) ; 8,7 (s, 1 H) ; 8,5 (d, 2H) ; 8,3 (s, 1 H) ; 7,8 (d, 1 H) ; 7,75 (s, 1 H) ; 7,6 (d, 1 H) ; 7,0 (m, 2H) ; 6,35 (t, 1 H) ; 6,0 (s, 2H) ; 4,9 (d, 2H).

### Exemple 27 (Composé N°27)

*N*-(2-Hydroxyméthyl-thiazolo[5,4-*b*]pyridin-6-yl)-6-trifluorométhyl-1-[(pyridin-4-yl)méthyl]-1*H*-indole-2-carboxamide
Le produit est préparé selon un procédé similaire à celui décrit à l'exemple 16.
MP : 261-263°C
R.M.N. ¹H (DMSO D₆), δ (ppm) : 8,9 (s, 1H) ; 8,7 (s, 1H) ; 8,5 (d, 2H) ; 8,1 (m, 2H) ; 7,7 (s, 1 H) ; 7,5 (d, 1 H) ; 6,95 (d, 2H) ; 6,35 (t, 1 H) ; 6,0 (s, 2H) ; 4,9 (d, 2H).

### Exemple 28 (Composé N°28)

### N-(2-Hydroxyméthyl-thiazolo[5,4-b]pyridin-6-yl)-5-triméthylsilyl-1-[(pyridin-4-yl)méthyl]-1H-indole-2-carboxamide

### 28.1 5-Triméthylsilyl-1-[(pyridin-4-yl)méthyl]-1H-indole-2-carboxylate d'éthyle (Composé N° IId).

Le produit est préparé selon un procédé similaire à celui décrit à l'exemple 16.1 à partir du 5-triméthylsilyl-1*H*-indole-2-carboxylate d'éthyle préparé à l'étape 18.4.
R.M.N. ¹H (DMSO D₆), δ (ppm) : 8,37 (d, 2H), 7,83 (s, 1H), 7,47 (d, 1H), 7,37 (d, 1 H), 7,34 (s, 1H), 6,84 (d, 2H), 5,80 (s, 2H), 4,18 (q, 2H), 1,18 (t, 3H), 0,19 (s, 9H).

### 28.2 N-(2-Hydroxyméthyl-thiazolo[5,4-b]pyridin-6-yl)-5-triméthylsilyl-1-[(pyridin-4-yl)méthyl]-1H-indole-2-carboxamide (Composé N° 28)

Le produit est préparé selon un procédé similaire à celui décrit à l'exemple 16.2 à partir du 5-triméthylsilyl-1-[(pyridin-4-yl)méthyl]-1*H*-indole-2-carboxylate d'éthyle préparé à l'étape précédente.
PF : 208-210°C
R.M.N. ¹H (DMSO D₆), δ (ppm) : 8,9 (s, 1 H) ; 8,7 (s, 1H) ; 8,45 (d, 2H) ; 7,95 (s, 1 H) ; 7,58 (s, 1 H) ; 7,5 (d, 1H) ; 7,4 (d, 1H) ; 7,0 (d, 2H) ; 6,35 (t, 1 H) ; 5,9 (s, 2H) ; 4,9 (d, 2H) ; 0,3 (s, 9H).

### Exemple 29 (Composé N°29)

### N-(2-Hydroxyméthyl-thiazolo[5,4-b]pyridin-6-yl)-6-fluoro-1-[(pyridin-4-yl)méthyl]-1H-indole-2-carboxamide

Le produit est préparé selon un procédé similaire à celui décrit à l'exemple 16.
PF : 290-291 °C
R.M.N. ¹H (DMSO D₆), δ (ppm) : 8,9 (s, 1 H) ; 8,7 (s, 1 H) ; 8,45 (d, 2H) ; 7,8 (m, 1H) ; 7,6 (s, 1 H) ; 7,5 (dd, 1 H) ; 7,1 (m, 1 H) ; 7,0 (d, 2H) ; 6,35 (t, 1H) ; 5,9 (s, 2H) ; 4,9 (d, 2H).

### Exemple 30 (Composé N°30)

### N-(2-Hydroxyméthyl-thiazolo[5,4-b]pyridin-6-yl)-5-trifluorométhyl-1-[(pyridin-4-yl)méthyl]-1H-pyrrolo[2,3-b]pyridine-2-carboxamide

### 30.1 5-Trifluorométhyl-1-[(4-pyridinyl)méthyl)]-1H-pyrrolo[2,3-b]pyridin-2-carboxylate d'éthyle

Ce composé a été préparé selon un procédé similaire à celui décrit à l'étape 16.1 en faisant réagir 480 mg (1,859 mmole) de 5-trifluorométhyl-1*H*-pyrrolo[2,3-*b*]pyridin-2-carboxylate d'éthyle avec 406 mg (3,718 mmoles) de 4-pyridinylméthanol en présence de 0,89 g (3,718 mmoles) de cyanométhylènetributylphosphorane (CMBP). Le brut réactionnel est ensuite purifié par chromatographie flash sur colonne de gel de silice dans un mélange d'heptane et d'acétate d'éthyle pour donner 582 mg du produit attendu sous la forme d'un solide blanc.
R.M.N. ¹H (DMSO D₆), δ (ppm) : 8,84 (s, 1 H) ; 8,72 (s, 1 H) ; 8,45 (d, 2H) ; 7,56 (s, 1H) ; 6,98 (d, 2H) ; 5,94 (s, 2H) ; 4,29 (q, 2H) ; 1,25 (t, 3H).
LC-MS: 350 ([M+H]⁺

### 30.2 N-(2-Hydroxyméthyl-thiazolo[5,4-b]pyridin-6-yl)-5-trifluorométhyl-1-[(pyridin-4-yl)méthyl]-1H-pyrrolo[2,3-b]pyridine-2-carboxamide (Composé N°30)

Ce composé a été préparé selon un procédé similaire à celui décrit à l'étape 16.2 en faisant réagir 200 mg (0,573 mmole) de 5-trifluorométhyl-1-[(4-pyridinyl)méthyl)]-1*H-*pyrrolo[2,3-*b*]pyridin-2-carboxylate d'éthyle, préparé selon le protocole décrit à l'étape précédente, avec 182 mg (0,687 mmole) de 2,2-diméthyl-propanoate de (6-amino-thiazolo[5,4-b]pyridin-2-yl)méthyle, obtenu à l'étape 4.2, en présence de 0,43 mL 0,859 mmoles) d'une solution de triméthylaluminium (2M / toluène). Le brut réactionnel est ensuite purifié par chromatographie sur colonne de silice en éluant avec un mélange de dichlorométhane et de méthanol pour donner 71 mg du produit attendu.
PF : 275 - 276°C
R.M.N. ¹H (DMSO D₆), δ (ppm) : 11,03 (s, 1H) ; 8,89 (d, 1H) ; 8,82 (s, 2H) ; 8,65 (d, 1H) ; 8,45 (d, 2H) ; 7,74 (s, 1H) ; 7,04 (d, 2H) ; 6,38 (t, 1H) ; 5,99 (s, 2H) ; 4,87 (d, 2H).
LC-MS: 485 ([M+H]⁺

### Exemple 31 (Composé N°31)

### N-(2-Hydroxyméthyl-thiazolo[5,4-b]pyridin-6-yl)-6-triméthylsilyl-1-[(pyridin-4-yl)méthyl]-1H-indole-2-carboxamide

### 31.1 6-Timéthylsilyl-1-[(pyridin-4-yl)méthyl]-1H-indole-2-carboxylate d'éthyle (Composé N° IIe)

Le produit est préparé selon un procédé similaire à celui décrit à l'exemple 16.1 à partir du 6-triméthylsilyl-1*H*-indole-2-carboxylate d'éthyle préparé à l'étape 25.2.
R.M.N. ¹H (DMSO D₆), δ (ppm) : 8,2 (d, 2H) ; 7,49 (d, 1 H) ; 7,42 (s, 1 H) ; 7,15 (s, 1H) ; 7,05 (d, 1H) ; 6,70 (d, 2H) ; 5,68 (s, 2H) ; 4,01 (q, 2H) ; 1,01 (t, 3H) ; 0,0 (s, 9H).

### 31.2 N-(2-Hydroxyméthyl-thiazolo[5,4-b]pyridin-6-yl)-6-triméthylsilyl-1-[(pyridin-4-yl)méthyl]-1H-indole-2-carboxamide (Composé N°31)

Le produit est préparé selon un procédé similaire à celui décrit à l'exemple 16.2 à partir du 6-triméthylsilyl-1-[(pyridin-4-yl)méthyl]-1*H*-indole-2-carboxylate d'éthyle préparé à l'étape précédente.
PF : 213-214°C
R.M.N. ¹H (DMSO D₆), δ (ppm) : 8,9 (s, 1 H) ; 8,7 (s, 1 H) ; 8,45 (d, 2H) ; 7,8 (d, 1H) ; 7,65 (s, 1 H) ; 7,55 (s, 1 H) ; 7,35 (d, 1 H) ; 7,05 (d, 2H) ; 6,35 (t, 1 H) ; 6,0 (s, 2H) ; 4,9 (d, 2H) ; 0,25 (s, 9H).

### Exemple 32 (Composé N°32)

### N-(2-Hydroxyméthyl-thiazolo[5,4-b]pyridin-6-yl)-5-triméthylsilyl-1-[[(3-trifluorométhyl)phényl]méthyl]-1H-indole-2-carboxamide

### 32.1 5-Triméthylsilyl-1-[[(3-trifluorométhyl)phényl]méthyl]-1H-indole-2-carboxylate d'éthyle (Composé N° IIf)

Le composé a été préparé selon un procédé similaire à celui décrit à l'étape 16.1 en faisant réagir 0,49 g (1,87 mmole) de 5-triméthylsilyl-1*H*-indole-2-carboxylate d'éthyle, préparé à l'étape 18.4, avec 0,51 mL (3,749 mmoles) de 3-(trifluorométhyl)phénylméthanol en présence de 0,9 g (3,749 mmoles) de cyanométhylènetributylphosphorane (CMBP). Le brut réactionnel est ensuite purifié par chromatographie sur colonne de silice dans un mélange d'heptane et d'acétate d'éthyle pour donner 730 mg du produit attendu.
R.M.N. ¹H (DMSO D₆), δ (ppm) : 7,90 (s, 1 H) ; 7,62-7,57 (m, 2H) ; 7,51-7,43 (m, 3H) ; 7,40 (s, 1H) ; 7,17 (d, 1H) ; 5,92 (s, 2H) ; 4,28 (q, 2H) ; 1,26 (t, 3H) ; 0,27 (s, 9H). LC-MS: 420 ([M+H]⁺

### 32.2 N-(2-Hydroxyméthyl-thiazolo[5,4-b]pyridin-6-yl)-5-triméthylsilyl-1-[[(3-trifluorométhyl)phényl]méthyl]-1H-indole-2-carboxamide

Ce composé a été préparé selon un procédé similaire à celui décrit à l'étape 16.2 en faisant réagir 200 mg (0,477 mmole) de 5-triméthylsilyl-1-[[(3-trifluorométhyl)phényl]méthyl]-1*H*-indole-2-carboxylate d'éthyle, préparé à l'étape précédente, avec 152 mg (0,572 mmole) de 2,2-Diméthyl-propanoate de (6-amino-thiazolo[5,4-*b*]pyridin-2-yl)méthyle, obtenu à l'étape 4.2, en présence de 0,36 mL (0,715 mmoles) d'une solution de triméthylaluminium (2M / toluène). Le produit est isolé par purification par chromatographie flash sur colonne de gel de silice dans un mélange d'heptane et d'acétate d'éthyle. On obtient 128 mg de produit attendu
PF : 152 -153 °C
R.M.N. ¹H (DMSO D₆), δ (ppm) : 10,82 (s, 1H) ; 8,91 (d, 1H) ; 8,68 (d, 1H) ; 7,92 (s, 1H) ; 7,61-7.42 (m, 6H) ; 7,31 (d, 1 H) ; 6,37 (t, 1 H) ; 5,97 (s, 2H) ; 4,87 (d, 2H) ; 0,28 (s, 9H).
LC-MS: 555 ([M+H]⁺

### Exemple 33 (Composé N°33)

### N-(2-Hydroxyméthyl-thiazolo[5,4-b]pyridin-6-yl)-6-fluoro-1-[[(3-trifluorométhyl)phényl]méthyl]-1H-indole-2-carboxamide

### 33.1 6-fluoro-1-[(3-trifluorométhylphényl)méthyl]-1H-indole-2-carboxylate de méthyle

Ce composé a été préparé selon un procédé similaire à celui décrit à l'étape 16.1 en faisant réagir 475 mg (2,459 mmoles) de 6-fluoro-1*H*-indole-2-carboxylate de méthyle avec 0,67 mL (4,918 mmoles) de 3-trifluorométhylphénylméthanol en présence de 1,18 g (4,918 mmoles) de cyanométhylènetributylphosphorane (CMBP). Le brut réactionnel est ensuite purifié par chromatographie sur colonne de silice dans un mélange d'heptane et d'acétate d'éthyle pour donner 706 mg du produit attendu sous la forme d'un solide blanc.
R.M.N. ¹H (DMSO D₆), δ (ppm) : 7,78 (dd, 1H) ; 7,61-7,49 (m, 4H) ; 7,43 (s, 1H) ; 7,19 (d, 1 H) ; 7,05 (dt, 1H) ; 5,91 (s, 2H) ; 3,80 (s, 3H).
LC-MS: 351 ([M+H]⁺

### 33.2 N-(2-Hydroxyméthyl-thiazolo[5,4-b]pyridin-6-yl)-6-fluoro-1-[[(3-trifluorométhyl)phényl]méthyl]-1H-indole-2-carboxamide (Composé N°33)

Ce composé a été préparé selon un procédé similaire à celui décrit à l'étape 16.2 en faisant réagir 200 mg (0,569 mmole) de 6-fluoro-1-[(3-trifluorométhylphényl)méthyl]-1*H-*indole-2-carboxylate de méthyle, préparé selon le protocole décrit à l'étape précédente, avec 181 mg (0,683 mmole) de 2,2-Diméthyl-propanoate de (6-amino-thiazolo[5,4-*b*]pyridin-2-yl)méthyle, obtenu à l'étape 4.2, en présence de 0,43 mL (0,853 mmoles) d'une solution de triméthylaluminium (2M / toluène). Le brut réactionnel est ensuite purifié par chromatographie sur colonne de gel de silice dans un mélange de dichlorométhane et de méthanol pour donner 89 mg du produit attendu.
PF : 217 - 218°C
R.M.N. ¹H (DMSO D₆), δ (ppm) : 10,81 (s, 1H) ; 8,89 (d, 1H) ; 8,67 (d, 1H) ; 7,82 (dd, 1 H) ; 7.60-7.49 (m, 5H) ; 7,33 (d, 1 H) ; 7,06 (dt, 1 H) ; 6,37 (t, 1 H) ; 5,95 (s, 2H) ; 4,87 (d, 2H).
LC-MS: 501 ([M+H]⁺

### Exemple 34 (Composé N°34)

### N-(2-Hydroxyméthyl-thiazolo[5,4-b]pyridin-6-yl)-5-trifluorométhyl-1-[[(3-trifluorométhyl)phényl]méthyl]-1H-pyrrolo[2,3-b]pyridine-2-carboxamide

Le produit est préparé selon un procédé similaire à celui décrit à l'exemple 16.
PF : 312-314°C
R.M.N. ¹H (DMSO D₆), δ (ppm) : 11,0 (s, 1 H) ; 8,90 (s, 1 H) ; 8,85 (s, 1 H) ; 8,8 (s, 1H) ; 8,65 (s, 1 H) ; 7,7 (s, 1 H) ; 7,6 (m, 2H) ; 7,5 (m, 1 H) ; 7,35 (m, 1 H) ; 6,35 (t, 1H) ; 6,05 (s, 2H) ; 4,9 (d, 2H).

### Exemple 35 (Composé N°35)

### N-(2-Hydroxyméthyl-thiazolo[5,4-b]pyridin-6-yl)-6-triméthylsilyl-1-[[(3-trifluorométhyl)phényl]méthyl]-1H-indole-2-carboxamide

### 35.1 6-Triméthylsilyl-1-[[(3-trifluorométhyl)phényl]méthyl]-1H-indole-2-carboxylate d'éthyle (Composé N° IIg)

Le produit est préparé selon un protocole similaire à celui décrit à l'exemple 32.1 à partir du 6-triméthylsilyl-1*H*-indole-2-carboxylate d'éthyle décrit à l'exemple 25.
R.M.N. ¹H (DMSO D₆), δ (ppm) : 7,49-7,45 (m, 2H) ; 7,36-7,33 (m, 2H) ; 7,25 (t, 1H) ; 7,12 (s, 1H) ; 7,04-7,00 (m, 2H) ; 5,73 (s, 2H) ; 4,04 (q, 2H) ; 1,03 (t, 3H) ; 0,00 (s, 9H).

### 35.2 N-(2-Hydroxyméthyl-thiazolo[5,4-b]pyridin-6-yl)-6-triméthylsilyl-1-[[(3-trifluorométhyl)phényl]méthyl]-1H-indole-2-carboxamide (Composé N° 35)

Le produit est préparé selon un procédé similaire à celui décrit à l'exemple 32.2 à partir du 6-triméthylsilyl-1-[[(3-trifluorométhyl)phényl]méthyl]-1*H*-indole-2-carboxylate d'éthyle préparé à l'étape précédente.
PF : 180-181 °C
R.M.N. ¹H (DMSO D₆), δ (ppm) : 10,85 (s, 1H) ; 8,95 (s, 1H) ; 8,75 (s, 1H) ; 7,75 (m, 2H) ; 7,65 (s, 1 H) ; 7,6-7,4 (m, 4H) ; 7,3 (d, 1H) ; 6,35 (t, 1 H) ; 6,0 (s, 2H) ; 4,9 (d, 2H) ; 0,25 (s, 9H).

### Exemple 36 (Composé N°36)

### N-(2-Hydroxyméthyl-thiazolo[5,4-b]pyridin-6-yl)-5-trifluorométhyl-1-[(3-méthylphényl)méthyl]-1H-pyrrolo[2,3-b]pyridine-2-carboxamide

Le produit est préparé selon un procédé similaire à celui décrit à l'exemple 16.
PF : 335-336°C
R.M.N. ¹H (DMSO D₆), δ (ppm) : 11,0 (s, 1 H) ; 8,9 (s, 1 H) ; 8,85 (s, 1 H) ; 8,75 (s, 1H) ; 8,65 (s, 1 H) ; 7,6 (s, 1 H) ; 7,1 (m, 1 H) ; 7,0 (m, 2H) ; 6,9 (m, 1 H) ; 6,3 (t, 1 H) ; 5,95 (s, 2H) ; 4,9 (d, 2H) ; 2,15 (s, 3H).

### Exemple 37 (Composé N°37)

### N-(2-Hydroxyméthyl-thiazolo[5,4-b]pyridin-6-yl)-6-triméthylsilyl-1-[(3-méthylphényl)méthyl]-1H-indole-2-carboxamide

### 37.1 6-triméthylsilyl-1-[(3-méthylphényl)méthyl]-1H-indole-2-carboxylate d'éthyle (Composé N° IIh)

Le produit est préparé selon un protocole similaire à celui décrit à l'exemple 32.1 à partir du 6-triméthylsilyl-1*H*-indole-2-carboxylate d'éthyle décrit à l'exemple 25.
R.M.N. ¹H (DMSO D₆), δ (ppm): 7,71-7,68 (m, 2H) ; 7,33 (s, 1H) ; 7,24-7,21 (m, 1 H) ; 7,14-7,11 (m, 2H) ; 7,09-7,0 (m, 1H) ; 6,81-6,79 (m, 1H) ; 5,85 (s, 2H) ; 4,30 (q, 2 H) ; 2,21 (s, 3H) ; 1,17 (t, 3H), 0,25 (s, 9H).

### 37.2 N-(2-Hydroxyméthyl-thiazolo[5,4-b]pyridin-6-yl)-6-triméthylsilyl-1-[(3-méthylphényl)méthyl]-1H-indole-2-carboxamide (Composé N°37)

Le produit est préparé selon un procédé similaire à celui décrit à l'exemple 32.2 à partir du 6-triméthylsilyl-1-[(3-méthylphényl)méthyl]-1*H*-indole-2-carboxylate d'éthyle préparé à l'étape précédente.
PF : 168-169°C
R.M.N. ¹H (DMSO D₆), δ (ppm) : 10,8 (s, 1H) ; 8,9 (s, 1H) ; 8,7 (s, 1H) ; 7,7 (m, 2H) ; 7,48 (s, 1 H) ; 7,25 (d, 1 H) ; 7,15 (m, 1 H) ; 7,0 (m, 2H) ; 6,9 (m, 1 H) ; 6,35 (t, 1 H) ; 5,9 (s, 2H) ; 4,9 (d, 2H) ; 2,2 (s, 3H) ; 0,3 (s, 9H).

### Exemple 38 (Composé N°38)

*N*-(2-Hydroxyméthyl-thiazolo[5,4-*b*]pyridin-6-yl)-5-trifluorométhyl-1-[[(3-trifluorométhyl)phényl]méthyl]-1*H*-indole-2-carboxamide

Le produit est préparé selon un procédé similaire à celui décrit à l'exemple 16.
PF : 237-239°C
R.M.N. ¹H (DMSO D₆), δ (ppm) : 11,0 (s, 1H) ; 8,95 (s, 1H) ; 8,7 (s, 1H) ; 8,25 (s, 1H) ; 7,85 (d, 1 H) ; 7,7 (s, 1 H) ; 7,65-7,5 (m, 4H) ; 7,35 (m, 1H) ; 6,35 (t, 1H) ; 6,0 (s, 2H) ; 4,9 (d, 2H).

### Exemple 39(Composé N°39)

### N-(2-Hydroxyméthyl-thiazolo[5,4-b]pyridin-6-yl)-6-trifluorométhyl-1-[[(3-trifluorométhyl)phényl]méthyl]-1H-indole-2-carboxamide

Le produit est préparé selon un procédé similaire à celui décrit à l'exemple 16.
PF : 197-198°C
R.M.N. ¹H (DMSO D₆), δ (ppm) : 11,0 (s, 1H) ; 8,95 (s, 1H) ; 8,7 (s, 1H) ; 8,15 (s, 1H) ; 8,05 (d, 1H) ; 7,65-7,45 (m, 5H) ; 7,35 (m, 1H) ; 6,35 (t, 1H) ; 6,1 (s, 2H) ; 4,9 (d, 2H).

Le tableau 1 qui suit illustre les structures chimiques et les propriétés physiques de quelques exemples de composés selon l'invention.

Dans ce tableau :
- la colonne « PF (°C) renseigne les points de fusion des produits en degrés Celsius (°C) ;
- W représente un atome d'oxygène ;
- n est égal à 1 ;
- tous les composés sont sous forme de base libre.
- Me correspond à un groupe méthyle.
- tBu correspond à un groupe ter-butyle.

**Tableau 1**

| | | | | |
|---|---|---|---|---|
| 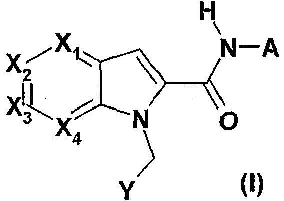 | | | | |

| **N°** | **X₁, X₂, X₃, X₄** | **Y** | **A** | **PF (°C)** |
|---|---|---|---|---|
| **1** | CH, C-F, CH, CH | 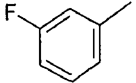 | 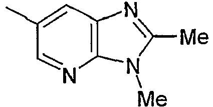 | 274 - 275 |
| **2** | CH, C-F, CH, CH | 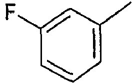 | 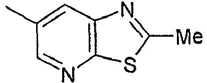 | 264 - 265 |
| **3** | CH, C-F, CH, CH | 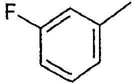 | 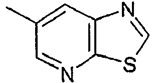 | 250 - 251 |
| **4** | CH, C-F, CH, CH | 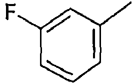 | 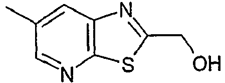 | 225 - 226 |
| **5** | CH, C-F, CH, CH | 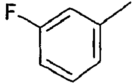 | 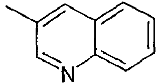 | 244 - 246 |
| **6** | CH, C-F, CH, CH | 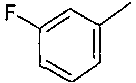 | 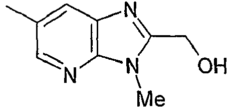 | 231 - 232 |
| **7** | CH, C-F, CH, CH | 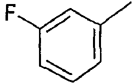 | 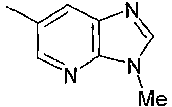 | 235 - 236 |
| **8** | CH, C-CF₃, CH, N | 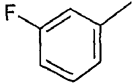 | 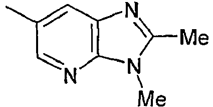 | 274 - 275 |
| **9** | CH, C-F, CH, CH | 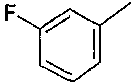 | 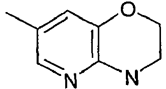 | 265 - 266 |
| **10** | CH, C-F, CH, CH | 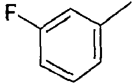 | 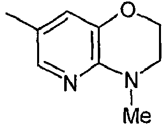 | 214 - 215 |
| **11** | CH, C-F, CH, CH | 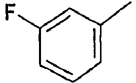 | 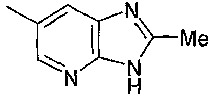 | 299 - 300 |
| **12** | CH, C-F, CH, CH | 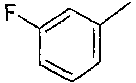 | 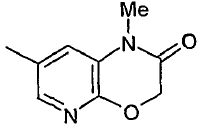 | 248 - 250 |
| **13** | CH, C-F, CH, CH | 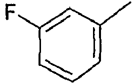 | 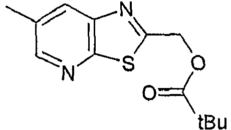 | 224 - 225 |
| **14** | CH, C-F, CH, CH | 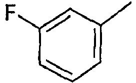 | 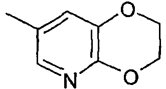 | 224 - 225 |
| **15** | CH, C-F, CH, CH | 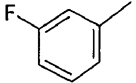 | 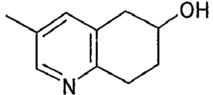 | 204 - 206 |
| **16** | CH, C-CF₃, CH, CH | 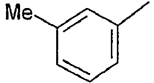 | 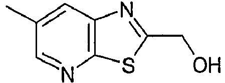 | 315 - 316 |
| **17** | CH, CH, C-CF₃, CH | 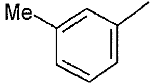 | 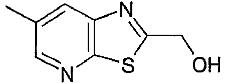 | 221 - 222 |
| **18** | CH, C-SiMe₃, CH, CH | 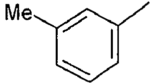 | 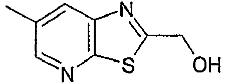 | 154 - 155 |
| **19** | CH, CH, C-F, CH | 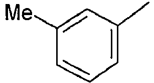 | 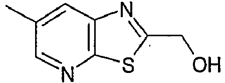 | 257 - 258 |
| **20** | CH, C-CF₃, CH, CH | 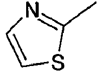 | 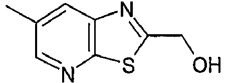 | 282 - 283 |
| **21** | CH, CH, C-CF₃, CH | 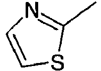 | 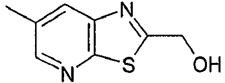 | 243 - 244 |
| **22** | CH, C-SiMe₃, CH, CH | 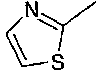 | 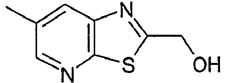 | 270 - 271 |
| **23** | CH, CH, C-F, CH | 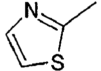 | 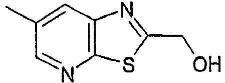 | 151 - 153 |
| **24** | CH, C-CF₃, CH, N | 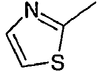 | 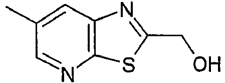 | 207 - 208 |
| **25** | CH, CH, C-SiMe₃, CH | 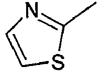 | 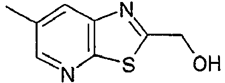 | 219 - 220 |
| **26** | CH, C-CF₃, CH, CH | 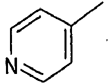 | 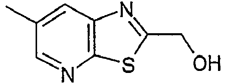 | 237 - 238 |
| **27** | CH, CH, C-CF₃, CH | 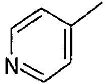 | 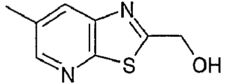 | 261 - 263 |
| **28** | CH, C-SiMe₃, CH, CH | 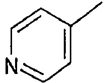 | 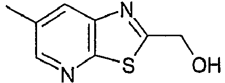 | 208 - 210 |
| **29** | CH, CH, C-F, CH | 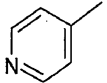 | 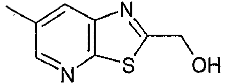 | 290 - 291 |
| **30** | CH, C-CF₃, CH, N | 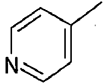 | 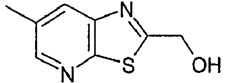 | 275 - 276 |
| **31** | CH, CH, C-SiMe₃, CH | 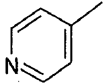 | 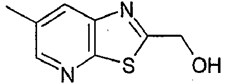 | 213 - 214 |
| **32** | CH, C-SiMe₃, CH, CH | 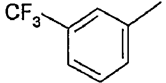 | 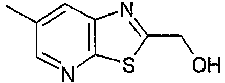 | 152 - 153 |
| **33** | CH, CH, C-F, CH | 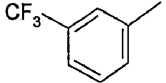 | 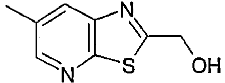 | 217 - 218 |
| **34** | CH, C-CF₃, CH, N | 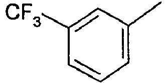 | 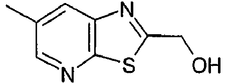 | 312 - 314 |
| **35** | CH, CH, C-SiMe₃, CH | 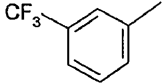 | 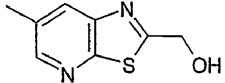 | 180 - 181 |
| **36** | CH, C-CF₃, CH, N | 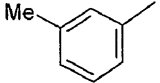 | 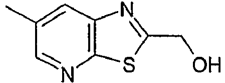 | 335 - 336 |
| **37** | CH, CH, C-SiMe₃, CH | 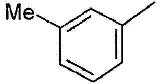 | 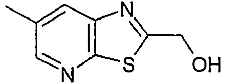 | 168 - 169 |
| **38** | CH, C-CF₃, CH, CH | 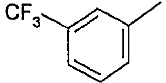 | 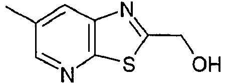 | 237 - 239 |
| **39** | CH, CH, C-CF₃, CH | 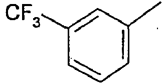 | 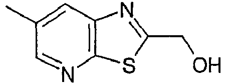 | 197 - 198 |

Les composés selon l'invention ont fait l'objet d'essais pharmacologiques *in vitro* et *in vivo* qui ont mis en évidence leur intérêt comme substances à activités thérapeutiques. Ces composés présentent une activité antagoniste ou agoniste vis-à-vis des récepteurs TRPV1 (ou VR1).

### Test d'inhibition du courant induit par la capsaïcine sur les DRG de rat

### - Culture primaire de cellules de ganglions de racine dorsale (DRG) de rat :

Les neurones du DRG expriment naturellement le récepteur TRPV1.

Les cultures primaires de DRG de rats nouveaux nés sont préparées à partir de ratons de 1 jour. Brièvement, après dissection, les ganglions sont trypsinés et les cellules dissociées mécaniquement par trituration ménagée. Les cellules sont re-suspendues dans un milieu de culture basal Eagle contenant 10 % de sérum de veau foetal, 25 mM KCI, 2 mM glutamine, 100 µg/mL gentamicine et 50 ng/mL de NGF, puis déposées sur des lamelles de verre recouvertes de laminine (0,25 x 10⁶ cellules par lamelle) qui sont ensuite placées dans des boîtes 12 puits Corning. Les cellules sont incubées à 37°C en atmosphère humidifiée contenant 5% de CO₂ et 95% d'air. De la cytosine β-D-arabinoside (1 µM) est ajoutée 48 h après la mise en culture, pour prévenir le développement des cellules non neuronales. Les lamelles sont transférées dans les chambres expérimentales pour les études de patch-clamp après 7-10 jours de culture.

### - Electrophysiologie :

Les chambres de mesure (volume 800 µl) contenant la préparation cellulaire sont placées sur la platine d'un microscope inversé (Olympus IMT2) équipé d'optiques Hoffman (Modulation Contrast, New York) et observées au grossissement de 400X.

Les chambres sont continuellement perfusées par gravité (2,5 mL/min) à l'aide d'un distributeur de solutions acceptant 8 entrées et dont la sortie unique, constituée par un tube de polyéthylène (ouverture 500µm) est placée à moins de 3 mm de la cellule étudiée. La configuration "cellule entière" de la technique de patch-clamp a été utilisée.

Les pipettes en verre borosilicaté (résistance 5-10 MOhms) sont approchées de la cellule grâce à un micromanipulateur piézoélectrique 3D (Burleigh, PC1000). Les courants globaux (potentiel de membrane fixé à -60 mV) sont enregistrés avec un amplificateur Axopatch 1D (Axon Instruments, Foster city, Californie), connecté à un PC piloté par les logiciels de Pclamp8 (Axon Instrument). Les traces de courant sont enregistrées sur papier et simultanément digitalisées (fréquence d'échantillonnage 15 à 25 Hz) et acquises sur le disque dur du PC.

L'application d'une solution de capsaïcine 300 nM provoque sur les cellules de DRG (voltage fixé à -70 mV) un courant cationique entrant. Afin de minimiser la désensibilisation des récepteurs, l'intervalle d'une minute minimum entre deux applications de capsaïcine est respecté. Après une période contrôle (stabilisation de la réponse capsaïcine seule), les composés de l'invention à tester sont appliqués seuls à une concentration donnée (concentration de 10 nM ou de 1 nM) pendant une durée de 4 à 5 minutes, au cours desquelles plusieurs tests « capsaïcine + composé » sont réalisés (obtention de l'inhibition maximale). Les résultats sont exprimés en pourcentage d'inhibition de la réponse capsaïcine contrôle.

Dans le cas des composés antagonistes VR1, les pourcentages d'inhibition de la réponse capsaïcine (1µM) sont compris entre 20% et 100% pour les composés les plus actifs de l'invention testés à des concentrations de 0,1 à 100 nM. Ce sont donc des antagonistes efficaces des récepteurs de type TRPV1. Le tableau 2 donne un exemple de pourcentage d'inhibition obtenu avec les composés de l'invention.

**Tableau 2**

| N° composé | % inhibition en patch DRG |
|---|---|
| 4 | 65,5% (0,1nM) |
| 11 | 62% (100nM) |

Douleur induite par l'administration intraplantaire de capsaicine chez la souris.

L'injection intraplantaire de capsaicine chez la souris produit rapidement un comportement nociceptif de courte durée, qui se traduit par des léchages, des mordillements et un fléchissement de la patte administrée. Ces réponses nociceptives sont probablement liées à activation des récepteurs TRPV1 locaux par la capsaicine. Méthodologie :

La (E)-capsaicine est initialement diluée à 3 mg/ml dans du DMSO, puis diluée à nouveau pour son utilisation finale à 1,5 µg / 20µl dans du sérum physiologique.

L'administration de solvant n'a aucun effet sur le comportement de la souris. La capsaicine est injectée dans une des pattes arrière de l'animal, au niveau de la face supérieure.

Les composés à tester sont administrés par voie orale 120 minutes avant l'injection de capsaicine. Deux heures après l'administration des composés, les souris sont placées dans un bécher en verre. Le comportement nociceptif des animaux est alors immédiatement évalué par l'expérimentateur et la durée des manifestations comportementales induites par la capsaicine est chronométrée pendant une période de 2 minutes (léchages et mordillements, fléchissement total ou partiel de la patte injectée).

Pour chaque composé, est déterminée une inhibition correspondant à la moyenne des réponses nociceptives induites par la capsaicine, en réponse à une dose de produit étudié (exprimée en mg/kg) administrée par voie orale sur un échantillon d'un nombre déterminée de souris (n).

Le tableau 3 donne un exemple de pourcentage d'inhibition obtenu avec les composés de l'invention.

**Tableau 3**

| N° composé | Dose | n | % inhibition des réponses nociceptives induites par la capsaicine |
|---|---|---|---|
| 4 | 10 mg/kg | 10 | 37 % (+/- 8%) |

Les composés de l'invention peuvent donc être utilisés pour la préparation de médicaments, notamment pour la préparation d'un médicament destiné à prévenir ou à traiter les pathologies dans lesquelles les récepteurs de type TRPV1 sont impliqués.

Les composés de l'invention peuvent être utiles pour prévenir ou traiter les pathologies dans lesquelles les récepteurs de type TRPV1 sont impliqués.

Ainsi, l'invention a pour objet des médicaments qui comprennent au moins un composé de formule (I), ou un sel pharmaceutiquement acceptable, ou encore un hydrate ou un solvate dudit composé.

Ces médicaments peuvent trouver leur emploi en thérapeutique, notamment dans la prévention et/ou le traitement de la douleur et de l'inflammation, de la douleur chronique, neuropathique (traumatique, diabétique, métabolique, infectieuse, toxique, induite par un traitement anticancéreux ou iatrogène), (ostéo-) arthritique, rhumatismale, des fibromyalgies, de la douleur du dos, de la douleur liée au cancer, de la névralgie faciale, des céphalées, de la migraine, de la douleur dentaire, de la brûlure, du coup de soleil, de la morsure ou de la piqûre, de la névralgie post-herpétique, de la douleur musculaire, de la compression nerveuse (centrale et/ou périphérique), des traumatismes de la moelle et/ou du cerveau, de l'ischémie (de la moelle et/ou du cerveau), de la neurodégénérescence, des accidents vasculaires hémorragiques (de la moelle et/ou du cerveau), de la douleur post-stroke.

Les composés de l'invention peuvent également être utilisés pour prévenir et/ou traiter les désordres métaboliques tels que le diabète.

Les composés de l'invention peuvent également être utilisés pour prévenir et/ou traiter les désordres urologiques tels que l'hyperactivité de la vessie, l'hyperéflexie vésicale, l'instabilité vésicale, l'incontinence, la miction d'urgence, l'incontinence urinaire, la cystite, la colique néphrétique, l'hypersensibilité pelvienne et la douleur pelvienne.

Les composés de l'invention peuvent être utiles pour prévenir et/ou traiter les désordres gynécologiques comme la vulvodynie, les douleurs liées aux salpingites, aux dysménorrhées.

On peut également utiliser ces produits pour prévenir et/ou traiter les désordres gastro-intestinaux tels que le désordre du réflexe gastro-esophagique, l'ulcère de l'estomac, l'ulcère du duodénum, la dyspepsie fonctionnelle, la colite, l'IBS, la maladie de Crohn, la pancréatite, l'oesophagite, la colique hépatique.

De même, les produits de la présente invention peuvent être utiles dans la prévention et/ou le traitement des désordres respiratoires tels que l'asthme, la toux, la maladie pulmonaire obstructive chronique (COPD), la bronchoconstriction et les désordres inflammatoires de la sphère respiratoire.

Ces produits peuvent également être utilisés pour prévenir et/ou traiter le psoriasis, le pruritis, les irritations dermiques, des yeux ou des muqueuses, l'herpès, le zona.

Les composés de l'invention peuvent également être utilisés pour traiter la dépression. Les composés de l'invention peuvent aussi être utilisés pour traiter les maladies du système nerveux central telles que la sclérose en plaques.

Les composés de l'invention pourraient aussi être utilisés pour traiter les cancers.

Selon un autre de ses aspects, la présente invention concerne des compositions pharmaceutiques comprenant, en tant que principe actif, au moins un composé selon l'invention. Ces compositions pharmaceutiques contiennent une dose efficace d'au moins un composé selon l'invention, ou un sel pharmaceutiquement acceptable, un hydrate ou solvate dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'homme du métier.

Les compositions pharmaceutiques de la présente invention peuvent être administrées par voie orale, sublinguale, sous-cutanée, intramusculaire, intra-veineuse, topique, locale, intratrachéale, intranasale, transdermique ou rectale. Ces compositions peuvent être administrées sous forme unitaire, en mélange avec des excipients pharmaceutiques classiques. Elles sont destinées à être administrées aux animaux et aux êtres humains pour la prophylaxie ou le traitement des troubles ou des maladies citées ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,001 à 30 mg de principe actif par kg de poids corporel, selon la forme galénique.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

Les composés de l'invention peuvent également être utilisés pour la préparation de médicaments, notamment pour la préparation d'un médicament destiné à prévenir ou à traiter les pathologies dans lesquelles les récepteurs de type TRPV1 sont impliqués, telles que mentionnées précédemment.

## Revendications

1. Composé répondant à la formule (I) 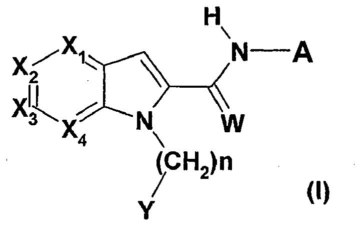 dans laquelle :
X₁, X₂, X₃, et X₄ représentent, indépendamment l'un de l'autre, un atome d'azote ou un groupe C-R₁ ;
Etant entendu que quand l'un des X₁, X₂, X₃, et X₄ représente un atome d'azote, les autres correspondent à un groupe C-R₁ ;
W représente un atome d'oxygène ou de soufre ;
n est égal à 0, 1, 2 ou 3 ;
Y représente un aryle ou un hétéroaryle éventuellement substitué par un ou plusieurs groupes choisis parmi un atome d'halogène, un groupe C₁-C₆-alkyle, C₃-C₇-cydoalkyle, C₃-C₇-cycloalkyl-C₁-C₃-alkylène, C₁-C₆-fluoroalkyle, hydroxy, C₁-C₆-alcoxy, C₃-C₇-cycloalkyloxy, C₃-C₇-cycloalkyl-C₁-C₆-alkylène-O-, C₁-C₆-fluoroalcoxy, cyano, C(O) NR₄R₅, nitro, NR₄R₅, C₁-C₆-thioalkyle, thiol, -S(O)-C₁-C₆-alkyle, -S(O)₂-C₁-C₆-alkyle, SO₂NR₄R₅, NR₆C(O)R₇, NR₆SO₂R₈, C(O)NR₄R₅, OC(O)NR₄R₅, -Si-(C₁-C₆-alkyl)₃, -SF₅, aryle-C₁-C₅-alkylène ou aryle, hétéroaryl-C₁-C₅-alkylène ou hétéroaryle ; les groupes C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₃-C₁-cycloalkyl-C₁-C₆-alkylène-O- étant éventuellement substitués par un groupe hydroxy, C₁-C₆-alcoxy ou NR₄R₅ ; les groupes aryle et hétéroaryle étant éventuellement substitués par un ou plusieurs substituants R₉ identiques ou différents l'un de l'autre ;
A représente le groupe de formule : 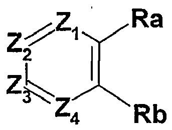
Z₁, Z₂, Z₃ et Z₄ représentent, indépendamment l'un de l'autre, un atome d'azote, un atome de carbone ou un groupe C-R₂,
l'un, au moins, des Z₁, Z₂, Z₃ et Z₄ correspondant à un atome d'azote et
l'un des Z₁, Z₂, Z₃ et Z₄, correspondant à un atome de carbone, étant lié à l'atome d'azote de l'amide ou du thioamide de formule (1) ;
Ra et Rb forment ensemble avec les atomes de carbone qui les portent,
soit un cycloalkyle partiellement insaturé, soit un aryle ;
soit un hétérocycle, soit un hétéroaryle de 5 à 7 chaînons comprenant de un à trois hétéroatomes choisi parmi O, S ou N ;
étant entendu que le cas où Ra et Rb forment ensemble avec les atomes de carbone qui les portent un cycle à cinq chaînons, ce cycle comportant un atome d'azote et des atomes de carbone, ce cycle étant partiellement saturé ou insaturé, est exclu ;
ce cycloalkyle partiellement insaturé, cet aryle, cet hétérocycle ou cet hétéroaryle pouvant être substitués par un ou plusieurs substituants R₃ ;
R₁ est choisi parmi un atome d'hydrogène, un atome d'halogène, un groupe C₁-C₆-alkyle, C₃-C₇-cycloalkyle, C₃-C₇-cycloalkyl-C₁-C₃-alkylène, C₁-C₆-fluoroalkyle, aryloxy-C₁-C₆-alkyle, hétéroaryloxy-C₁-C₆-alkyle, aryl-C₁-C₃-alkylènoxy-C₁-C₆-alkyle, hétéroaryl-C₁-C₃-alkylènoxy-C₁-C₆-alkyle, arylthio-C₁-C₆-alkyle, hétéroarylthio-C₁-C₆-alkyle, aryl-C₁-C₃-alkylène-thio-C₁-C₆-alkyle, hétéroaryl-C₁-C₃-alkylène-thio-C₁-C₆-alkyle, C₁-C₆-alcoxy, C₃-C₇-cycloalkyloxy, C₃-C₇-cycloalkyl-C₁-C₃-alkylènoxy, C₁-C₆-fluoroalcoxy, cyano, C(O)NR₄R₅, nitro, NR₄R₅, C₁-C₆-thioalkyle, C₃-C₇-cycloalkylthio, C₃-C₇-cycloalkyl-C₁-C₃-alkylène-thio, -S(O)-C₁-C₆-alkyle, -S(O)-C₃-C₇-cycloalkyle, - S(O)-C₁-C₃-alkylène-C₃-C₇-cycloalkyle, C₁-C₆-alkyle-S(O)₂-, C₁-C₆-fluoroalkyle-S(O)₂-, C₃-C₇-cycloalkyl-S(O)₂-, C₃-C₇-cycloalkyl-C₁-C₃-alkylène-S(O)₂-, SO₂NR₄R₅, -Si-(C₁-C₆-alkyl)₃, SF₅, NR₆C(O)R₇, NR₆SO₂R₈, C(O)NR₄R₅, OC(O)NR₄R₅, aryle, hétéroaryle, aryl-C₁-C₅-alkylène, hétéroaryl-C₁-C₅-alkylène, aryloxy, arylthio, hétéroaryloxy ou hétéroarylthio ; les groupes hétéroaryle ou aryle étant éventuellement substitués par un ou plusieurs substituants R₉, identiques ou différents l'un de l'autre ;
R₂ représente un atome d'hydrogène, un atome d'halogène, un groupe C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₃-C₇-cycloalkyl-C₁-C₃-alkylénoxy, hydroxy, thiol, C₁-C₆-fluoroalcoxy ;
R₃, représente, lorsqu'il est porté par un atome de carbone, un atome d'hydrogène, un groupe hydroxy, thiol, C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₃-C₇-cycloalkyl-C₁-C₃-alkylénoxy, C₁-C₆-alcoxy-C₁-C₃-alkylène, C₃-C₇-cycloalkyloxy-C₁-C₃-alkylène, C₃-C₇-cycloalkyl-C₁-C₃-alkylénoxy-C₁-C₃-alkylène, C(O)NR₄R₅, C(O)O-C₁-C₆-alkyle, CO₂H, oxo, thio ; les groupes C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cydoalkyloxy, C₃-C₇-cycloalkyl-C₁-C₃-alkylénoxy, C₁-C₆-alcoxy-C₁-C₃-alkylène, C₃-C₇-cycloalkyloxy-C₁-C₃-alkylène, C₃-C₇-cycloalkyl-C₁-C₃-alkylénoxy-C₁-C₃-alkylène pouvant être substitués par un groupe hydroxy, C₁-C₆-alcoxy, -OC(O)-C₁-C₆-alkyle ou NR₄R₅ ;
ou
R₃, représente, lorsqu'il est porté par un atome d'azote, un atome d'hydrogène, un groupe C₁-C₆-alkyle, C₃-C₇-cycloalkyle, C₃-C₇-cycloalkyl-C₁-C₃-alkylène, C₁-C₆-fluoroalkyle, aryle-C(O)-, C₁-C₆-alkyle-C(O)-, C₃-C₇-cycloalkyl-C(O)-, C₃-C₇-cycloalkyl-C₁-C₃-alkylène-C(O)-, C₁-C₆-fluoroalkyle-C(O)-, aryle-S(O), C₁-C₆-alkyle-S(O)-, C₁-C₆-fluoroalkyle-S(O)-, aryle-S(O)₂-, C₁-C₆-alkyle-S(O)₂-, C₁-C₆-fluoroalkyle-S(O)₂-, C₃-C₇-cycloalkyl-S(O)₂-, C₃-C₇-cycloalkyl-C₁-C₃-alkylène-S(O)₂-, C₁-C₆-alkyle-OC(O)-, aryle-C₁-C₃-alkyle-O-C(O)-, C₃-C₇-cycloalkyl-O-C(O)-, C₃-C₇-cycloalkyl-C₁-C₃-alkylène-O-C(O)-, C₁-C₆-fluoroalkyle-O-C(O)-, aryle-O-C(O)-, hétéroaryl-O-C(O)-, hétéroaryle ou aryle ; les groupes hétéroaryle et aryle étant éventuellement substitués par un ou plusieurs substituants R₉, identiques ou différents l'un de l'autre ; les groupes C₁-C₆-alkyle, C₃-C₇-cycloalkyle, C₃-C₇-cycloalkyl-C₁-C₃-alkylène, C₁-C₆-fluoroalkyle, pouvant être substitués par un groupe hydroxy, C₁-C₆-alcoxy ou NR₄R₅ ;
R₄ et R₅, représentent, indépendamment l'un de l'autre, un atome d'hydrogène ou un groupe C₁-C₆-alkyle, C₃-C₇-cycloalkyle, C₃-C₇-cycloalkyl-C₁-C₃-alkylène, aryle-C₁-C₅-alkylène ou aryle, ou R₄ et R₅ forment ensemble, avec l'atome d'azote qui les porte, un groupe azétidine, pyrrolidine, pipéridine, azépine, morpholine, thiomorpholine, pipérazine, homopipérazine ; le groupe NR₄R₅ étant éventuellement substitué par un groupe C₁-C₆-alkyle, C₃-C₇-cycloalkyle, C₃-C₇-cycloalkyl-C₁-C₃-alkylène, aryle-C₁-C₆-alkylène, aryle, hétéroaryle, aryle-S(O)₂-, C₁-C₆-alkyle-S(O)₂-, C₁-C₆-fluoroalkyle-S(O)₂, C₃-C₇-cycloalkyl-S(O)₂-, C₃-C₇-cycloalkyl-C₁-C₃-alkylène-S(O)₂-, aryle-C(O)-, C₁-C₆-alkyle-C(O)-, C₃-C₇-cycloalkyl-C(O)-, C₃-C₇-cycloalkyl-C₁-C₃-alkylène-C(O)-, C₁-C₆-fluoroalkyle-C(O)-, hydroxy, C₁-C₆-alkyloxy, C₃-C₇-cycloalkyloxy, C₃-C₇-cycloalkyl-C₁-C₃-alkylénoxy, C₁-C₆-fluoroalkyle, aryloxy-C₁-C₆-alkylène, aryloxy, hétéroaryloxy-C₁-C₆-alkylène, hétéroaryloxy ;
R₆ et R₇ représentent, indépendamment l'un de l'autre, un atome d'hydrogène, un groupe C₁-C₆-alkyle, C₃-C₇-cycloalkyle, C₃-C₇-cycloalkyl-C₁-C₃-alkylène, aryle-C₁-C₆-alkylène ou aryle ; le groupe aryle étant éventuellement substitué par un ou plusieurs substituants choisis parmi un atome d'halogène, un groupe C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₃-C₇-cycloalkyl-C₁-C₃-alkylénoxy, C₁-C₆-fluoroalcoxy, nitro ou cyano ; ou R₆ et R₇ forment ensemble un lactame de 4 à 7 chaînons comprenant l'atome d'azote et le groupe C(O) qui les portent ;
R₈ représente un groupe C₁-C₆-alkyle, C₃-C₇-cydoalkyle, C₃-C₇-cycloalkyl-C₁-C₃-alkylène, aryle-C₁-C₆-alkylène ou aryle ; le groupe aryle étant éventuellement substitué par un ou plusieurs substituants choisis parmi un atome d'halogène, un groupe C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₃-C₇-cycloalkyl-C₁-C₃-alkylénoxy, C₁-C₆-fluoroalcoxy, nitro ou cyano ;
ou R₆ et R₈ forment ensemble un sultame de 4 à 7 chaînons comprenant l'atome d'azote et le groupe S(O)₂ qui les portent ;
R₉ représente un atome d'halogène, un groupe C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₃-C₇-cycloalkyl-C₁-C₃-alkylénoxy, C₁-C₆-fluoroalcoxy ; ces groupes étant éventuellement substitués par un groupe OH, C₁-C₆-alcoxy ou NR₄R₅; ou bien R₉ représente un groupe nitro, cyano ou NR₄R₅ ;
le ou les atomes de soufre du composé de formule générale (I) pouvant être sous forme oxydée ;
le ou les atomes d'azote du composé de formule générale (**I**) pouvant être sous forme oxydée ;
à l'état de base ou de sel d'addition à un acide, ainsi qu'à l'état d'hydrate ou de solvate.

2. Composé de formule (**I**) selon la revendication 1, **caractérisé en ce que**
X₁, X₂, X₃, et X₄ représentent, indépendamment l'un de l'autre un groupe C-R₁ ; R₁
étant tel que défini dans la formule générale (**I**) selon la revendication 1 ;
à l'état de base ou de sel d'addition à un acide, ainsi qu'à l'état d'hydrate ou de solvate.

3. Composé de formule (I) selon la revendication 1, **caractérisé en ce que**
X₁, X₂, X₃ représentent un groupe C-R₁ ; X₄ représente un atome d'azote ;
R₁ étant tel que défini dans la formule générale (I) selon la revendication 1 ;
à l'état de base ou de sel d'addition à un acide, ainsi qu'à l'état d'hydrate ou de solvate.

4. Composé de formule (I) selon l'une quelconque des revendications 1 à 3,
**caractérisé en ce que**
R₁ est choisi parmi un atome d'hydrogène, un atome d'halogène, un groupe C₁-C₆-fluoroalkyle, -Si-(C₁-C₆-alkyl)₃ ;
à l'état de base ou de sel d'addition à un acide, ainsi qu'à l'état d'hydrate ou de solvate.

5. Composé de formule (I) selon l'une quelconque des revendications 1 à 4,
**caractérisé en ce que**
n est égal à 1 ;
à l'état de base ou de sel d'addition à un acide, ainsi qu'à l'état d'hydrate ou de solvate.

6. Composé de formule (I) selon l'une quelconque des revendications 1 à 5,
**caractérisé en ce que**
Y représente un aryle ou un hétéroaryle éventuellement substitué par un ou plusieurs groupes choisis parmi un atome d'halogène, un groupe C₁-C₆-alkyle, C₁-C₆-fluoroalkyle ;
à l'état de base ou de sel d'addition à un acide, ainsi qu'à l'état d'hydrate ou de solvate.

7. Composé de formule (I) selon l'une quelconque des revendications 1 à 6,
**caractérisé en ce que**
W représente un atome d'oxygène ;
à l'état de base ou de sel d'addition à un acide, ainsi qu'à l'état d'hydrate ou de solvate.

8. Composé de formule (I) selon l'une quelconque des revendications 1 à 7,
**caractérisé en ce que**
A représente le groupe de formule : 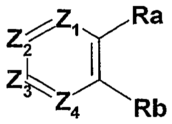 où A est choisi parmi les groupes 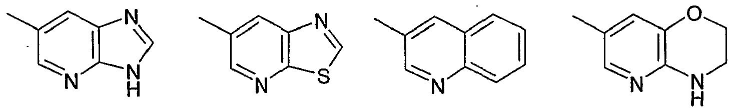 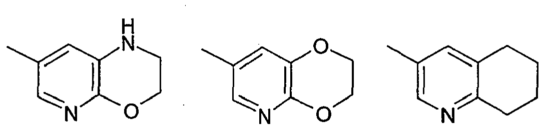 ces groupes étant éventuellement substitués par R₂ et R₃ comme défini dans la formule générale (I) ci-dessus ;
R₂ représente un atome d'hydrogène ;
R₃, représente, lorsqu'il est porté par un atome de carbone, un atome d'hydrogène, un groupe hydroxy, C₁-C₆-alkyle, oxo ; le groupe C₁-C₆-alkyle pouvant être substitué par un groupe hydroxy ou -OC(O)-C₁-C₆-alkyle ;
ou
R₃, représente, lorsqu'il est porté par un atome d'azote, un atome d'hydrogène ou un groupe C₁-C₆-alkyle ;
à l'état de base ou de sel d'addition à un acide, ainsi qu'à l'état d'hydrate ou de solvate.

9. Composé de formule (I) selon l'une quelconque des revendications 1 à 8,
**caractérisé en ce que**
X₁, X₂, X₃, et X₄ représentent, indépendamment l'un de l'autre un groupe C-R₁ ; ou bien X₁, X₂, X₃ représentent un groupe C-R₁ ; X₄ représente un atome d'azote ;
R₁ est choisi parmi un atome d'hydrogène, un atome d'halogène, un groupe C₁-C₆-fluoroalkyle, -Si-(C₁-C₆-alkyl)₃;
n est égal à 1 ;
Y représente un aryle ou un hétéroaryle éventuellement substitué par un ou plusieurs groupes choisis parmi un atome d'halogène, un groupe C₁-C₆-alkyle, C₁-C₆-fluoroalkyle ;
W représente un atome d'oxygène ;
A représente le groupe de formule : 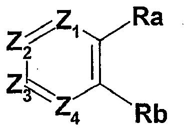 où.A est choisi parmi les groupes 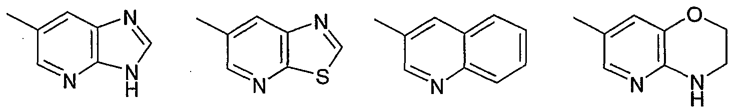 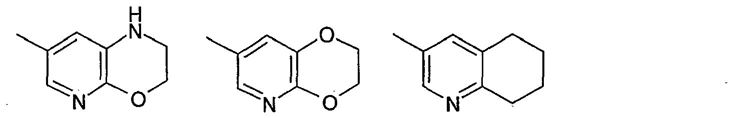 ces groupes étant éventuellement substitués par R₂ et R₃ comme défini dans la formule générale (I) ci-dessus ;
R₂ représente un atome d'hydrogène ;
R₃, représente, lorsqu'il est porté par un atome de carbone, un atome d'hydrogène, un groupe hydroxy, C₁-C₆-alkyle, oxo ; le groupe C₁-C₆-alkyle pouvant être substitué par un groupe hydroxy ou -OC(O)-C₁-C₆-alkyle ;
ou
R₃, représente, lorsqu'il est porté par un atome d'azote, un atome d'hydrogène ou un groupe C₁-C₆-alkyle ;
à l'état de base ou de sel d'addition à un acide, ainsi qu'à l'état d'hydrate ou de solvate.

10. Composé de formule (I) selon la revendication 1 choisi parmi :
*N*-(2,3-Diméthyl-3*H*-imidazo[4,5-*b*]pyridin-6-yl)-5-fluoro-1-[(3-fluorophényl)méthyl]-1*H-*indole-2-carboxamide ;
*N*-(2-Méthyl-thiazolo[5,4-*b*]pyridin-6-yl)-5-fluoro-1-[(3-fluorophényl)méthyl]-1*H*-indole-2-carboxamide ;
*N*-(Thiazolo[5,4-*b*]pyridin-6-yl)-5-fluoro-1-[(3-fluorophényl)méthyl]-1*H*-indole-2-carboxamide ;
*N*-(2-Hydroxyméthyl-thiazolo[5,4-*b*]pyridin-6-yl)-5-fluoro-1-[(3-fluorophényl)méthyl]-1*H-*indole-2-carboxamide ;
*N*-(Quinoléin-3-yl)-5-fluoro-1-[(3-fluorophényl)méthyl]-1*H*-indole-2-carboxamide ;
*N*-(2-Hydroxyméthyl-3-méthyl-3*H*-imidazo[4,5-*b*]pyridin-6-yl)-5-fluoro-1-[(3-fluorophényl)méthyl]-1*H*-indole-2-carboxamide;
*N*-(3-Méthyl-3*H*-imidazo[4,5-*b*]pyridin-6-yl)-5-fluoro-1-[(3-fluorophényl)méthyl]-1*H-*indole-2-carboxamide;
*N*-(2,3-Diméthyl-3*H*-imidazo[4,5-*b*]pyridin-6-yl)-5-trifluorométhyl-1-[(3-fluorophényl)méthyl]-1*H*-pyrrolo[2,3-*b*]pyridine-2-carboxamide;
*N*-(3,4-Dihydro-2H-pyrido[3,2-*b*][1,4]oxazin-7-yl)-5-fluoro-1-[(3-fluorophényl)méthyl]-1*H-*indole-2-carboxamide;
*N*-(4-Méthyl-3,4-dihydro-2H-pyrido[3,2-*b*][1,4]oxazin-7-yl)-5-fluoro-1-[(3-fluorophényl)méthyl]-1*H*-indole-2-carboxamide;
*N*-(2-Méthyl-3*H*-imidazo[4,5-*b*]pyridin-6-yl)-5-fluoro-1-[(3-fluorophényl)méthyl]-1*H-*indole-2-carboxamide;
*N*-(-Méthyl-2-oxo-2,3-dihydro-1H-pyrido[2,3-*b*][1,4]oxazin-7-yl)-5-fluoro-1-[(3-fluorophényl)méthyl]-1*H*-indole-2-carboxamide;
2,2-Diméthylpropanoate de [6-[[[5-fluoro-1-[(3-fluorophényl)méthyl]-1*H*-indol-2-yl]carbonyl]amino]thiazolo[5,4-*b*]pyridin-2-yl]méthyle;
*N*-(2,3-dihydro-[1,4]dioxino[2,3-*b*]pyridin-7-yl))-5-fluoro-1-[(3-fluorophényl)méthyl]-1*H-*indole-2-carboxamide;
*N*-(6-Hydroxy-5,6,7,8-tétrahydroquinoléin-3-yl)-5-fluoro-1-[(3-fluorophényl)méthyl]-1*H-*indole-2-carboxamide;
*N*-(2-Hydroxyméthyl-thiazolo[5,4-*b*]pyridin-6-yl)-5-trifluorométhyl-1-[(3-méthylphényl)méthyl]-1*H*-indole-2-carboxamide;
*N*-(2-Hydroxyméthyl-thiazolo[5,4-*b*]pyridin-6-yl)-6-trifluorométhyl-1-[(3-méthylphényl)méthyl]-1*H*-indole-2-carboxamide;
*N*-(2-Hyd roxyméthyl-thiazolo[5,4-*b*]pyridin-6-yl)-5-tri méthylsilyl-1-[(3-méthylphényl)méthyl]-1*H*-indole-2-carboxamide;
*N*-(2-Hydroxyméthyl-thiazolo[5,4-*b*]pyridin-6-yl)-6-fluoro-1-[(3-méthylphényl)méthyl]-1*H-*indole-2-carboxamide;
*N*-(2-Hydroxyméthyl-thiazolo[5,4-*b*]pyridin-6-yl)-5-trifluorométhyl-1-[(thiazol-2-yl)méthyl]-1*H*-indole-2-carboxamide;
*N*-(2-Hydroxyméthyl-thiazolo[5,4-*b*]pyridin-6-yl)-6-trifluorométhyl-1-[(thiazol-2-yl)méthyl]-1*H*-indole-2-carboxamide;
*N*-(2-Hydroxyméthyl-thiazolo[5,4-*b*]pyridin-6-yl)-5-triméthylsilyl-1-[(thiazol-2-yl)méthyl]-1*H*-indole-2-carboxamide;
*N*-(2-Hydroxyméthyl-thiazolo[5,4-*b*]pyridin-6-yl)-6-fluoro-1-[(thiazol-2-yl)méthyl]-1*H-*indole-2-carboxamide;
*N*-(2-Hydroxyméthyl-thiazolo[5,4-*b*]pyridin-6-yl)-5-trifluorométhyl-1-[(thiazol-2-yl)méthyl]-1*H*-pyrrolo[2,3-*b*]pyridine-2-carboxamide;
*N*-(2-Hydroxyméthyl-thiazolo[5,4-*b*]pyridin-6-yl)-6-triméthylsilyl-1-[(thiazol-2-yl)méthyl]-1*H*-indole-2-carboxamide;
*N*-(2-Hydroxyméthyl-thiazolo[5,4-*b*]pyridin-6-yl)-5-trifluorométhyl-1-[(pyridin-4-yl)méthyl]-1*H*-indole-2-carboxamide;
*N*-(2-Hydroxyméthyl-thiazolo[5,4-*b*]pyridin-6-yl)-6-trifluorométhyl-1-[(pyridi n-4-yl)méthyl]-1*H*-indole-2-carboxamide;
*N*-(2-Hydroxyméthyl-thiazolo[5,4-*b*]pyridin-6-yl)-5-triméthylsilyl-1-[(pyridin-4-yl)méthyl]-1*H*-indole-2-carboxamide;
*N*-(2-Hydroxyméthyl-thiazolo[5,4-*b*]pyridin-6-yl)-6-fluoro-1-[(pyridin-4-yl)méthyl]-1*H-*indole-2-carboxamide;
*N*-(2-Hydroxyméthyl-thiazolo[5,4-*b*]pyridin-6-yl)-5-trifluorométhyl-1-[(pyridin-4-yl)méthyl]-1*H*-pyrrolo[2,3-*b*]pyridine-2-carboxamide;
*N*-(2-Hydroxyméthyl-thiazolo[5,4-*b*]pyridin-6-yl)-6-triméthylsilyl-1-[(pyridin-4-yl)méthyl]-1*H*-indole-2-carboxamide;
*N*-(2-Hydroxyméthyl-thiazolo[5,4-*b*]pyridin-6-yl)-5-triméthylsilyl-1-[[(3-trifluorométhyl)phényl]méthyl]-1*H*-indole-2-carboxamide;
*N*-(2-Hydroxyméthyl-thiazolo[5,4-*b*]pyridin-6-yl)-6-fluoro-1-[[(3-trifluorométhyl)phényl]méthyl]-1*H*-indole-2-carboxamide;
*N*-(2-Hydroxyméthyl-thiazolo[5,4-*b*]pyridin-6-yl)-5-trifluorométhyl-1-[[(3-trifluorométhyl)phényl]méthyl]-1*H*-pyrrolo[2,3-*b*]pyridine-2-carboxamide;
*N*-(2-Hydroxyméthyl-thiazolo[5,4-*b*]pyridin-6-yl)-6-triméthylsilyl-1-[[(3-trifluorométhyl)phényl]méthyl]-1*H*-indole-2-carboxamide;
*N*-(2-Hydroxyméthyl-thiazolo[5,4-*b*]pyridin-6-yl)-5-trifluorométhyl-1-[(3-méthylphényl)méthyl]-1*H*-pyrrolo[2,3-*b*]pyridine-2-carboxamide;
*N*-(2-Hydroxyméthyl-thiazolo[5,4-*b*]pyridin-6-yl)-6-triméthylsilyl-1-[(3-méthylphényl)méthyl]-1*H*-indole-2-carboxamide;
*N*-(2-Hydroxyméthyl-thiazolo[5,4-*b*]pyridin-6-yl)-5-trifluorométhyl-1-[[(3-trifluorométhyl)phényl]méthyl]-1*H*-indole-2-carboxamide;
*N*-(2-Hydroxyméthyl-thiazolo[5,4-*b*]pyridin-6-yl)-6-trifluorométhyl-1-[[(3-trifluorométhyl)phényl]méthyl]-1*H*-indole-2-carboxamide.

11. Procédé de préparation d'un composé de formule (I) selon l'une quelconque des revendications 1 à 10, **caractérisé en ce que** l'on fait réagir un composé de formule générale (II) 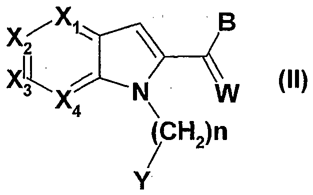 dans laquelle X₁, X₂, X₃, X₄, n, Y et W sont tels que définis dans la formule générale (I) selon la revendication 1,
avec composé de formule générale (III), 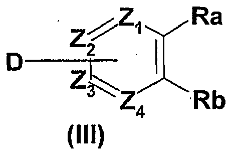 dans laquelle Z₁, Z₂, Z₃, Z₄, Ra et Rb sont tels que définis dans la formule générale (I) selon la revendication 1,
- lorsque B représente un groupe hydroxyle et D représente un groupe amino, en présence d'un agent de couplage dans un solvant ;
- lorsque B représente un atome de chlore et D représente un groupe amino, par réaction en solution dans un solvant ;
- lorsque B représente un groupe C₁-C₆-alcoxy, C₃-C₇-cycloalkyloxy, C₃-C₇-cycloalkyl-C₁-C₃-alkylènoxy- ou aryle-C₁-C₃-alkylènoxy et D représente un groupe amino, par transformation du composé de formule (III) en amidure par action préalable du triméthylaluminium sur les amines de formule générale (III), puis par réaction de l'amidure obtenu avec le composé de formule (II) au reflux d'un solvant ;
- lorsque B représente un groupe NH₂, W représente un atome d'oxygène et D correspond à un groupe partant, en présence d'un sel de cuivre en quantité catalytique, d'une quantité catalytique d'un ligand du cuivre, et d'une base dans un solvant.

12. Procédé de préparation d'un composé de composé de formule générale (II) 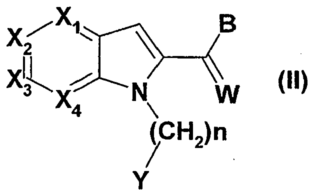 dans laquelle X₁, X₂, X₃, X₄, n, Y et W sont tels que définis dans la formule générale (I) selon la revendication 1, l'un des X₁, X₂, X₃ ou X₄ correspondant à un groupe C-R₁ où R₁ représente un groupe -Si-(C₁-C₆-alkyl)₃ tel que défini dans la formule générale (I) selon la revendication 1 et B représente un groupe C₁-C₆-alcoxyle,
**caractérisé en ce que** l'on fait réagir un composé de formule générale (VI) 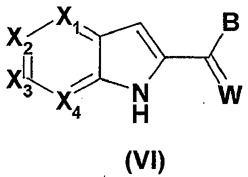 dans laquelle X₁, X₂, X₃, X₄ et W sont tels que définis dans la formule générale (I) selon la revendication 1 et B représente un groupe C₁-C₆-alcoxyle
avec un composé de formule générale (VIII) 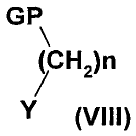 dans laquelle Y et n sont tels que définis dans la formule générale (I) selon la revendication 1,
- quand n est égal à 1, 2 ou 3, avec un réactif de formule générale (VIII) dans lequel GP représente un groupe partant, en présence d'une base dans un solvant polaire ;
- quand n est égal à 1, 2 ou 3, avec un réactif de formule générale (VIII) dans lequel GP représente un groupe hydroxyle, en présence d'une phosphine et de l'azodicarboxylate de diéthyle en solution dans un solvant ; ou bien en présence d'une phosphine supportée sur une résine et de l'azodicarboxylate de diisopropyle en solution dans un solvant ;
- quand n est égal à 0, avec un réactif de formule générale (VIII) dans lequel GP représente un groupe partant, sous atmosphère inerte en milieu basique, en présence d'un sel de cuivre dans un solvant organique.

13. Composé de formule générale (IIa), (IIb), (IIc), (IId), (IIe), (IIf), (IIg) ou (IIh) :

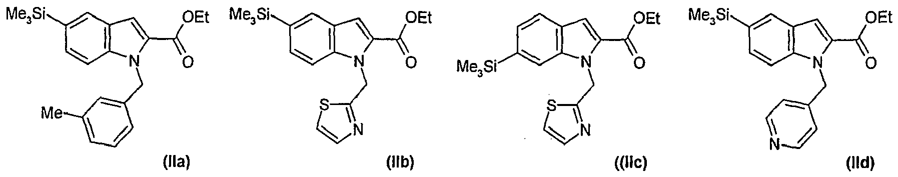

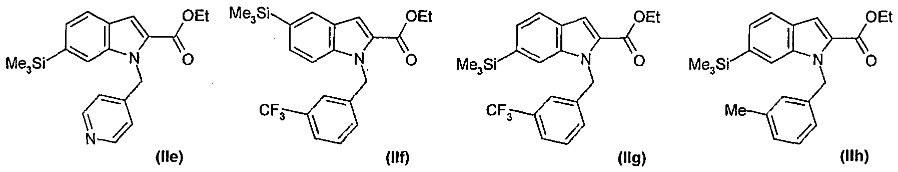

14. Médicament, **caractérisé en ce qu'**il comprend un composé de formule (I) selon l'une quelconque des revendications 1 à 10, ou un sel d'addition de ce composé à un acide pharmaceutiquement acceptable, ou encore un hydrate ou un solvate du composé de formule (I).

15. Composition pharmaceutique, **caractérisée en ce qu'**elle comprend un composé de formule (I) selon l'une quelconque des revendications 1 à 10, ou un sel pharmaceutiquement acceptable, un hydrate ou un solvate de ce composé, ainsi qu'au moins un excipient pharmaceutiquement acceptable.

16. Utilisation d'un composé de formule (I) selon l'une quelconque des revendications 1 à 10 pour la préparation d'un médicament destiné à la prévention et au traitement des pathologies dans lesquelles les récepteurs de type TRPV1 sont impliqués.

17. Utilisation d'un composé de formule (I) selon l'une quelconque des revendications 1 à 10 pour la préparation d'un médicament destiné à prévenir ou à traiter la douleur, l'inflammation, les désordres métaboliques, les désordres urologiques, les désordres gynécologiques, les désordres gastro-intestinaux, les désordres respiratoires, le psoriasis, le pruritis, les irritations dermiques, des yeux ou des muqueuses, l'herpès, le zona, la sclérose en plaques, la dépression, les cancers.

## Patentansprüche

1. Verbindung der Formel (I), 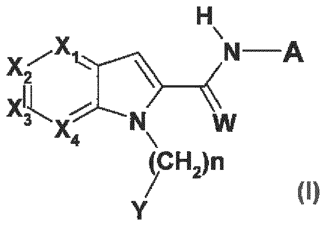 mit den folgenden Bedeutungen:
X₁, X₂, X₃ und X₄ bedeuten unabhängig voneinander ein Stickstoffatom oder eine C-R₁-Gruppe;
mit der Maßgabe, dass, wenn eines von X₁, X₂, X₃ und X₄ ein Stickstoffatom bedeutet, die anderen eine C-R₁-Gruppe bedeuten;
W bedeutet ein Sauerstoff- oder Schwefelatom;
n ist gleich 0, 1, 2 oder 3;
Y bedeutet ein Aryl oder ein Heteroaryl, das gegebenenfalls durch eine oder mehrere Gruppen, ausgewählt aus einem Halogenatom, einer C₁-C₆-Alkyl-, C₃-C₇-Cycloalkyl-, C₃-C₇-Cycloalkyl-C₁-C₃-alkylen-, C₁-C₆-Fluoralkyl-, Hydroxyl-, C₁-C₆-Alkoxy-, C₃-C₇-Cycloalkyloxy-, C₃-C₇-Cycloalkyl-C₁-C₆-alkylen-O-, C₁-C₆-Fluoralkoxy-, Cyano-, C(O)NR₄R₅-, Nitro-, NR₄R₅-, C₁-C₆-Thioalkyl-, Thiol-, -S (O) -C₁-C₆-Alkyl-, -S (O)₂-C₁-C₆-Alkyl-, SO₂NR₄R₅-, NR₆C(O) R₇-, NR₆SO₂R₈-, C(O) NR₄R₅-, OC(O)NR₄R₅-,
-Si- (C₁-C₆-alkyl)₃-, -SF₅-, Aryl-C₁-C₅-alkylen- oder Aryl-, Heteroaryl-C₁-C₅-alkylen- oder Heteroaryl-Gruppe; wobei die C₁-C₆-Alkyl-, C₃-C₇-Cycloalkyl-, C₃-C₇-Cycloalkyl-C₁-C₃-alkylen-, C₁-C₆-Fluoralkyl-, C₁-C₆-Alkoxy-, C₃-C₇-Cycloalkyloxy-, C₃-C₇-Cycloalkyl-C₁-C₆-alkylen-O-Gruppen gegebenenfalls durch eine Hydroxyl-, C₁-C₆-Alkoxy- oder NR₄R₅-Gruppe substituiert sind; wobei die Aryl- und Heteroarylgruppen gegebenenfalls durch einen oder mehrere Substituenten R₉, die gleich oder voneinander verschieden sind, substituiert sind;
A bedeutet die Gruppe der Formel: 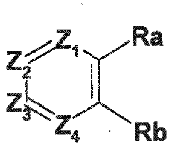
Z₁, Z₂, Z₃ und Z₄ bedeuten unabhängig voneinander ein Stickstoffatom, ein Kohlenstoffatom oder eine C-R₂-Gruppe,
wobei mindestens eines von Z₁, Z₂, Z₃ und Z₄ ein Stickstoffatom bedeutet und
eines von Z₁, Z₂, Z₃ und Z₄ ein Kohlenstoffatom bedeutet, das an das Amidstickstoffatom oder Thioamidstickstoffatom der Formel (I) gebunden ist;
Ra und Rb bedeuten zusammen mit den Kohlenstoffatomen, die sie tragen,
entweder ein teilweise ungesättigtes Cycloalkyl, oder ein Aryl;
oder einen Heterocyclus, oder ein Heteroaryl mit 5 bis 7 Ringgliedern, umfassend ein bis drei Heteroatome aus der Reihe O, S und N;
mit der Maßgabe, dass, wenn Ra und Rb zusammen mit den Kohlenstoffatomen, die sie tragen, einen Ring mit 5 Ringgliedern bilden, dieser Ring, der ein Stickstoffatom und Kohlenstoffatome umfasst und teilweise gesättigt oder ungesättigt ist, ausgeschlossen ist;
wobei das teilweise ungesättigte Cycloalkyl, das Aryl, der Heterocyclus oder das Heteroaryl durch einen oder mehrere Substituenten R₃ substituiert sein können;
R₁ ist aus einem Wasserstoffatom, einem Halogenatom, einer C₁-C₆-Alkyl-, C₃-C-₇-Cycloalkyl-, C₃-C₇-Cycloalkyl-C₁-C₃-alkylen-, C₁-C₆-Fluoralkyl-, Aryloxy-C₁-C₆-alkyl-, Heteroaryloxy-C₁-C₆-alkyl-, Aryl-C₁-C₃-alkylenoxy-C₁-C₆-alkyl-, Heteroaryl-C₁-C₃-alkylenoxy-C₁-C₆-alkyl-, Arylthio-C₁-C₆-alkyl-, Heteroarylthio-C₁-C₆-alkyl-, Aryl-C₁-C₃ - alkylenthio-C₁-C₆-alkyl-, Heteroaryl-C₁-C₃-alkylenthio-C₁-C₆-alkyl-, C₁-C₆-Alkoxy-, C₃-C₇-Cycloalkyloxy-, C₃-C₇-Cycloalkyl-C₁-C₃-alkylenoxy-, C₁-C₆-Fluoralkoxy-, Cyano-, C(O)NR₄R₅-, Nitro-, NR₄R₅-, C₁-C₆-Thioalkyl-, C₃-C₇-Cycloalkylthio-, C₃-C₇-Cycloalkyl-C₁-C₃-alkylenthio-, -S (O)-C₁-C₆-Alkyl-, -S (O) -C₃-C₇-Cycloalkyl-, -S(O) -C₁-C₃-Alkylen-C₃-C₇-cycloalkyl-, C₁-C₆-Alkyl-S(O)₂-,C₁-C₆-Fluoralkyl-S(O)₂-, C₃-C₇-Cycloalkyl-S(O)₂-, C₃-C₇-Cycloalkyl-C₁-C₃-alkylen-S (O)₂-, SO₂NR₄R₅-, -Si- (C₁-C₆-Alkyl)₃-, SF₅-, NR₆C(O)R₇-, NR₆SO₂R₈-, C(O)NR₄R₅-, OC(O)NR₄R₅-, Aryl-, Heteroaryl-, Aryl-C₁-C₅-alkylen-, Heteroaryl-C₁-C₅-alkylen-, Aryloxy-, Arylthio-, Heteroaryloxy- oder Heteroarylthio-Gruppe ausgewählt; wobei die Heteroaryl- oder Arylgruppen gegebenenfalls durch einen oder mehrere Substituenten R₉, die identisch oder voneinander verschieden sind, substituiert sind;
R₂ bedeutet ein Wasserstoffatom, ein Halogenatom, eine C₁-C₆-Alkyl-, C₃-C₇-Cycloalkyl-, C₃-C₇-Cycloalkyl-C₁-C₃-alkylen-, C₁-C₆-Fluoralkyl-,C₁-C₆-Alkoxy-, C₃-C₇-Cycloalkyloxy-, C₃-C₇-Cycloalkyl-C₁-C₃-alkylenoxy-, Hydroxyl-, Thiol- oder C₁-C₆-Fluoralkoxy-Gruppe;
R₃, wenn es von einem Kohlenstoffatom getragen wird, bedeutet ein Wasserstoffatom, eine Hydroxyl-, Thiol-, C₁-C₆-Alkyl-, C₃-C₇-Cycloalkyl-, C₃-C₇-Cycloalkyl-C₁-C₃-alkylen-, C₁-C₆-Fluoralkyl-, C₁-C₆-Alkoxy-, C₃-C₇-Cycloalkyloxy-, C₃-C₇-Cycloalkyl-C₁-C₃-alkylenoxy-, C₁-C₆-Alkoxy-C₁-C₃-alkylen-, C₃-C₇-Cycloalkyloxy-C₁-C₃-alkylen-, C₃-C₇-Cycloalkyl-C₁-C₃-alkylenoxy-C₁-C₃-alkylen-, C(O)NR₄R₅-, C(O)O-C₁-C₆-Alkyl-, CO₂H-, Oxo- oder Thio-Gruppe; die C₁-C₆-Alkyl-, C₃-C₇-Cycloalkyl-, C₃-C₇-Cycloalkyl-C₁-C₃-alkylen-, C₁-C₆-Fluoralkyl-, C₁-C₆-Alkoxy-, C₃-C₇-Cycloalkyloxy-, C₃-C₇-Cycloalkyl-C₁-C₃-alkylenoxy-, C₁-C₆-Alkoxy-C₁-C₃-alkylen-, C₃-C₇-Cycloalkyloxy-C₁-C₃-alkylen-, C₃-C₇-Cycloalkyl-C₁-C₃-alkylenoxy-C₁-C₃-alkylen-Gruppen können durch eine Hydroxyl-, C₁-C₆-Alkoxy-, -OC(O)-C₁-C₆-Alkyl- oder NR₄R₅-Gruppe substituiert sein; oder
R₃, wenn es von einem Stickstoffatom getragen wird, bedeutet ein Wasserstoffatom, eine C₁-C₆-Alkyl-, C₃-C₇-Cycloalkyl-, C₃-C₇-Cycloalkyl-C₁-C₃-alkylen-, C₁-C₆-Fluoralkyl-, Aryl-C(O)-, C₁-C₆-Alkyl-C(O)-, C₃-C₇-Cycloalkyl-C(O) -, C₃-C₇-Cycloalkyl-C₁-C₃-alkylen-C(O)-, C₁-C₆-Fluoralkyl-C(O)-, Aryl-S(O)-, C₁-C₆-Alkyl-S(O)-, C₁-C₆-Fluoralkyl-S(O)-, Aryl-S(O)₂-, C₁-C₆-Alkyl-S(O)₂-, C₁-C₆-Fluoralkyl-S(O)₂-, C₃-C₇-Cycloalkyl-S(O)₂-, C₃-C₇-Cycloalkyl- C₁-C₃-alkylen-S(O)₂-, C₁-C₆-Alkyl-O-C(O) -, Aryl-C₁-C₃-alkyl-O-C(O)-, C₃-C₇-Cycloalkyl-O-C(O)-, C₃-C₇-Cycloalkyl-C₁-C₃-alkylen-O-C(O)-, C₁-C₆-Fluoralkyl-O-C(O)-, Aryl-O-C(O)-, Heteroaryl-O-C(O)-, Heteroaryl- oder Aryl-Gruppe; wobei die Heteroaryl- und Arylgruppen gegebenenfalls durch einen oder mehrere Substituenten R₉, die identisch oder voneinander verschieden sind, substituiert sind; wobei die C₁-C₆-Alkyl-, C₃-C₇-Cycloalkyl-, C₃-C₇-Cycloalkyl-C₁-C₃-alkylen- und C₁-C₆-Fluoralkyl-Gruppen gegebenenfalls durch eine Hydroxyl-, C₁-C₆-Alkoxy- oder NR₄R₅-Gruppe substituiert sein können;
R₄ und R₅ unabhängig voneinander bedeuten ein Wasserstoffatom oder eine C₁-C₆-Alkyl-, C₃-C₇-Cycloalkyl-, C₃-C₇-Cycloalkyl-C₁-C₃-alkylen-, Aryl-C₁-C₅-alkylen- oder Aryl-Gruppe, oder R₄ und R₅ gemeinsam mit dem Stickstoffatom, das sie trägt, bilden eine Azetidin-, Pyrrolidin-, Piperidin-, Azepin-, Morpholin-, Thiomorpholin-, Piperazin- oder Homopiperazin-Gruppe; wobei die NR₄R₅-Gruppe gegebenenfalls durch eine C₁-C₆-Alkyl-, C₃-C₇-Cycloalkyl-, C₃-C₇-Cycloalkyl-C₁-C₃-alkylen-, Aryl-C₁-C₆-alkylen-, Aryl-, Heteroaryl-, Aryl-S(O)2-, C₁-C₆-Alkyl-S(O)₂-, C₁-C₆-Fluoralkyl-S(O)₂-, C₃-C₇-Cycloalkyl-S(O)₂-, C₃-C₇-Cycloalkyl-C₁-C₃-alkylen-S(O)₂-, Aryl-C(O)-, C₁-C₆-Alkyl-C (O) -, C₃-C₇-Cycloalkyl-C(O)-, C₃-C₇-Cycloalkyl-Cᵢ-C₃-alkylen-C(0)-, C₁-C₆-Fluoralkyl-C(O)-, Hydroxyl-, C₁-C₆-Alkyloxy-, C₃-C₇-Cycloalkyloxy-, C₃-C₇-Cycloalkyl-C₁-C₃-alkylenoxy-, C₁-C₆-Fluoralkyl-, Aryloxy-C₁-C₆-alkylen-, Aryloxy-, Heteroaryloxy-C₁-C₆-alkylen- oder Heteroaryloxy-Gruppe substituiert ist;
R₆ und R₇ bedeuten unabhängig voneinander ein Wasserstoffatom, eine C₁-C₆-Alkyl-, C₃-C₇-Cycloalkyl-, C₃-C₇-Cycloalkyl-C₁-C₃-alkylen-, Aryl-C₁-C₆-alkylen- oder Aryl-Gruppe; wobei die Arylgruppe gegebenenfalls durch einen oder mehrere Substituenten, ausgewählt aus einem Halogenatom, einer C₁-C₆-Alkyl-, C₃-C₇-Cycloalkyl-, C₃-C₇-Cycloalkyl-C₁-C₃-alkylen-, C₁-C₆-Fluoralkyl-, C₁-C₆-Alkoxy-, C₃-C₇-Cycloalkyloxy-, C₃-C₇-Cycloalkyl-C₁-C₃-alkylenoxy-, C₁-C₆-Fluoralkoxy-, Nitro- oder Cyano-Gruppe, substituiert ist;
oder R₆ und R₇ bilden zusammen ein Lactam mit 4 bis 7 Ringgliedern, das das Stickstoffatom und die C(O)-Gruppe, die sie trägt, umfasst;
R₈ bedeutet eine C₁-C₆-Alkyl-, C₃-C₇-Cycloalkyl-, C₃-C₇-Cycloalkyl-C₁-C₃-alkylen-, Aryl-C₁-C₆-alkylen- oder Aryl-Gruppe; wobei die Arylgruppe gegebenen-falls durch einen oder mehrere Substituenten, ausgewählt aus einem Halogenatom, einer C₁-C₆-Alkyl-, C₃-C₇-Cycloalkyl-, C₃-C₇-Cycloalkyl-C₁-C₃-alkylen-, C₁-C₆-Fluoralkyl-, C₁-C₆-Alkoxy-, C₃-C₇-Cycloalkyloxy-, C₃-C₇-Cycloalkyl-C₁-C₃-alkylenoxy-, C₁-C₆-Fluoralkoxy-, Nitro- oder Cyano-Gruppe, substituiert ist;
oder R₆ und R₈ bilden zusammen ein Sultam mit 4 bis 7 Ringgliedern, das das Stickstoffatom und die S(O)₂-Gruppe, die sie trägt, umfasst;
R₉ bedeutet ein Halogenatom, eine C₁-C₆-Alkyl-, C₃-C₇-Cycloalkyl-, C₃-C₇-Cycloalkyl-C₁-C₃-alkylen-, C₁-C₆-Fluoralkyl-, C₁-C₆-Alkoxy-, C₃-C₇-Cycloalkyl-oxy-, C₃-C₇-Cycloalkyl-C₁-C₃-alkylenoxy-, C₁-C₆-Fluoralkoxy-Gruppe; wobei diese Gruppen gegebenenfalls durch eine OH-, C₁-C₆-Alkoxy- oder NR₄R₅-Gruppe substituiert sind; oder R₉ bedeutet eine Nitro-, Cyano- oder NR₄R₅-Gruppe;
wobei das oder die Schwefelatom(e) der Verbindung der allgemeinen Formel (I) in oxidierter Form vorliegen kann/können;
wobei das oder die Stickstoffatom(e) der Verbindung der allgemeinen Formel (I) in oxidierter Form vorliegen kann/können;
als Base oder Säureadditionssalz sowie als Hydrat oder Solvat.

2. Verbindung der Formel (I) nach Anspruch 1, **dadurch gekennzeichnet, dass**
X₁, X₂, X₃ und X₄ unabhängig voneinander eine C-R₁-Gruppe bedeuten; wobei R₁ wie in der allgemeinen Formel (I) nach Anspruch 1 definiert ist;
als Base oder Säureadditionssalz sowie als Hydrat oder Solvat.

3. Verbindung der Formel (I) nach Anspruch 1, **dadurch gekennzeichnet, dass**
X₁, X₂, X₃ eine C-R₁-Gruppe bedeuten; X₄ ein Stick-stoffatom bedeutet; wobei R1 wie in der allgemeinen Formel (I) nach Anspruch 1 definiert ist; als Base oder Säureadditionssalz sowie als Hydrat oder Solvat.

4. Verbindung der Formel (I) nach einem der Ansprüche 1 bis 3, **dadurch gekennzeichnet, dass** R₁ aus einem Wasserstoffatom, einem Halogenatom, einer C₁-C₆-Fluoralkyl- oder -Si- (C₁-C₆-Alkyl)₃-Gruppe ausgewählt ist;
als Base oder Säureadditiönssalz sowie als Hydrat oder Solvat.

5. Verbindung der Formel (I) nach einem der Ansprüche 1 bis 4, **dadurch gekennzeichnet, dass** n gleich 1 ist;
als Base oder Säureadditionssalz sowie als Hydrat oder Solvat.

6. Verbindung der Formel (I) nach einem der Ansprüche 1 bis 5, **dadurch gekennzeichnet, dass** Y ein Aryl oder Heteroaryl, das gegebenenfalls durch eine oder mehrere Gruppen, ausgewählt aus einem Halogenatom, einer C₁-C₆-Alkyl- oder C₁-C₆-Fluoralkyl-Gruppe, substituiert ist, bedeutet;
als Base oder Säureadditionssalz sowie als Hydrat oder Solvat.

7. Verbindung der Formel (I) nach einem der Ansprüche 1 bis 6, **dadurch gekennzeichnet, dass** W ein Sauerstoffatom bedeutet;
als Base oder Säureadditionssalz sowie als Hydrat oder Solvat.

8. Verbindung der Formel (I) nach einem der Ansprüche 1 bis 7, **dadurch gekennzeichnet, dass** A die Gruppe der Formel: 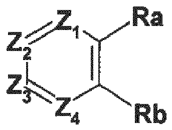 bedeutet,
wobei A aus den Gruppen 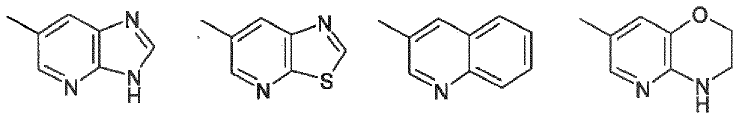 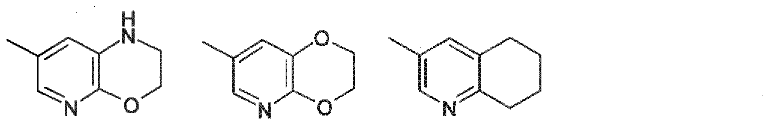 ausgewählt ist, wobei diese Gruppen gegebenenfalls durch R₂ und R₃ wie in der allgemeinen Formel (I) oben definiert substituiert sind;
R₂ ein Wasserstoffatom bedeutet;
R₃, wenn es von einem Kohlenstoffatom getragen wird, ein Wasserstoffatom, eine Hydroxyl-, C₁-C₆-Alkyl- oder Oxo-Gruppe bedeutet; wobei die C₁-C₆-Alkylgruppe durch eine Hydroxyl- oder -OC(O)-C₁-C₆-Alkyl-Gruppe substituiert sein kann;
oder
R₃, wenn es von einem Stickstoffatom getragen wird, ein Wasserstoffatom oder eine C₁-C₆-Alkylgruppe bedeutet;
als Base oder Säureadditionssalz sowie als Hydrat oder Solvat.

9. Verbindung der Formel (I) nach einem der Ansprüche 1 bis 8, **dadurch gekennzeichnet, dass** X₁, X₂, X₃ und X₄ unabhängig voneinander, eine C-R₁-Gruppe bedeuten; oder X₁, X₂ und X₃ eine C-R₁-Gruppe bedeuten; X₄ ein Stickstoffatom bedeutet;
R₁ aus einem Wasserstoffatom, einem Halogenatom, einer C₁-C₆-Fluoralkyl- oder -Si- (C₁-C₆-Alkyl)₃-Gruppe ausgewählt ist;
n gleich 1 ist;
Y ein Aryl oder Heteroaryl, das gegebenenfalls durch eine oder mehrere Gruppen, ausgewählt aus einem Halogenatom, einer C₁-C₆-Alkyl- oder C₁-C₆-Fluoralkyl-Gruppe substituiert ist, bedeutet; W ein Sauerstoffatom bedeutet;
A die Gruppe der Formel: 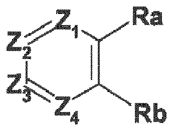 bedeutet,
wobei A aus den Gruppen 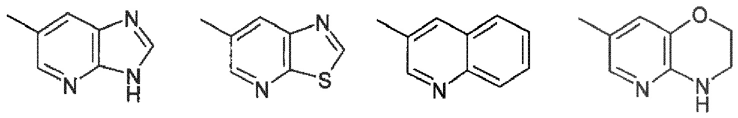 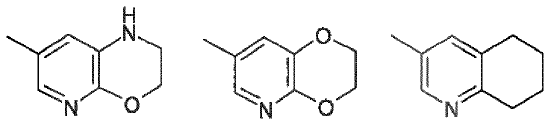 ausgewählt ist, wobei diese Gruppen gegebenenfalls durch R₂ und R₃ wie in der allgemeinen Formel (I) oben definiert substituiert sind;
R₂ ein Wasserstoffatom bedeutet;
R₃, wenn es von einem Kohlenstoffatom getragen wird, ein Wasserstoffatom, eine Hydroxyl-, C₁-C₆-Alkyl- oder Oxo-Gruppe bedeutet; wobei die C₁-C₆-Alkylgruppe durch eine Hydroxyl- oder -OC(O)-C₁-C₆-Alkyl-Gruppe substituiert sein kann;
oder
R₃, wenn es von einem Stickstoffatom getragen wird, ein Wasserstoffatom oder eine C₁-C₆-Alkylgruppe bedeutet;
als Base oder Säureadditionssalz sowie als Hydrat oder Solvat.

10. Verbindung der Formel (I) nach Anspruch 1, ausgewählt aus:
*N*-(2,3-Dimethyl-3*H*-imidazo[4,5-*b*]pyridin-6-yl)-5-fluor-1-[(3-fluorphenyl)methyl]-1*H*-indol-2-carboxamid;
*N*-(2-Methylthiazolo[5,4-*b*]pyridin-6-yl)-5-fluor-1-[(3-fluorphenyl)methyl]-1*H*-indol-2-carboxamid;
*N*-(Thiazolo[5,4-*b*]pyridin-6-yl)-5-fluor-1-[(3-fluorphenyl)methyl]-1*H*-indol-2-carboxamid;
*N*-(2-Hydroxymethylthiazolo[5,4-*b*]pyridin-6-yl)-5-fluor-1-[(3-fluorphenyl)methyl]-1*H*-indol-2-carboxamid;
*N*-(Chinolin-3-yl)-5-fluor-1-[(3-fluorphenyl)-methyl]-1*H*-indol-2-carboxamid;
*N*-(2-Hydroxymethyl-3-methyl-3*H*-imidazo[4,5-*b*]pyridin-6-yl)-5-fluor-1-[(3-fluorphenyl)methyl]-1*H*-indol-2-carboxamid;
*N*-(3-Methyl-3*H*-imidazo[4,5-*b*]pyridin-6-yl)-5-fluor-1-[(3-fluorphenyl)methyl]-1*H*-indol-2-carboxamid;
*N*-(2,3-Dimethyl-3*H*-imidazo[4,5-*b*]pyridin-6-yl)-5-trifluormethyl-1-[(3-fluorphenyl)methyl]-1*H-*pyrrolo[2,3-*b*]pyridin-2-carboxamid;
*N*-(3,4-Dihydro-2H-pyrido[3,2-*b*][1,4]oxazin-7-yl)-5-fluor-1-[(3-fluorphenyl)methyl]-1*H*-indol-2-carboxamid;
*N*-(4-Methyl-3,4-dihydro-2H-pyrido[3,2-*b*][1,4]-oxazin-7-yl)-5-fluor-1-[(3-fluorphenyl)methyl]-1*H-*indol-2-carboxamid;
*N*-(2-Methyl-3*H*-imidazo[4,5-*b*]pyridin-6-yl)-5-fluor-1-[(3-fluorphenyl)methyl]-1*H*-indol-2-carboxamid;
*N*-(1-Methyl-2-oxo-2,3-dihydro-1H-pyrido[2,3-*b*]-[1,4]oxazin-7-yl)-5-fluor-1-[(3-fluorphenyl)-methyl]-1*H*-indol-2-carboxamid;
[6-[[[5-Fluor-1-[(3-fluorphenyl)methyl]-1*H*-indol-2-yl]carbonyl]amino]thiazolo[5,4-*b*]pyridin-2-yl] -methyl-2,2-dimethylpropanoat;
*N*-(2,3-Dihydro-[1,4]dioxino[2,3-*b*]pyridin-7-yl))-5-fluor-1-[(3-fluorphenyl)methyl]-1*H*-indol-2-carboxamid;
*N*-(6-Hydroxy-5,5,7,8-tetrahydrochinolin-3-yl)-5-fluor-1-[(3-fluorphenyl)methyl]-1*H*-indol-2-carboxamid;
*N*-(2-Hydroxymethylthiazolo[5,4-*b*]pyridin-6-yl)-5-trifluormethyl-1-[(3-methylphenyl)methyl]-1*H-*indol-2-carboxamid;
*N*-(2-Hydroxymethylthiazolo[5,4-*b*]pyridin-6-yl)-6-trifluormethyl-1-[(3-methylphenyl)methyl]-1*H-*indol-2-carboxamid;
*N*-(2-Hydroxymethylthiazolo[5,4-*b*]pyridin-6-yl)-5-trimethylsilyl-1-[(3-methylphenyl)methyl]-1*H-*indol-2-carboxamid;
*N*-(2-Hydroxymethylthiazolo[5,4-*b*]pyridin-6-yl)-6-fluor-1-[(3-methylphenyl)methyl]-1*H*-indol-2-carboxamid;
*N*-(2-Hydroxymethylthiazolo[5,4-*b*]pyridin-6-yl) -5-trifluormethyl-1-[(thiazol-2-yl)methyl]-1*H*-indol-2-carboxamid;
*N*-(2-Hydroxymethylthiazolo[5,4-*b*]pyridin-6-yl)-6-trifluormethyl-1-[(thiazol-2-yl)methyl]-1*H*-indol-2-carboxamid;
*N*-(2-Hydroxymethylthiazolo[5,4-*b*]pyridin-6-yl)-5-trimethylsilyl-1-[(thiazol-2-yl)methyl]-1*H*-indol-2-carboxamid;
*N*-(2-Hydroxymethylthiazolo[5,4-*b*]pyridin-6-yl)-6-fluor-1-[(thiazol-2-yl)methyl]-1*H*-indol-2-carboxamid;
*N*-(2-Hydroxymethylthiazolo[5,4-*b*]pyridin-6-yl)-5-trifluormethyl-1-[(thiazol-2-yl)methyl]-1*H-*pyrrolo[2,3-b]pyridin-2-carboxamid;
*N*-(2-Hydroxymethylthiazolo[5,4-*b*]pyridin-6-yl)-6-trimethylsilyl-1-[(thiazol-2-yl)methyl]-1*H*-indol-2-carboxamid;
*N*-(2-Hydroxymethylthiazolo[5,4-*b*]pyridin-6-yl)-5-trifluormethyl-1-[(pyridin-4-yl)methyl]-1*H*-indol-2-carboxamid;
N-(2-Hydroxymethylthiazolo[5,4-*b*]pyridin-6-yl)-6-trifluormethyl-1-[(pyridin-4-yl)methyl]-1*H*-indol-2-carboxamid;
*N*-(2-Hydroxymethylthiazolo[5,4-*b*]pyridin-6-yl)-5-trimethylsilyl-1-[(pyridin-4-yl)methyl]-1*H*-indol-2-carboxamid;
*N*-(2-Hydroxymethylthiazolo[5,4-*b*]pyridin-6-yl)-6-fluor-1-[(pyridin-4-yl)methyl]-1*H*-indol-2-carboxamid;
*N*-(2-Hydroxymethylthiazolo[5,4-*b*]pyridin-6-yl)-5-trifluormethyl-1-[(pyridin-4-yl)methyl]-1*H-*pyrrolo[2,3-*b*]pyridin-2-carboxamid;
*N*- (2-Hydroxymethylthiazolo[5,4-*b*]pyridin-6-yl)-6-trimethylsilyl-1-[(pyridin-4-yl)methyl]-1*H*-indol-2-carboxamid;
*N*-(2-Hydroxymethylthiazolo[5,4-*b*]pyridin-6-yl)-5-trimethylsilyl-1-[[(3-trifluormethyl)phenyl]-methyl]-1*H*-indol-2-carboxamid;
*N*-(2-Hydroxymethylthiazolo[5,4-*b*]pyridin-6-yl)-6-fluor-1-[[(3-trifluormethyl)phenyl]methyl]-1*H-*indol-2-carboxamid;
*N*-(2-Hydroxymethylthiazolo[5,4-*b*]pyridin-6-yl)-5-trifluormethyl-1-[[(3-trifluormethyl)phenyl]-methyl]-1*H*-pyrrolo[2,3-*b*]pyridin-2-carboxamid;
*N*-(2-Hydroxymethylthiazolo[5,4-*b*]pyridin-6-yl)-6-trimethylsilyl-1-[[(3-trifluormethyl)phenyl]-methyl]-1*H*-indol-2-carboxamid;
*N*-(2-Hydroxymethylthiazolo[5,4-*b*]pyridin-6-yl)-5-trifluormethyl-1-[(3-methylphenyl)methyl]-1*H-*pyrrolo[2,3-*b*]pyridin-2-carboxamid;
*N*-(2-Hydroxymethylthiazolo[5,4-*b*]pyridin-6-yl)-5-trimethylsilyl-1-[(3-methylphenyl)methyl]-1*H-*indol-2-carboxamid;
*N*-(2-Hydroxymethylthiazolo[5,4-*b*]pyridin-6-yl)-5-trifluormethyl-1-[[(3-trifluormethyl)phenyl]-methyl]-1*H*-indol-2-carboxamid;
*N*-(2-Hydroxymethylthiazolo[5,4-*b*]pyridin-6-yl)-6-trifluormethyl-1-[[(3-trifluormethyl)phenyl]-methyl]-1*H*-indol-2-carboxamid.

11. Verfahren zur Herstellung einer Verbindung der Formel (I) nach einem der Ansprüche 1 bis 10, **dadurch gekennzeichnet, dass** man eine Verbindung der allgemeinen Formel (II), 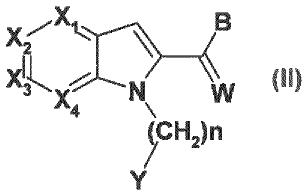 in der X₁, X₂, X₃, X₄, n, Y und W wie in der allgemeinen Formel (I) nach Anspruch 1 definiert sind,
mit einer Verbindung der allgemeinen Formel (III), 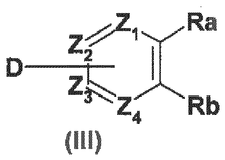 in der Z₁, Z₂, Z₃, Z₄, Ra und Rb wie in der allgemeinen Formel (I) nach Anspruch 1 definiert sind,
umsetzt, und zwar
- wenn B eine Hydroxylgruppe bedeutet und D eine Aminogruppe bedeutet, in einem Lösungsmittel in Gegenwart eines Kopplungsmittels;
- wenn B ein Chloratom bedeutet und D eine Aminogruppe bedeutet, durch Umsetzen in Lösung in einem Lösungsmittel;
- wenn B eine C₁-C₆-Alkoxy-, C₃-C₇-Cycloalkyloxy-, C₃-C₇-Cycloalkyl-C₁-C₃-alkylenoxy- oder Aryl-C₁-C₃-alkylenoxy-Gruppe bedeutet und D eine Aminogruppe bedeutet, durch Umwandeln der Verbindung der Formel (III) in das Amid durch zuvoriges Einwirkenlassen von Trimethylaluminium auf Amine der allgemeinen Formel (III) und anschließendes Umsetzen des erhaltenen Amids mit der Verbindung der Formel (II) unter Lösungsmittelrückfluss;
- wenn B eine NH₂-Gruppe bedeutet, W ein Sauerstoffatom bedeutet und D eine Abgangsgruppe bedeutet, in einem Lösungsmittel in Gegenwart eines Kupfersalzes in einer katalytischen Menge, einer katalytischen Menge eines Kupferliganden und einer Base.

12. Verfahren zur Herstellung einer Verbindung der allgemeinen Formel (II), 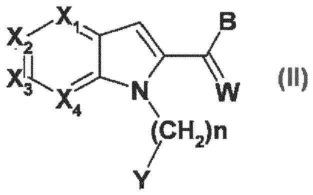 in der X₁, X₂, X₃, X₄, n, Y und W wie in der allgemeinen Formel (I) nach Anspruch 1 definiert sind, eines von X₁, X₂, X₃ und X₄ einer C-R₁-Gruppe entspricht oder R₁ eine -Si-(C₁-C₆-alkyl)₃-Gruppe wie in der allgemeinen Formel (I) nach Anspruch 1 definiert bedeutet und B eine C₁-C₆-Alkoxylgruppe bedeutet,
**dadurch gekennzeichnet, dass** man eine Verbindung der allgemeinen Formel (VI), 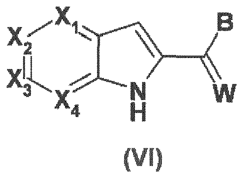 in der X₁, X₂ , X₃ , X₄ und W wie in der allgemeinen Formel (I) nach Anspruch 1 definiert sind und B eine C₁-C₆-Alkoxylgruppe bedeutet,
mit einer Verbindung der allgemeinen Formel (VIII), 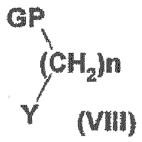 in der Y und n wie in der allgemeinen Formel (I) nach Anspruch 1 definiert sind,
umsetzt, und zwar
- wenn n gleich 1, 2 oder 3 ist, mit einem Reaktanten der allgemeinen Formel (VIII), worin GP eine Abgangsgruppe bedeutet, in einem polaren Lösungsmittel in Gegenwart einer Base;
- wenn n gleich 1, 2 oder 3 bedeutet, mit einem Reaktanten der allgemeinen Formel (VIII), worin GB eine Hydroxylgruppe bedeutet, in Gegenwart eines Phosphins und von Diethylazodicarboxylat in Lösung in einem Lösungsmittel; oder alternativ in Gegenwart eines auf einem Harz geträgerten Phosphins und von Diisopropylazodicarboxylat in Lösung in einem Lösungsmittel;
- wenn n gleich 0 ist, mit einem Reaktanten der allgemeinen Formel (VIII), in dem GP eine Abgangsgruppe bedeutet, in einem basischen Medium unter Inertatmosphäre in Gegenwart eines Kupfersalzes in einem organischen Lösungsmittel.

13. Verbindung der allgemeinen Formel (IIa), (IIb), (IIc), (IId), (IIe), (IIf), (IIg) oder (IIh) :

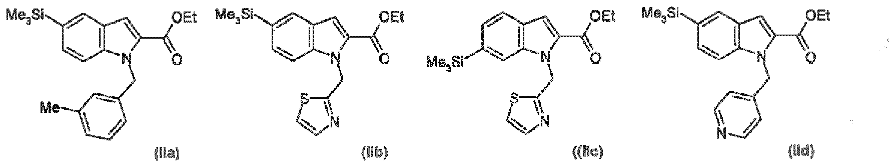

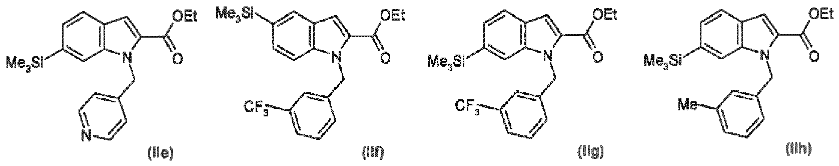

14. Arzneimittel, **dadurch gekennzeichnet, dass** es eine Verbindung der Formel (I) nach einem der Ansprüche 1 bis 10 oder ein Additionssalz dieser Verbindung mit einer pharmazeutisch unbedenklichen Säure oder ein Hydrat oder Solvat der Verbindung der Formel (I) umfasst.

15. Pharmazeutische Zusammensetzung, **dadurch gekennzeichnet, dass** sie eine Verbindung der Formel (I) nach einem der Ansprüche 1 bis 10 oder ein pharmazeutisch unbedenkliches Salz, ein Hydrat oder Solvat dieser Verbindung sowie mindestens einen pharmazeutisch unbedenklichen Grundstoff umfasst.

16. Verwendung einer Verbindung der Formel (I) nach einem der Ansprüche 1 bis 10 zur Herstellung eines Arzneimittels für die Vorbeugung und für die Behandlung von Pathologien, an denen die Typ-TRPV1-Rezeptoren beteiligt sind.

17. Verwendung einer Verbindung der Formel (I) nach einem der Ansprüche 1 bis 10 zur Herstellung eines Arzneimittels für die Vorbeugung oder Behandlung von Schmerzen, Entzündungen, Stoffwechselstörungen, Störungen der Harnwege, gynäkologischen Störungen, Magen-Darm-Störungen, Störungen der Atemwege, Schuppenflechte, Juckreiz, Haut-, Augen- oder Schleimhautreizungen, Herpes, Gürtelrose, multipler Sklerose, Depression und Karzinomen.

## Claims

1. Compound corresponding to formula (I) 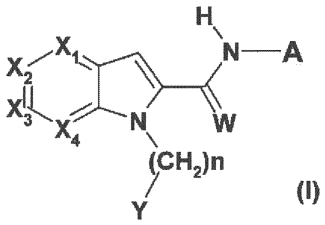 in which:
X₁, X₂, X₃ and X₄ represent, independently of each other, a nitrogen atom or a group C-R₁;
it being understood that when one from among X₁, X₂, X₃ and X₄ represents a nitrogen atom, the others correspond to a group C-R₁;
W represents an oxygen or sulfur atom;
n is equal to 0, 1, 2 or 3;
Y represents an aryl or a heteroaryl optionally substituted with one or more groups chosen from a halogen atom, a group C₁-C₆-alkyl, C₃-C₇-cycloalkyl, C₃-C₇-cydoalkyl-C₁-C₃-alkylene, C₁-C₆-fluoroalkyl, hydroxyl, C₁-C₆-alkoxy, C₃-C₇-cycloalkyloxy, C₃-C₇-cycloalkyl-C₁-C₆-alkylene-O-, C₁-C₆-fluoroalkoxy, cyano, C(O)NR₄R₅, nitro, NR₄R₅, C₁-C₆-thioalkyl, thiol, -S(O)-C₁C₆-alkyl, -S(O)₂-C₁-C₆-alkyl, SO₂NR₄R₅, NR₆C(O)R₇, NR₆SO₂R₈, C(O)NR₄R₅, OC(O)NR₄R₅, -Si-(C₁-C₆-alkyl)₃, -SF₅, aryl-C₁-C₅-alkylene or aryl, heteroaryl-C₁-C₅-alkylene or heteroaryl; the groups C₁-C₆-alkyl, C₃-C₇-cycloalkyl, C₃-C₇-cycloalkyl-C₁-C₃-alkylene, C₁-C₆-fluoroalkyl, C₁-C₆-alkoxy, C₃-C₇-cycloalkyloxy, C₃-C₇-cycloalkyl-C₁-C₆-alkylene-O- being optionally substituted with a hydroxyl group, C₁-C₆-alkoxy or NR₄R₅, the aryl and heteroaryl groups being optionally substituted with one or more substituents R₉, which may be identical to or different from each other;
A represents the group of formula: 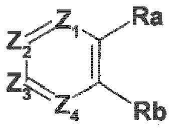
Z₁, Z₂, Z₃ and Z₄ represent, independently of each other, a nitrogen atom, a carbon atom or a group C-R₂,
at least one from among Z₁, Z₂, Z₃ and Z₄ corresponding to a nitrogen atom and
one from among Z₁, Z₂, Z₃ and Z₄, corresponding to a carbon atom, being bonded to the nitrogen atom of the amide or of the thioamide of formula (I);
Ra and Rb form, together with the carbon atoms that bear them,
either a partially unsaturated cycloalkyl, or an aryl;
or a heterocycle, or a heteroaryl, which is 5- to 7-membered, comprising from 1 to 3 heteroatoms chosen among O, S or N;
it being understood that when Ra and Rb together form, with the carbon atoms that bear them, a 5-membered ring, this ring comprising a nitrogen atom and carbon atoms, this ring being partially saturated or unsaturated, is excluded;
this partially unsaturated cycloalkyl, this aryl, this heterocycle or this heteroaryl possibly being substituted with one or more substituents R₃;
R₁ is chosen from a hydrogen atom, a halogen atom, a group C₁-C₆-alkyl, C₃-C₇-cycloalkyl, C₃-C₇-cycloalkyl-C₁-C₃-alkylene, C₁-C₆-fluoroalkyl, aryloxy-C₁-C₆-alkyl, heteroaryloxy-C₁-C₆-alkyl, aryl-C₁-C₃-alkylenoxy-C₁-C₆-alkyl, heteroaryl-C₁-C₃-alkylenoxy-C₁-C₆-alkyl, arylthio-C₁-C₆-alkyl, heteroarylthio-C₁-C₆-alkyl, aryl-C₁-C₃-alkylene-thio-C₁-C₆-alkyl, heteroaryl-C₁-C₃-alkylene-thio-C₁-C₆-alkyl, C₁-C₆-alkoxy, C₃-C₇-cycloalkyloxy, C₃-C₇-cycloalkyl-C₁-C₃-alkylenoxy, C₁-C₆-fluoroalkoxy, cyano, C(O)NR₄R₅, nitro, NR₄R₅, C₁-C₆-thioalkyl, C₃-C₇-cydoalkylthio, C₃-C₇-cycloalkyl-C₁-C₃-alkylene-thio, -S(O)-C₁-C₆-alkyl, -S(O)-C₃-C₇-cycloalkyl, -S(O)-C₁-C₃-alkylene-C₃-C₇-cycloalkyl, C₁-C₆-alkyl-S(O)₂-, C₁-C₆-fluoroalkyl-S(O)₂-, C₃-C₇-cycloalkyl-S(O)₂-, C₃-C₇-cycloalkyl-C₁-C₃-alkylene-S(O)₂-, SO₂NR₄R₅, -Si-(C₁-C₆-alkyl)₃, -SF₅, NR₆C(O)R₇, NR₈SO₂R₈, C(O)NR₄R₅, OC(O)NR₄R₅, aryl, heteroaryl, aryl-C₁-C₅-alkylene, heteroaryl-C₁-C₅-alkylene, aryloxy, arylthio, heteroaryloxy or heteroarylthio; the heteroaryl or aryl groups being optionally substituted with one or more substituents R₉, which may be identical to or different from each other;
R₂ represents a hydrogen atom, a halogen atom or a group C₁-C₆-alkyl, C₃-C₇-cycloalkyl, C₃-C₇-cycloalkyl-C₁-C₃-alkylene, C₁-C₆-fluoroalkyl, C₁-C₆-alkoxy, C₃-C₇-cycloalkyloxy, C₃-C₇-cycloalkyl-C₁-C₃-alkylenoxy, hydroxyl, thiol or C₁-C₆-fluoroalkoxy;
R₃ represents, when it is borne by a carbon atom, a hydrogen atom or a hydroxyl, thiol, C₁-C₆-alkyl, C₃-C₇-cycloalkyl, C₃-C₇-cycloalkyl-C₁-C₃-alkylene, C₁-C₈-fluoroalkyl, C₁-C₆-alkoxy, C₃-C₇-cycloalkyloxy, C₃-C₇-cycloalkyl-C₁-C₃-alkylenoxy, C₁C₆-alkoxy-C₁-C₃-alkylene, C₃-C₇-cycloalkyloxy-C₁-C₃-alkylene, C₃-C₇-cycloalkyl-C₁-C₃-alkylenoxy-C₁-C₃-alkylene, C(O)NR₄R₅, C(O)O-C₁-C₆-alkyl, CO₂H, oxo or thio group; the groups C₁-C₆-alkyl, C₃-C₇-cycloalky), C₃-C₇-cycloalkyl-C₁-C₃-alkylene, C₁-C₆-fluoroalkyl, C₁-C₆-alkoxy, C₃-C₇-cycloalkyloxy, C₃-C₇-cycloalkyl-C₁-C₃-alkylenoxy, C₁-C₆-alkoxy-C₁-C₃-alkylene, C₃-C₇-cycloalkyloxy-C₁-C₃-alkylene, C₃-C₇-cycloalkyl-C₁-C₃-alkylenoxy-C₁-C₃-alkylene possibly being substituted with a hydroxyl group, C₁-C₆-alkoxy, -OC(O)-C₁-C₆-alkyl or NR₄R₅;
or
R₃ represents, when it is borne by a nitrogen atom, a hydrogen atom or a group C₁-C₆-alkyl, C₃-C₇-cycloalkyl, C₃-C₇-cycloalkyl-C₁-C₃-alkylene, C₁-C₆-fluoroalkyl, aryl-C(O)-, C₁-C₆-alkyl-C(O)-, C₃-C₇-cycloalkyl-C(O)-, C₃-C₇-cycloalkyl-C₁-C₃-alkylene-C(O)-, C₁-C₆-fluoroalkyl-C(O)-, aryl-S(O), C₁-C₆-alkyl-S(O)-, C₁-C₆-fluoroalkyl-S(O)-, aryl-S(O)₂-, C₁-C₈-alkyl-S(O)₂-, C₁-C₆-fluoroalkyl-S(O)₂-, C₃-C₇-cycloalkyl-S(O)₂-, C₃-C₇-cycloalkyl-C₁-C₃-alkylene-S(O)₂-, C₁-C₆-alkyl-O-C(O)-, aryl-C₁-C₃-alkyl-O-C(O)-, C₃-C₇-cydoalkyl-O-C(O)-, C₃-C₇-cycloalkyl-C₁-C₃-alkylene-O-C(O)-, C₁-C₆-fluoroalkyl-O-C(O), aryl-O-C(O)-, heteroaryl-O-C(O)-, heteroaryl or aryl; the heteroaryl and aryl groups being optionally substituted with one or more substituents R₉, which may be identical to or different from each other; the groups C₁-C₆-alkyl, C₃-C₇-cycloalkyl, C₃-C₇-cycloalkyl-C₁-C₃-alkylene and C₁-C₆-fluoroalkyl possibly being substituted with a hydroxyl group, C₁-C₆-alkoxy or NR₄R₅;
R₄ and R₅, represent, independently of each other, a hydrogen atom or a group C₁-C₆-alkyl, C₃-C₇-cycloalkyl, C₃-C₇-cycloalkyl-C₁-C₃-alkylene, aryl-C₁-C₅-alkylene or aryl, or R₄ and R₅ together form, with the nitrogen atom that bears them, an azetidine, pyrrolidine, piperidine, azepine, morpholine, thiomorpholine, piperazine or homopiperazine group; the group NR₄R₅ being optionally substituted with a group C₁-C₆-alkyl, C₃-C₇-cycloalkyl, C₃-C₇-cycloalkyl-C₁-C₃-alkylene, aryl-C₁-C₆-alkylene, aryl, heteroaryl, aryl-S(O)₂-, C₁-C₆-alkyl-S(O)₂-, C₁-C₆-fluoroalkyl-S(O)₂, C₃-C₇-cycloalkyl-S(O)₂-, C₃-C₇-cycloalkyl-C₁-C₃-alkylene-S(O)₂-, aryl-C(O)-, C₁-C₆-alkyl-C(O)-, C₃-6₇-cycloalkyl-C(O)-, C₃-C₇-cycloalkyl-C₁-C₃-alkylene-C(O)-, C₁-C₆-fluoroalkyl-C(O)-, hydroxyl, C₁-C₆-alkyloxy, C₃-C₇-cycloalkyloxy, C₃-C₇-cycloalkyl-C₁-C₃-alkylenoxy, C₁-C₆-fluoroalkyl, aryloxy-C₁-C₆-alkylene, aryloxy, heteroaryloxy-C₁-C₆-alkylene or heteroaryloxy;
R₆ and R₇ represent, independently of each other, a hydrogen atom or a group C₁-C₆-alkyl, C₃-C₇-cycloalkyl, C₃-C₇-cycloalkyl-C₁-C₃-alkylene, aryl-C₁-C₆-alkylene or aryl; the aryl group being optionally substituted with one or more substituents chosen from a halogen atom and a group C₁-C₆-alkyl, C₃-C₇-cycloalkyl, C₃-C₇-cycloalkyl-C₁-C₃-alkylene, C₁-C₆-fluoroalkyl, C₁-C₆-alkoxy, C₃-C₇-cycloalkyloxy, C₃-C₇-cycloalkyl-C₁-C₃-alkylenoxy, C₁-C₆-fluoroalkoxy, nitro or cyano;
or R₆ and R₇ together form a 4- to 7-membered lactam comprising the nitrogen atom and the C(O) group that bear them;
R₈ represents a group C₁-C₆-alkyl, C₃-C₇-cycloalkyl, C₃-C₇-cycloalkyl-C₁-C₃-alkylene, aryl-C₁-C₆-alkylene or aryl; the aryl group being optionally substituted with one or more substituents chosen from a halogen atom, a group C₁-C₆-alkyl, C₃-C₇-cycloalkyl, C₃-C₇-cycloalkyl-C₁-C₃-alkylene, C₁-C₆-fluoroalkyl, C₁-C₆-alkoxy, C₃-C₇-cycloalkyloxy, C₃-C₇-cycloalkyl-C₁-C₃-alkylenoxy, C₁-C₆-fluoroalkoxy, nitro or cyano;
or R₆ and R₈ together form a 4- to 7-membered sultam comprising the nitrogen atom and the S(O)₂ group that bear them;
R₉ represents a halogen atom, a group C₁-C₆-alkyl, C₃-C₇-cycloalkyl, C₃-C₇-cycloalkyl-C₁-C₃-alkylene, C₁-C₆-fluoroalkyl, C₁-C₆-alkoxy, C₃-C₇-cycloalkyloxy, C₃-C₇-cycloalkyl-C₁-C₃-alkylenoxy, C₁-C₆-fluoroalkoxy; these groups being optionally substituted with a group OH, C₁-C₆-alkoxy or NR₄R₅; or alternatively R₉ represents a nitro group, cyano or NR₄R₅;
the sulfur atom(s) of the compound of general formula (I) possibly being in oxidized form;
the nitrogen atom(s) of the compound of general formula (I) possibly being in oxidized form;
in the form of the base or of an acid-addition salt, and also in the form of hydrate or solvate.

2. Compound of formula (I) according to Claim 1, **characterized in that** X₁, X₂, X₃ and X₄ represent, independently of each other, a group C-R₁; R₁ being as defined in the general formula (I) according to Claim 1;
in the form of the base or of an acid-addition salt, and also in the form of hydrate or solvate.

3. Compound of formula (I) according to Claim 1, **characterized in that** X₁, X₂, X₃ represent a group C-R₁; X₄ represents a nitrogen atom;
R₁ being as defined in the general formula (I) according to Claim 1;
in the form of the base or of an acid-addition salt, and also in the form of hydrate or solvate.

4. Compound of formula (I) according to any one of Claims 1 to 3, **characterized in that** R₁ is chosen from a hydrogen atom, a halogen atom and a group C₁-C₆-fluoroalkyl or -Si(C₁-C₆-alkyl)₃;
in the form of the base or of an acid-addition salt, and also in the form of hydrate or solvate.

5. Compound of formula (I) according to any one of Claims 1 to 4, **characterized in that** n is equal to 1;
in the form of the base or of an acid-addition salt, and also in the form of hydrate or solvate.

6. Compound of formula (I) according to any one of Claims 1 to 5, **characterized in that** Y represents an aryl or a heteroaryl optionally substituted with one or more groups chosen from a halogen atom and a group C₁-C₆-alkyl or C₁-C₆-fluoroalkyl;
in the form of the base or of an acid-addition salt, and also in the form of hydrate or solvate.

7. Compound of formula (I) according to any one of Claims 1 to 6, **characterized in that** W represents an oxygen atom;
in the form of the base or of an acid-addition salt, and also in the form of hydrate or solvate.

8. Compound of formula (I) according to any one of Claims 1 to 7, **characterized in that** A represents the group of formula: 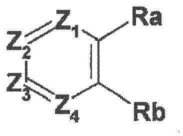 in which A is chosen from the groups 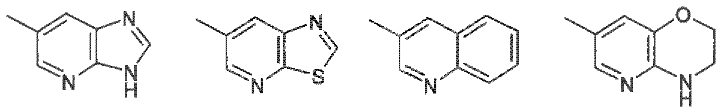 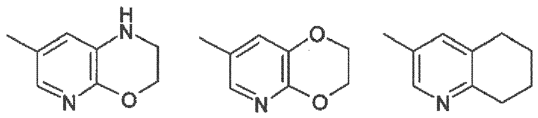 these groups being optionally substituted with R₂ and R₃ as defined in the general formula (I) hereinabove;
R₂ represents a hydrogen atom;
R₃ represents, when it is borne by a carbon atom, a hydrogen atom or a hydroxyl, C₁-C₆-alkyl or oxo group; the C₁-C₆-alkyl group possibly being substituted with a hydroxyl or -OC(O)-C₁C₆-alkyl group;
or
R₃ represents, when it is borne by a nitrogen atom, a hydrogen atom or a group C₁-C₆-alkyl;
in the form of the base or of an acid-addition salt, and also in the form of hydrate or solvate.

9. Compound of formula (I) according to any one of Claims 1 to 8, **characterized in that** X₁, X₂, X₃ and X₄ represent, independently of each other, a group C-R₁; or alternatively X₁, X₂ and X₃ represent a group C-R₁; X₄ represents a nitrogen atom;
R₁ is chosen from a hydrogen atom, a halogen atom and a group C₁-C₆-fluoroalkyl or -Si-(C₁-C₆-alkyl)₃;
n is equal to 1;
Y represents an aryl or a heteroaryl optionally substituted with one or more groups chosen from a halogen atom and a group C₁-C₆-alkyl or C₁-C₆-fluoroalkyl;
W represents an oxygen atom;
A represents the group of formula: 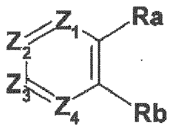 in which A is chosen from the groups 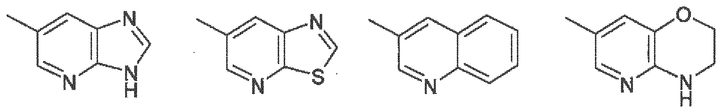 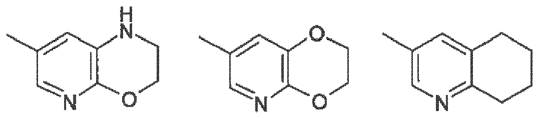 these groups being optionally substituted with R₂ and R₃ as defined in the general formula (I) hereinabove;
R₂ represents a hydrogen atom;
R₃ represents, when it is borne by a carbon atom, a hydrogen atom or a hydroxyl, C₁-C₆-alkyl or oxo group; the C₁-C₆-alkyl group possibly being substituted with a hydroxyl or -OC(O)-C₁-C₆-alkyl group;
or
R₃ represents, when it is borne by a nitrogen atom, a hydrogen atom or a group C₁-C₆-alkyl;
in the form of the base or of an acid-addition salt, and also in the form of hydrate or solvate.

10. Compound of formula (I) according to Claim 1, chosen from:
*N*-(2,3-Dimethyl-3*H*-imidazo[4,5-*b*]pyrid-6-yl)-5-fluoro-1-[(3-fluorophenyl)methyl]-1*H-*indole-2-carboxamide;
*N*-(2-Methylhiazolo[5,4-*b*]pirid-6-yl)-5-fluoro-1-[(3-fluorophenyl)methyl]-1*H*-indole-2-carboxamide;
*N*-(Thiazolo[5,4-*b*]pyrid-6-yl)-5-fluoro-1-[(3-fluorophenyl)methyl]-1*H*-indole-2-carboxamide;
*N*-(2-Hydroxymethylthiazolo[5,4-*b*]pyrid-6-yl)-5-fluoro-1-[(3-fluorophenyl)methyl]-1*H-*indole-2-carboxamide;
*N*-(Quinol-3-yl)-5-fluoro-1-[(3-fluorophenyl)methyl]-1*H*-indole-2-carboxamide;
*N*-(2-Hydroxymethyl-3-methyl-3*H*-imidazo[4,5-*b*]pyrid-6-yl)-5-fluoro-1-[(3-fluorophenyl)-methyl]-1*H*-indole-2-carboxamide;
*N*-(3-Methyl-3*H*-imidazo[4,5-*b*]pyrid-6-yl)-5-fluoro-1-[(3-fluorophenyl)methyl]-1*H*-indole-2-carboxamide;
*N*-(2,3-Dimethyl-3*H*-imidazo[4,5-*b*]pyrid-6-yl)-5-trifluoromethyl-1-[(3-fluorophenyl)-methyl]-1*H*-pyrrolo[2,3-*b*]pyridine-2-carboxamide;
*N*-(3,4-Dihydro-2H-pyrido[3,2-*b*][1,4]oxazin-7-yl)-5-fluoro-1-[(3-fluorophenyl)methyl]-1*H-*indole-2-carboxamide;
*N*-(4-Methyl-3,4-dihydro-2H-pyrido[3,2-*b*][1,4]oxazin-7-yl)-5-fluoro-1-[(3-fluorophenyl)-methyl]-1*H*-indole-2-carboxamide;
*N*-(2-Methyl-3*H*-imidazo[4,5-*b*]pyrid-6-yl)-5-fluoro-1-[(3-fluorophenyl)methyl]-1*H*-indole-2-carboxamide;
*N*-(1-Methyl-2-oxo-2,3-dihydro-1H-pyrido[2,3-*b*][1,4]oxazin-7-yl)-5-fluoro-1-[(3-fluorophenyl)methyl]-1*H*-indole-2-carboxamide;
[6-[[[5-Fluoro-1-[(3-fluorophenyl)methyl]-1*H*-indol-2-yl]carbonyl]amino]thiazolo[5,4-*b*]pyrid-2-yl]methyl 2,2-dimethylpropanoate;
*N*-(2,3-Dihydro-[1,4]dioxino[2,3-*b*]pyrid-7-yl))-5-fluoro-1-[(3-fluorophenyl)methyl]-1*H-*indole-2-carboxamide;
*N*-(6-Hydroxy-5,6,7,8-tetrahydroquinol-3-yl)-5-fluoro-1-[(3-fluorophenyl)methyl]-1*H-*indole-2-carboxamide;
*N*-(2-Hydroxymethylthiazolo[5,4-*b*]pyrid-6-yl)-5-trifluoromethyl-1-[(3-methylphenyl)-methyl]-1*H*-indole-2-carboxamide;
*N*-(2-Hydroxymethylthiazolo[5,4-*b*]pyrid-6-yl)-6-trifluoromethyl-1-[(3-methylphenyl)-methyl]-1*H*-indole-2-carboxamide;
*N*-(2-Hydroxymethylthiazolo[5,4-*b*]pyrid-6-yl)-5-trimethylsilyl-1-[(3-methylphenyl)-methyl]-1*H*-indole-2-carboxamide;
*N*-(2-Hydroxymethylthiazolo[5,4-*b*]pyrid-6-yl)-6-fluoro-1-[(3-methylphenyl)methyl]-1*H-*indole-2-carboxamide;
*N*-(2-Hydroxymethylthiazolo[5,4-*b*]pyrid-6-yl)-5-trifluoromethyl-1-[(thiazol-2-yl)methyl]-1*H*-indole-2-carboxamide;
*N*-(2-Hydroxymethylthiazolo[5,4-*b*]pyrid-6-yl)-6-trifluoromethyl-1-[(thiazol-2-yl)methyl]-1*H*-indole-2-carboxamide;
*N*-(2-Hydroxymethylthiazolo[5,4-*b*]pyrid-6-yl)-5-trimethylsilyl-1-[(thiazol-2-yl)methyl]-1*H-*indole-2-carboxamide;
*N*-(2-Hydroxymethylthiazolo[5,4-*b*]pyrid-6-yl)-6-fluoro-1-[(thiazol-2-yl)methyl]-1*H*-indole-2-carboxamide;
*N*-(2-Hydroxymethylthiazolo[5,4-*b*]pyrid-6-yl)-5-trifluoromethyl-1-[(thiazol-2-yl)methyl]-1*H*-pyrrolo[2,3-*b*]pyridine-2-carboxamide;
*N*-(2-Hydroxymethylthiazolo[5,4-*b*]pydd-6-yl)-6-trimethylsilyl-1-[(thiazol-2-yl)methyl]-1*H-*indole-2-carboxamide;
*N*-(2-Hydroxymethylthiazolo[5,4-*b*]pyrid-6-yl)-5-trifluoromethyl-1-[(pyrid-4-yl)methyl]-1*H-*indole-2-carboxamide;
*N*-(2-Hydroxymethylthiazolo[5,4-*b*]pyrid-6-yl)-6-trifluoromethyl-1-[(pyrid-4-yl)methyl]-1*H-*indole-2-carboxamide;
*N*-(2-Hydroxymethylthiazolo[5,4-*b*]pirid-6-yl)-5-trimethylsilyl-1-[(pyrid-4-yl)methyl]-1*H-*indole-2-carboxamide;
*N*-(2-Hydroxymethylthiazolo[5,4-*b*]pyrid-6-yl)-6-fluoro-1-[(pyrid-4-yl)methyl]-1*H*-indole-2-carboxamide;
*N*-(2-Hydroxymethylthiazolo[5,4-*b*]pyrid-6-yl)-5-trifluoromethyl-1-[(pyrid-4-yl)methyl]-1*H-*pyrrolo[2,3-b]pyridine-2-carboxamide;
*N*-(2-Hydroxymethylthiazolo[5,4-*b*]pyrid-6-yl)-6-trimethylsilyl-1-[(pyrid-4-yl)methyl]-1*H-*indole-2-carboxamide;
*N*-(2-Hydroxymethylthiazolo[5,4-*b*]pyrid-6-yl)-5-trimethylsilyl-1-[[(3-trifluoromethyl)-phenyl]methyl]-1*H*-indole-2-carboxamide;
*N*-(2-Hydroxymethylthiazolo[5,4-*b*]pyrid-6-yl)-6-fluoro-1-[[(3-trifluoromethyl)phenyl]-methyl]-1*H*-indole-2-carboxamide;
*N*-(2-Hydroxymethylthiazolo[5,4-*b*]pyrid-6-yl)-5-trifluoromethyl-1-[[(3-trifluoromethyl)-phenyl]methyl]-1*H*-pyrrolo[2,3-b]pyridine-2-carboxamide;
*N*-(2-Hydroxymethylthiazolo[5,4-*b*]pyrid-6-yl)-6-trimethylsilyl-1-[[(3-trifluoromethyl)-phenyl]methyl]-1*H*-indole-2-carboxamide;
*N*-(2-Hydroxymethylthiazolo[5,4-*b*]pyrid-6-yl)-5-trifluoromethyl-1-[(3-methylphenyl)-methyl]-1*H*-pyrrolo[2,3-b]pyridine-2-carboxamide;
*N*-(2-Hydroxymethylthiazolo[5,4-*b*]pyrid-6-yl)-6-trimethylsilyl-1-[(3-methylphenyl)-methyl]-1*H*-indole-2-carboxamide;
*N*-(2-Hydroxymethylthiazolo[5,4-*b*]pyrid-6-yl)-5-trifluoromethyl-1-[[(3-trifluoromethyl)-phenyl]methyl]-1*H*-indole-2-carboxamide;
*N*-(2-Hydroxymethylthiazolo[5,4-*b*]pyrid-6-yl)-6-trifluoromethyl-1-[[(3-trifluoromethyl)-phenyl]methyl]-1*H*-indole-2-carboxamide.

11. Process for preparing a compound of formula (I) according to any one of Claims 1 to 10, **characterized in that** a compound of general formula (II) 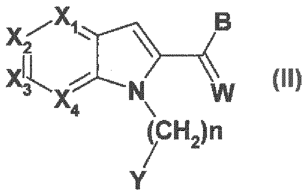 in which X₁, X₂, X₃, X₄, n, Y and W are as defined in the general formula (I) according to Claim 1,
is reacted with a compound of general formula (III), 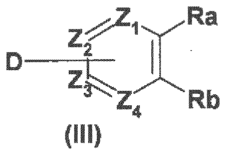 in which Z₁, Z₂, Z₃, Z₄, Ra and Rb are as defined in the general formula (I) according to Claim 1,
- when B represents a hydroxyl group and D represents an amino group, in the presence of a coupling agent in a solvent;
- when B represents a chlorine atom and D represents an amino group, by reaction in solution in a solvent;
- when B represents a group C₁-C₆-alkoxyl, C₃-C₇-cycloalkyloxy, C₃-C₇-cycloalkyl-C₁-C₃-alkylenoxy- or aryl-C₁-C₃-alkylenoxy and D represents an amino group, by transformation of the compound of formula (III) into an amide via the prior action of trimethylaluminium on the amines of general formula (III), and then by reacting the amide obtained with the compound of formula (II) in a refluxing solvent;
- when B represents an NH₂ group, W represents an oxygen atom and D corresponds to a leaving group, in the presence of a copper salt in catalytic amount, a catalytic amount of a copper ligand, and a base, in a solvent.

12. Process for preparing a compound of general formula (II) 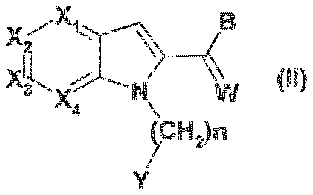 in which X₁, X₂, X₃, X₄, n, Y and W are as defined in the general formula (I) according to Claim 1, one from among X₁, X₂, X₃ and X₄ corresponding to a group C-R₁ in which R₁ represents a group -Si-(C₁-C₆-alkyl)₃ as defined in the general formula (I) according to Claim 1 and B represents a group C₁-C₆-alkoxyl,
**characterized in that** a compound of general formula (VI) 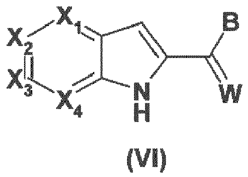 in which X₁, X₂, X₃, X₄ and W are as defined in the general formula (I) according to Claim 1 and B represents a group C₁-C₆-alkoxyl
is reacted with a compound of general formula (VIII) 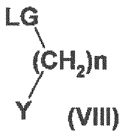 in which Y and n are as defined in the general formula (I) according to Claim 1,
- when n is equal to 1, 2 or 3, with a reagent of general formula (VIII) in which LG represents a leaving group, in the presence of a base in a polar solvent;
- when n is equal to 1, 2 or 3, with a reagent of general formula (VIII) in which LG represents a hydroxyl group, in the presence of a phosphine and diethyl azodicarboxylate in a solvent; or alternatively in the presence of a phosphine supported on a resin and diisopropyl azodicarboxylate in solution in a solvent;
- when n is equal to 0, with a reagent of general formula (VIII) in which LG represents a leaving group, under an inert atmosphere in basic medium, in the presence of a copper salt in an organic solvent.

13. Compound of general formula (IIa), (IIb), (IIc), (IId), (IIe), (IIf), (IIg) or (IIh):

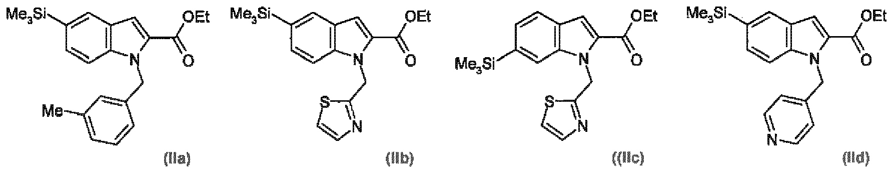

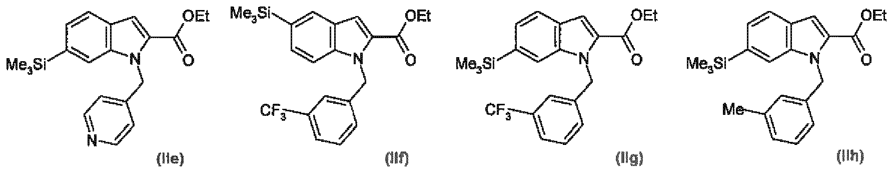

14. Medicament, **characterized in that** it comprises a compound of formula (I) according to any one of Claims 1 to 10, or an addition salt of this compound with a pharmaceutically acceptable acid, or alternatively a hydrate or a solvate of the compound of formula (I).

15. Pharmaceutical composition, **characterized in that** it comprises a compound of formula (I) according to any one of Claims 1 to 10, or a pharmaceutically acceptable salt, a hydrate or a solvate of this compound, and also at least one pharmaceutically acceptable excipient.

16. Use of a compound of formula (I) according to any one of Claims 1 to 10, for the preparation of a medicament for preventing or treating pathologies in which receptors of TRPV1 type are involved.

17. Use of a compound of formula (I) according to any one of Claims 1 to 10, for the preparation of a medicament for preventing or treating pain, inflammation, metabolic disorders, urological disorders, gynaecological disorders, gastrointestinal disorders, respiratory disorders, psoriasis, pruritus, dermal, ocular or mucous irritations, herpes, zona, multiple sclerosis, depression and cancers.
